# EUROPEAN PATENT SPECIFICATION

(11) **EP 3 800 474 B1**
(45) Date of publication and mention of the grant of the patent: **01.11.2023**
(21) Application number: 20197258.5
(22) Date of filing: 30.03.2012
(51) Int. Cl.: G01N 33/68, G01N 33/543, G01N 33/573

(54) **DETECTION OF MULTIPLE ANALYTES**
NACHWEIS MEHRERER ANALYTE
DÉTECTION D'ANALYTES MULTIPLES

(30) Priority: 31.03.2011 US 201161470359 P; 31.03.2011 US 201161470395 P; 15.08.2011 US 201161523687 P
(43) Date of publication of application: 07.04.2021
(62) Divisional of application: 18182576.1
(73) Proprietor: TGR BIOSCIENCES PTY LTD, Thebarton, SA 5031 (AU)
(72) Inventor: SHEEHAN, Antony James, Thebarton, South Australia 5031 (AU); OSMOND, Ronald Ian William, Thebarton, South Australia 5031 (AU); CROUCH, Michael Francis, Thebarton, South Australia 5031 (AU)
(74) Representative: Boult Wade Tennant LLP

(56) References cited:
- EP-A1- 2 550 147
- WO-A1-02/071067
- WO-A1-2011/057347
- WO-A2-2005/017485
- ALEXANDER D. EDWARDS ET AL: "A simple device for multiplex ELISA made from melt-extruded plastic microcapillary film", LAB ON A CHIP, vol. 11, no. 24, 1 January 2011 (2011-01-01), page 4267, XP055226990, GB ISSN: 1473-0197, DOI: 10.1039/c0lc00357c & Alexander D Edwards ET AL: "A simple device for multiplex ELISA made from melt-extruded plastic microcapillary film; Supplementary Data", , 1 January 2011 (2011-01-01), XP055226994, Retrieved from the Internet: URL:http://www.rsc.org/suppdata/lc/c0/c0lc 00357c/c0lc00357c.pdf [retrieved on 2015-11-09]
- Rick Wiese ET AL: "Simultaneous Multianalyte ELISA Performed on a Microarray Platform", Clinical Chemistry, 1 August 2001 (2001-08-01), page 1451, XP055226803, United States Retrieved from the Internet: URL:http://www.clinchem.org/content/47/8/1 451.full.pdf#page=1&view=FitH
- EMIL KARTALOV ET AL: "High-throughput multi-antigen microfluidic fluorescence immunoassays", BIOTECHNIQUES, vol. 40, no. 1, 1 January 2006 (2006-01-01), pages 85-90, XP055227253, US ISSN: 0736-6205, DOI: 10.2144/000112071
- DENNIS R. TRUNE ET AL: "Simultaneous measurement of multiple ear proteins with multiplex ELISA assays", HEARING RESEARCH, vol. 275, no. 1-2, 7 December 2010 (2010-12-07), pages 1-7, XP055227059, NL ISSN: 0378-5955, DOI: 10.1016/j.heares.2010.11.009
- MICHAEL LIEW ET AL: "Validating a custom multiplex ELISA against individual commercial immunoassays using clinical samples", BIOTECHNIQUES, vol. 42, no. 3, 1 March 2007 (2007-03-01), pages 327-333, XP055227060, US ISSN: 0736-6205, DOI: 10.2144/000112332
- Cat # Qah-Mmp-: "Quantibody Human MMP Array 1 - quantitative measurement of 10 human matrix metallopreoteinase related proteins", , 1 July 2010 (2010-07-01), XP055227057, Retrieved from the Internet: URL:http://www.filgen.jp/Product/Bioscienc e15-Raybio-quantibody/Quantibody_Human_MMP _Array_1.pdf [retrieved on 2015-11-09]
- MAO Y Q ET AL: "PS3-71 High-throughput cytokine quantification using multiplex ELISA microarrays", CYTOKINE, ACADEMIC PRESS LTD, PHILADELPHIA, PA, US, vol. 52, no. 1-2, 1 October 2010 (2010-10-01), page 96, XP027261969, ISSN: 1043-4666 [retrieved on 2010-09-02]
- Paula Denney Eason ET AL: "MULTI-SPOT(TM) Assays: Detection of Multiple Phosphoproteins in Whole Cell Lysates in a Single Well", , 11 May 2006 (2006-05-11), XP055159400, Retrieved from the Internet: URL:http://www.mesoscale.com/CatalogSystem Web/WebRoot/literature/applications/pdf/Mu ltiSpotPhospho.pdf [retrieved on 2014-12-18]
- QIU-PING QIN ET AL: "Point-of-Care Time-resolved Immunofluorometric Assay for Human Pregnancy-associated Plasma Protein A: Use in First-Trimester Screening for Down Syndrome Background: Screening for Down syndrome in the", CLINICAL CHEMISTRY., vol. 48, no. 3, 1 March 2002 (2002-03-01), pages 473-483, XP055364489, WASHINGTON, DC. ISSN: 0009-9147
- YONG-PING TANG ET AL: "Determination of median levels of the free &bgr; subunit of human chorionic gonadotropin in women from mainland China using a new time-resolved fluoroimmunoassay", CLINICAL CHEMISTRY AND LABORATORY MEDICINE, vol. 48, no. 1, 1 January 2010 (2010-01-01), XP055159405, ISSN: 1434-6621, DOI: 10.1515/CCLM.2010.005

## Description

### FIELD

The present disclosure relates to methods and/or kits for detecting multiple analytes.

### BACKGROUND

In many cases, analysis of the sample requires multiple analytes to be detected. Traditionally this has been accomplished by detecting the multiple analytes by parallel analysis of the sample. However, there is an increasing need to be able to detect multiple analytes in a single sample.

In some circumstances it is useful to be able to detect multiple analytes in the same reaction. This is generally referred to as "multiplex detection". This may arise for many reasons, for example, under circumstances where there is a limiting amount of sample able to be analysed, where it is either not practical or not economic to detect analytes in separate reactions, and/or for reasons where it is advantageous to have the detection occurring in the same reaction environment.

Despite advances in the detection of analytes generally, detection of multiple analytes remains challenging in many situations, for example the detection of multiple analytes in biological samples and the detection of multiple analytes in complex samples. Detection of analytes in such samples often utilises recognition of the analyte by binding to another molecule. However, techniques involving binding of the analyte to another molecule present challenges when multiple analytes need to be detected in the same reaction, including for example one or more of the following: the ability to distinguish between different analytes, overlap and/or interference between detection signals, insufficient sensitivity to allow detection of low amount of analyte, and interference from other agents in a sample.

In addition, multiple analyte detection using some technologies is often expensive and/or requires the acquisition of new equipment, therefore placing a financial limitation to investing in such technologies.

Accordingly, there is a need to provide methods and/or kits for detection of multiple analytes to address one or more problems in the art and/or provide one or more advantages.

Reference to any prior art in this specification is not, and should not be taken as, an acknowledgment or any form of suggestion that this prior art forms part of the common general knowledge in any country.

Wiese, R., et al., (2001), Clinical Chemistry, page 1451 (D4) relates to a multianalyte ELISA performed on a microarray platform. Kartalov, E., et al (2006), Biotechniques, 40(1)85-90 relates to high-throughput multi-antigen microfluidic fluorescence immunoassays. Trune, DR., et al, (2010), 275(1-2)1-7 relates to simultaneous measurement of multiple proteins with multiplex ELISA assays. Liew, M., et al, (2007), Biotechniques, 42(3)327-333 (D7) relates to validating a custom multiplex ELISA against individual commercial immunoassays using clinical samples. The Quantibody Human MMP Array relates to quantitative measurement of 10 human matrix metalloproteinase related proteins. Mao, YQ., et al (2010), 52(1-2)96 relates to high-throughput cytokine quantification using multiplex ELIA microarrays. WO2005017485A2 relates to a multiplexed ELISA. Eason, PD., et al, (2006), relates to assays for the detection of multiple phosphoproteins in whole cell lysates in a single well. WO2002071067A1 relates to a method for determining simultaneously 2 or more modified proteins using substrate bound antibodies. Qin, QP., et al, (2002), Clinical Chemistry, 48(3) 743-483 relates to a point-of-care time-resolved immunofluorimetric assay for human pregnancy-associated plasma protein A. Tang, YP., et al, (2010) Clinical Chemistry and Laboratory Medicine, 48(1)109-114 (D14) relates to the determination of median levels of the free β subunit of human chorionic gonadotropin in women from mainland China using a time-resolved fluoroimmunoassay.

### SUMMARY

The present disclosure relates to the detection of multiple analytes. Certain embodiments of the present disclosure are directed to methods and/or kits for detecting multiple analytes.

In one aspect the present invention is directed to a method for detecting different non-nucleic acid analytes in a sample, the method comprising:
(a) providing to a reaction vessel:
   (i) at least one solid substrate comprising one or more immobilisation agents;
   (ii) the sample;
   (iii) at least two different antibody capture agents that bind to at least two different analytes, wherein the at least two different capture agents can be immobilised on the at least one solid substrate;
      wherein one or more of the at least two capture agents comprises a plurality of ligands for the immobilisation agent and wherein the ligand and the immobilisation agent are a binding pair comprising a peptide-tag and an anti-peptide antibody; and
   (iv) at least two different detectable agents that bind to the at least two different analytes;
(b) washing the at least one solid substrate in the reaction vessel to remove one or more different capture agents and one or more different detectable agents not bound to the at least one solid substrate; and
(c) detecting the at least two different analytes in the sample by detecting the presence of the at least two different analytes bound to the at least one solid substrate, wherein said detecting is via detecting the presence of the at least two different detectable agents bound to the at least one solid substrate via the at least two analytes.

In one embodiment the method comprises
(a) providing one or more of the at least two different capture agents at a concentration of 10 ng/ml to 1000 ng/ml;
(b) providing one or more of the at least two different detectable agents at a concentration of 10 ng/ml to 1000 ng/ml; and/or
(c) providing one or more of the at least two different capture agents in solution.

In one embodiment one or more of the at least two different detectable agents comprises an antibody.

In one embodiment one or more of the at least two detectable agents comprises one or more detectable tags.

In one embodiment the one or more detectable tags comprises a fluorophore.

In one embodiment the one or more detectable tags comprise one or more lanthanide ions, wherein the lanthanide ion is preferably one or more of Eu³⁺, Sm³⁺, Tb³⁺, and Dy³⁺, for example wherein one of the detectable tags comprises Eu³⁺ and another of the detectable tags comprises Sm³⁺,

In one embodiment the one or more of the detectable tags comprises an enzyme that converts a substrate into a detectable product, wherein the enzyme is preferably selected from the group consisting of horse radish peroxidase, alkaline phosphatase, and beta-galactosidase.

In one embodiment the method comprises contacting the sample, one or more of the at least two different capture agents, one or more of the at least two different detectable agents and the at least one solid substrate in the reaction vessel.

In one embodiment the sample and the solid substrate are contacted prior to contacting with one or more of the different capture agents and/or one or more of the different detectable agents and/or
wherein the sample and the at least one solid substrate are first contacted in the reaction vessel.

In one embodiment (a) the sample and the at least one solid substrate are not substantially incubated prior to contacting with one or more of the at least two different capture agents and/or one or more of the at least different detectable agents; or
(b) the method comprises an incubation of the contacted sample, the at least one solid substrate, one or more of the at least two different capture agents and one or more of the at least two different detectable agents.

In one embodiment (a) the at least two different analytes are detected simultaneously; or
(b) the at least two different analytes are detected sequentially.

In one embodiment (a) the reaction vessel comprises the at least one solid substrate;
(b) the reaction vessel comprises a pipette tip, wherein the pipette tip preferably comprises the at least one solid substrate; and/or
(c) the at least one solid substrate comprises a plurality of beads, wherein the plurality of beads are preferably retained in a pipette tip.

In one embodiment (a) the sample is a blood sample, a serum sample, a urine sample, a cell sample, a cell lysate or a combination of any of the aforementioned;
(b) one or more of the at least two different analytes are selected from the group consisting of a component of a cell signalling pathway, a cytokine, or a tumour suppressor; and/or
(c) one or more of the at two different analytes comprises a protein, wherein the protein is preferably a phosphoprotein, more preferably wherein one or more of the at least two different analytes is selected from the group consisting of phospho-ERK 1/2; total ERK 1/2; phospho-Akt 1/2/3; total Akt 1/2/3; phospho-NF-Kβ p65; total NF-Kβ p65; phospho-1-kBa; total-kBcc; phospho-STAT3; total STAT3; phospho-STATS A/B; phospho-JNK 1/2/3; total JNK 1/2/3; phospho-p38 MAPKcc; total p38 MAPKD ; phospho-p53; total p53; phospho-p70S6K; total p70S6K; and GAPDH.

In one embodiment the method comprises production of
(a) at least one detectable signal with a peak emission wavelength in the range from 500-560 nm and another detectable signal with a peak emission wavelength in the range from 570-610;
(b) at least one detectable signal with a peak emission wavelength in the range from 380-490 nm and another detectable signal with a peak emission wavelength in the range from 500-560;
(c) at least one detectable signal with a peak emission wavelength in the range from 380-490 nm and another detectable signal with a peak emission wavelength in the range from 570 to 750 nm; or
(d) at least one detectable signal with a peak emission wavelength in the range from 500-560 nm and another detectable signal with a peak emission wavelength in the range from 570 to 750 nm.

In one embodiment the detecting of the at least two different analytes is achieved in a time of 2 hours or less; and/or
wherein the method comprises providing one or more filters and/or one or more light sources and/or one or more light detectors to a plate reader device to enable detection of the at least two different analytes.

In another aspect the present invention is directed to a kit for performing the methods of the invention, the kit comprising
(i) at least one solid substrate comprising one or more immobilisation agents;
(ii) at least two different antibody capture agents that bind to at least two different analytes, wherein the at least two different capture agents can be immobilised on the at least one solid substrate;
   wherein one or more of the at least two capture agents comprises a plurality of ligands for the immobilisation agent and wherein the ligand and the immobilisation agent are a binding pair comprising a peptide-tag and an anti-peptide antibody;
   and
(iii) at least two different detectable agents that bind to the at least two different analytes; and optionally
a reaction vessel; a solution for washing the reaction vessel to remove capture agents and/or detectable agents not bound to the reaction vessel; a cell lysis buffer, a signal enhancing agent, and/or instructions for detection of the different analytes.

### BRIEF DESCRIPTION OF THE FIGURES

Certain embodiments and reference Examples are illustrated by the following figures.

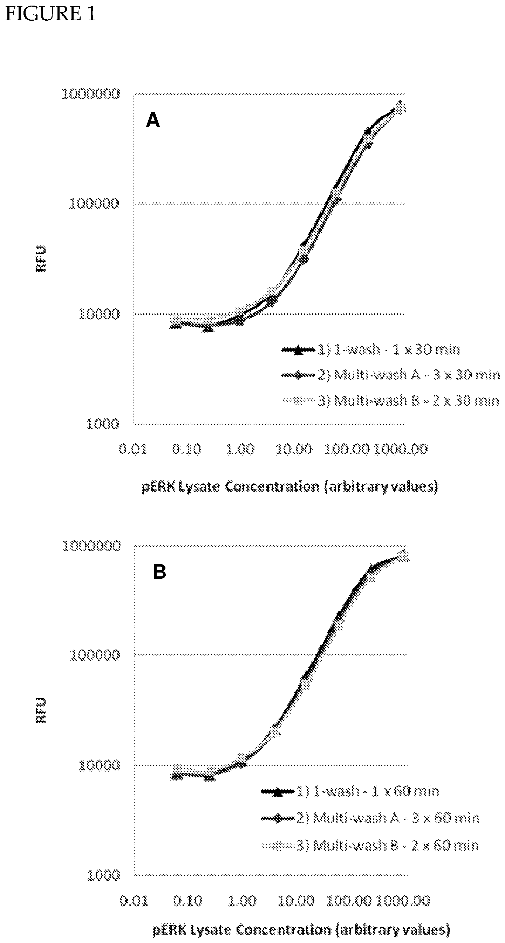

Figure 1 shows for the purposes of comparison, three ELISA protocols for the detection of phosphorylated ERK 1/2 (pERK) were examined, using various concentrations of a cellular lysate containing pERK. (1) A simultaneous ELISA format, whereby the assay components, namely the capture antibody (anti-pERK-biotin), the analyte (cellular lysate), and the detection antibody (anti-ERK-HRP), were incubated concurrently in a streptavidin-coated microplate. (2) A standard multi-incubation ELISA format, whereby the capture antibody was first incubated in a streptavidin-coated microplate, followed by the analyte, and finally the detection antibody. (3) A standard multi-incubation ELISA format, whereby the analyte was incubated in a capture-antibody coated microplate, followed by a detection antibody. The assays were incubated for either 30 min (Figure 1A) for each incubation step or 60 min (Figure 1B) for each incubation step, and the wells were subjected to a standard wash cycle between each incubation step for each assay. After the final incubation and wash, QuantaRed^{™} HRP substrate was added to the wells, and each plate was incubated for 10 min in the dark. The fluorescent signal in the wells was measured at 550ex/600em nm. Figures 1A and 1B show the mean and standard deviations for the duplicate data points at each pERK lysate concentration analyzed. In this Figure the comparison clearly demonstrate comparable assay performance over a shorter time period when the assay components are incubated concurrently, compared with standard ELISA protocols whereby assay components are incubated sequentially.

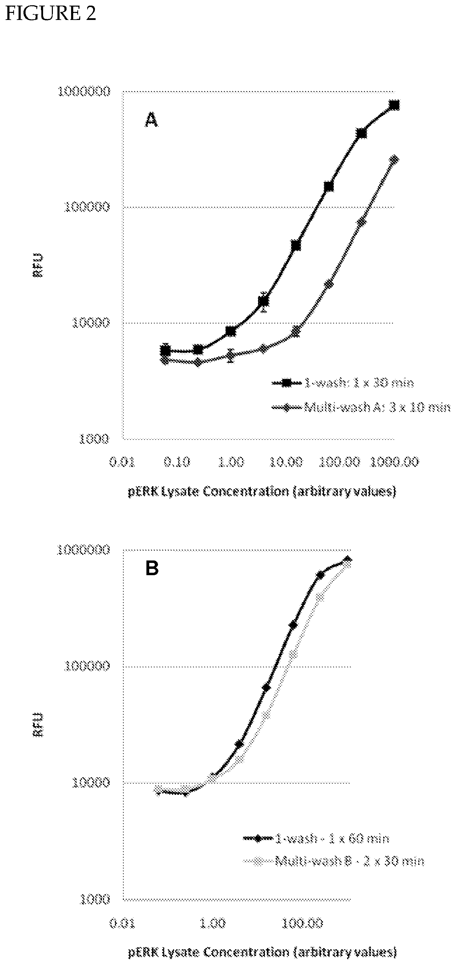

Figure 2 shows for the purposes of comparison, three ELISA protocols for the detection of phosphorylated ERK 1/2 (pERK), using various concentrations of a cellular lysate containing pERK. (1) A simultaneous ELISA format, whereby the assay components, namely the capture antibody (anti-pERK-biotin), the analyte (cellular lysate), and the detection antibody (anti-ERK-HRP), were incubated concurrently in a streptavidin-coated microplate for either 30 min (Figure 2A) or 60 min (Figure 2B). (2) A standard multi-incubation ELISA format, whereby the capture antibody was first incubated in a streptavidin-coated microplate for 10 min, followed by the analyte for 10 min, and finally the detection antibody for 10 min, giving a total cumulative assay incubation time of 30 min (Figure 2A). (3) A standard multi-incubation ELISA format, whereby the analyte was incubated in a capture-antibody coated microplate for 30 min, followed by the detection antibody for 30 min, giving a total cumulative assay time of 60 min (Figure 1B). The wells were subjected to a standard wash cycle between each incubation step for each assay. After the final incubation and wash, QuantaRed^{™} HRP substrate was added to the wells, and each plate was incubated for 10 min in the dark. The fluorescent signal in the wells was measured at 550ex/600em nm. Figures 2A and 2B show the mean and standard deviations for the duplicate data points at each pERK lysate concentration analyzed. In this Figure, the comparison clearly demonstrate better assay performance the same total assay time period when the assay components are incubated concurrently, compared with standard ELISA protocols whereby assay components are incubated sequentially.

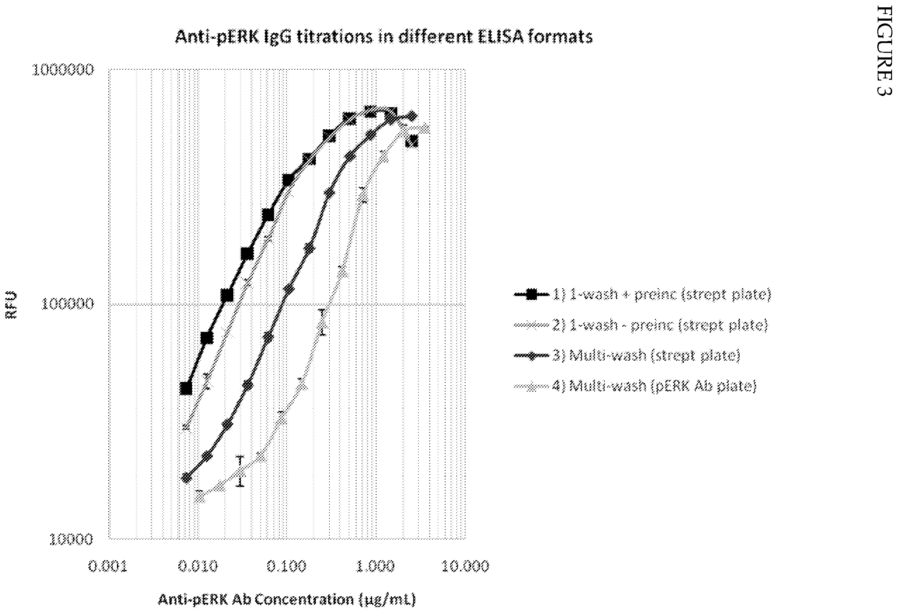

Figure 3 shows for the purposes of comparison, the concentration of the capture antibody (anti-phospho-ERK) required for optimal assay performance for three ELISA protocols for the detection of phosphorylated ERK 1/2, using varying concentrations of the capture antibody in combination with a fixed concentration of both cellular lysate, and detection antibody. (1) A simultaneous ELISA format, whereby the assay components, namely the capture antibody (anti-pERK-biotin), the analyte (cellular lysate), and the detection antibody (anti-ERK-HRP), were incubated concurrently in a streptavidin-coated microplate for 120 min. (2) A simultaneous ELISA format, whereby the assay components, namely the capture antibody (anti-pERK-peptide), the analyte (cellular lysate), and the detection antibody (anti-ERK-HRP), were incubated concurrently in an anti-peptide antibody-coated microplate for 120 min. (3) A standard multi-incubation ELISA format, whereby the analyte was incubated in a capture-antibody (non-biotinylated) coated microplate for 120 min, followed by the detection antibody for 120 min. The wells were subjected to a standard wash cycle between each incubation step for each assay. After the wash cycle, HRP substrate was added to the wells, and the plates were incubated for 10 min in the dark. The fluorescent signal in the wells was measured at 540ex/590em nm. Figure 3 shows the mean and standard deviations for the duplicate data points for each target analyzed. In this Figure, the comparison clearly demonstrates that optimal assay performance is achieved with lower capture antibody concentrations when the assay components are incubated concurrently for both biotin-capture and peptide-capture protocols, when compared with standard ELISA protocols whereby analytes are incubated sequentially, and washed between incubations. This data demonstrates that the assay has the potential to lower input costs for ELISA plate manufacture.

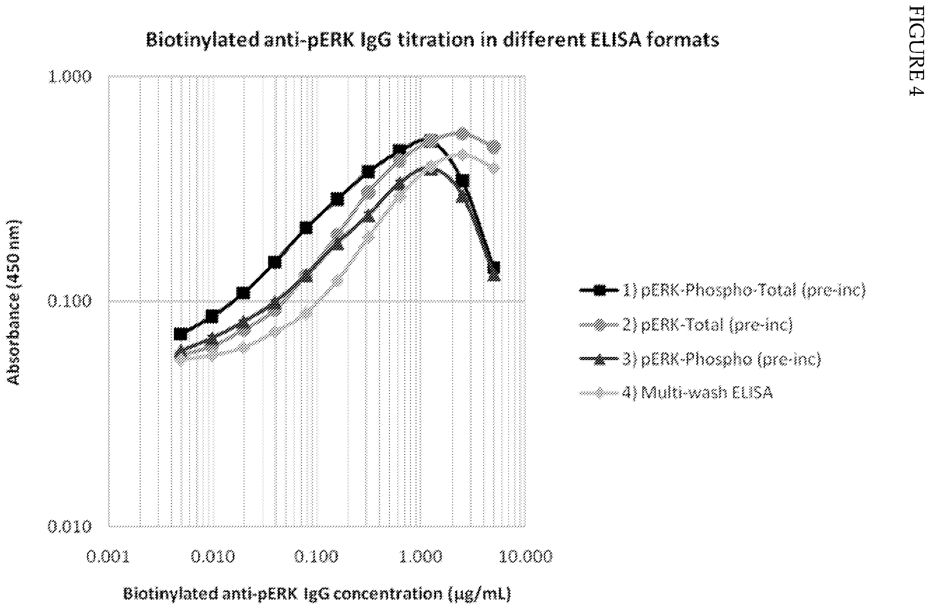

Figure 4 shows for the purposes of comparison, the requirement for sequential incubations for optimal assay performance for two ELISA protocols for the detection of phosphorylated ERK 1/2. (1) A simultaneous ELISA format, whereby the assay components, namely the capture antibody (anti-phospho-ERK-peptide), the analyte (cellular lysate), and the detection antibody (anti-ERK-HRP), were incubated concurrently in an antipeptide antibody-coated microplate for 120 min. (2) A sequential ELISA format, whereby the solution-phase assay components, namely the capture antibody (anti-pERK-peptide), the analyte (cellular lysate), and the detection antibody (anti-ERK-HRP), were incubated concurrently in a separate reaction vessel for 60 min. The assay components were subsequently transferred to an antipeptide antibody-coated microplate for 60 min. At the conclusion of incubation on the antipeptide antibody-coated assay microplate, both protocols required a standard wash cycle. After the wash cycle, HRP substrate was added to the wells, and the plates were incubated for 10 min in the dark. The fluorescent signal in the wells was measured at 540ex/590em nm.
Figure 4 shows the mean and standard deviations for the duplicate data points for each target analyzed. In this Figure, the comparison clearly demonstrates that no benefit to assay performance is achieved with the inclusion of a pre-incubation step prior to introduction to a solid substrate carrying the immobilization agent.

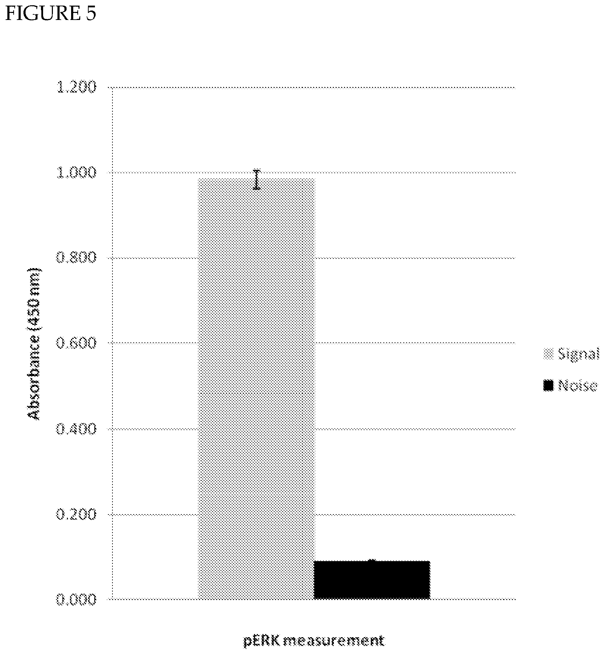

Figure 5 shows a single-incubation, single-wash ELISA, was performed using a 3-antibody configuration. The assay components, namely the capture antibody (anti-pERK-biotin), the analyte (cellular lysate), the detection antibody (rabbit anti-ERK), and a generic anti-rabbit-HRP antibody, were incubated concurrently in a streptavidin-coated microplate for 120 min (signal), and compared with a similar assay run with a buffer-only control for the analyte (noise). The wells were subjected to a standard wash cycle after the incubation step, and SigmaFAST^{™} HRP substrate was added to the wells, and each plate was incubated for 10 min in the dark. The colorimetric signal in the wells was measured at 450 nm. Figures 5 shows the mean and standard deviations for the duplicate data points at each pERK lysate concentration analyzed. In this Figure, the assay clearly demonstrates the utility whereby the assay components are incubated concurrently.

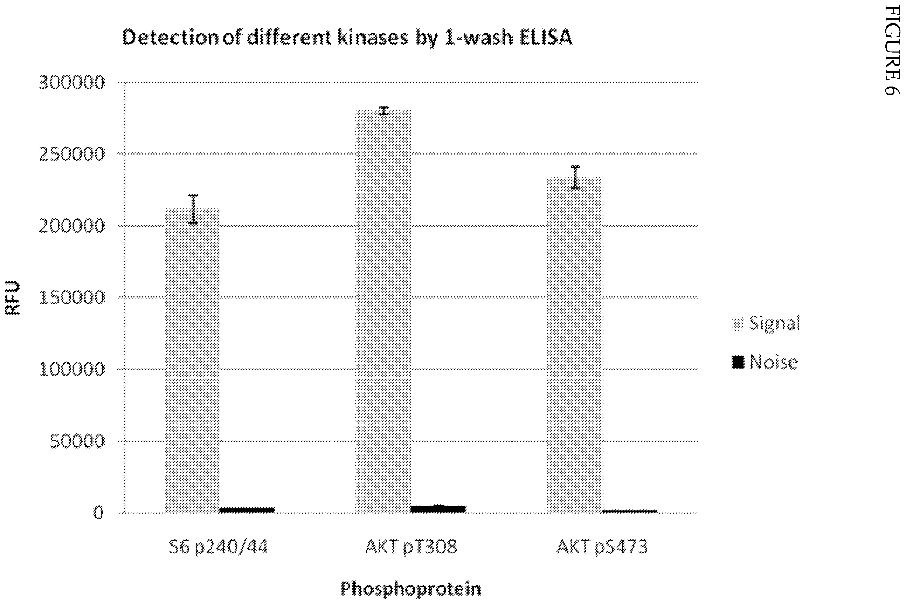

Figure 6 shows the detection of different kinases by a single incubation, single wash ELISA. Cell lysates containing either phosphorylated S6 p240/44, AKT pT308 or AKT pS473 (signal), or buffer-only controls (noise) were added to separate wells of an assay microplate (streptavidin coated 384-well Nunc Maxisorp^{™} plate). The reaction was started by the addition of target-specific antibody pairs (one biotinylated and the other conjugated to HRP) to the lysates. The assays were incubated for 2 h, then subjected to a wash cycle. After the wash cycle, QuantaRed^{™} HRP substrate was added to the wells, and the plate was incubated for 10 min in the dark. The fluorescent signal in the wells was measured at 550ex/600em nm. Figure 6 shows the mean and standard deviations for the duplicate data points for each target analyzed. In this Figure, the assay clearly demonstrates efficacy for several different targets, whereby the assay components are incubated concurrently.
Figure 7 is a schematic diagram showing a microfluidic cartridge suitable for use in accordance with some embodiments of the present disclosure.
Figure 8 demonstrates the results of electrochemical detection of pERK in a microfluidic system. Figure 8A shows the raw results of electrochemical detection (in mV) during the substrate flow through phase and substrate incubation phase. Figure 8B shows a pERK standard curve generated using data taken from 60 seconds after injection of substrate (during flow through phase). Figure 8C shows a pERK standard curve generated using data taken from 180 seconds after injection of substrate (at the end of the substrate incubation phase).
Figure 9 shows the results of electrochemical detection of pAKT473 in a microfluidic system. Figure 9A shows the raw results of electrochemical detection (in mV) during the substrate flow through phase and substrate incubation phase. Figure 9B shows a pAKT473 standard curve generated using data taken from 60 seconds after injection of substrate (during flow through phase). Figure 9C shows a pAKT473 standard curve generated using data taken from 180 seconds after injection of substrate (at the end of the substrate incubation phase).

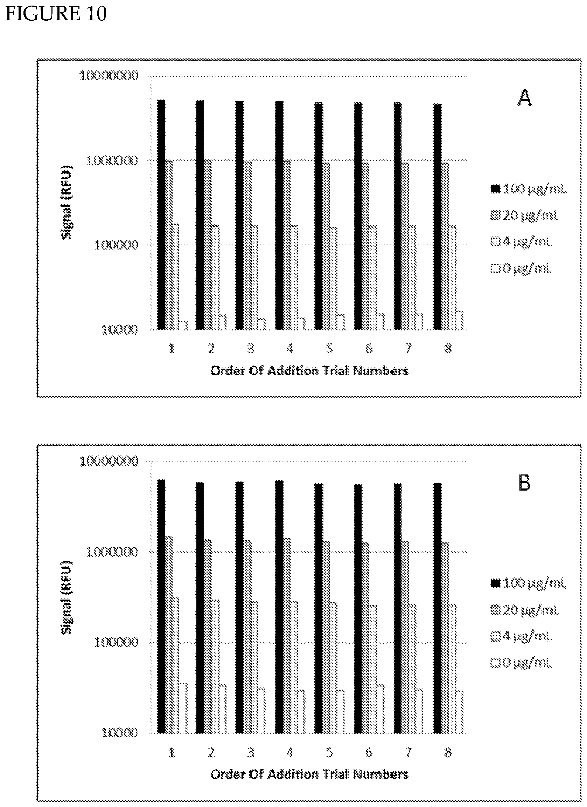

Figure 10 demonstrates equivalent assay performance with various permutations on the order of delivery of assay components to the assay well, using (A) a peptide capture antibody conjugate (anti-pERK-peptide), (B) or biotin capture antibody conjugate (anti-pERK-biotin), as the assay capture reagent The assay components were added in various permutations (refer to example 7, Tables 1 and 2). Individual assay components were added 1 min apart to the plates and incubated for 2 h at room temperature, then subjected to a wash cycle. After the wash cycle, HRP substrate was added to the wells, and the plates were incubated for 10 min in the dark. The fluorescent signal in the wells was measured at 540ex/590em nm. Figure 10 shows the mean and standard deviations for the duplicate data points for each target analyzed. In this Figure, the assays clearly demonstrate that when added within the short time period described, the order of addition of individual assay components does not affect assay performance, compared with assay components that are added simultaneously.

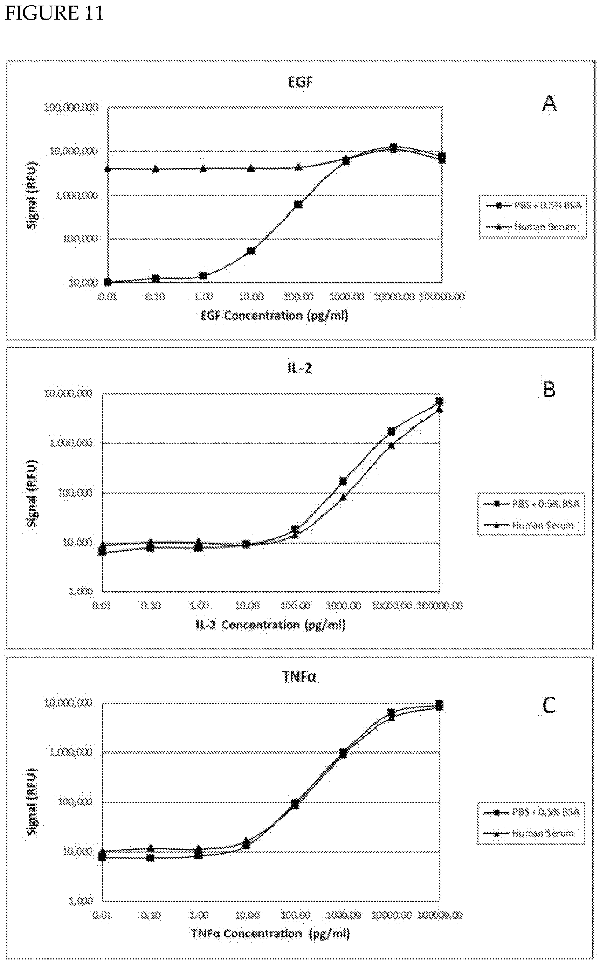

Figure 11 shows detection of recombinant human EGF, IL-2 and TNFα, in either PBS/0.5% BSA or human serum. Peptide-capture antibody conjugates, and HRP-detection antibody conjugates specific for each of EGF (A), IL-2 (B) and TNFα (C) were prepared. Recombinant EGF, IL-2 or TNFα were prepared at concentrations ranging from 100 ng/mL to 10 fg/mL, in either PBS/0.5%BSA, or human serum, and 50 µL/well of each analyte was added to an ELISA assay plate coated with an anti-peptide antibody. The assays were initiated by addition of mixtures containing both specific antibodies for each of EGF, IL-2 or TNFα, along with a general anti-HAMA composition available commercially from Bioreclamation LLS (Westbury, NY, USA - 'Immunoglobulin Inhibiting Reagent (IIR)), to the appropriate ELISA plate wells. The assays were incubated for 1 h, then subjected to a wash cycle. After the wash cycle, HRP substrate was added to the wells, and the plates were incubated for 10 min in the dark. The fluorescent signal in the wells was measured at 540ex/590em nm. Figure 11 shows the mean and standard deviations for the duplicate data points for each target analyzed. In this Figure, the assay clearly demonstrates efficacy for several different targets in serum, whereby the assay components are incubated concurrently. The high signal for EGF in human serum is due to the presence of endogenous EGF protein(s) in this medium.

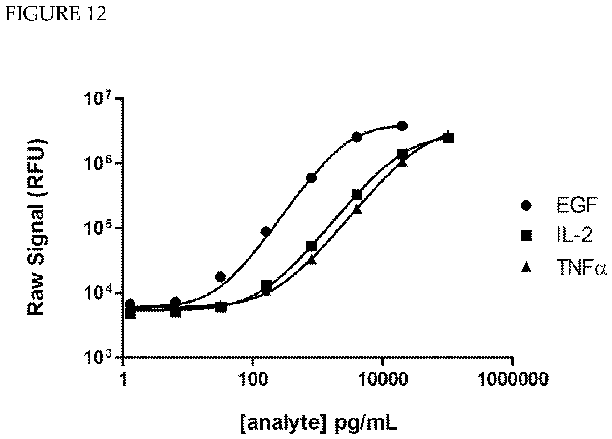

Figure 12 shows detection of recombinant human EGF, IL-2 and TNFα in a 15 min total assay time. Peptide-capture antibody conjugates, and HRP-detection antibody conjugates specific for each of EGF (A), IL-2 (B) and TNFα (C) were prepared. Recombinant EGF, IL-2 or TNFα were prepared at concentrations ranging from 100 ng/mL to 10 fg/mL, in PBS/0.5%BSA, and 50 µL/well of each analyte was added to an ELISA assay plate coated with an anti-peptide antibody. The assays were initiated by addition of mixtures containing both specific antibodies for each of EGF, IL-2 or TNFα to the appropriate ELISA plate wells. The assays were incubated for 10 min, then subjected to a wash cycle. After the wash cycle, HRP substrate was added to the wells, and the plates were incubated for 5 min in the dark. The fluorescent signal in the wells was measured at 540ex/590em nm. Figure 12 shows the mean and standard deviations for the duplicate data points for each target analyzed. In this Figure, the assay clearly demonstrates efficient detection within 15 min total assay time for several different targets, using certain embodiments, whereby the assay components are incubated concurrently.
Figure 13 shows intra-plate variation observed for 2 separate single-incubation ELISAs for either phospho-AKT (pSer473) or phospho-STAT3.
Figure 14 shows detection of TNFα in tissue culture supernates.

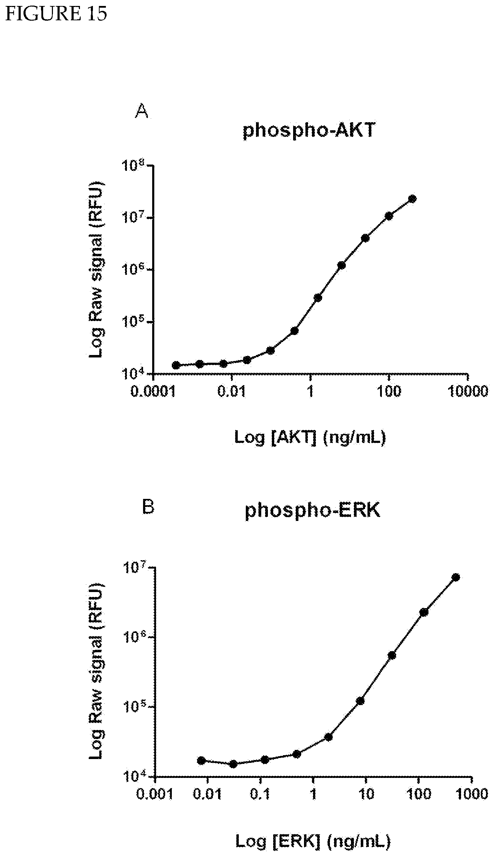

Figure 15 shows detection of either phospho-AKT (pSer473) or phospho-ERK in a 25 min total assay time. For each target, recombinant active (A) phospho-AKT or (B) phospho-ERK was diluted as indicated, to various concentrations using 1X Lysis buffer containing 0.1% BSA and added to 4 replicate wells of a 96-well streptavidin-coated microplate. To initiate the assay reaction, for either target, a mixture of the biotin-conjugated capture antibody, and the HRP-conjugated detection antibody were added to the lysates, and incubated for 1 hour. The wells were subjected to a standard wash cycle for each assay. After the wash cycle, QuantaRed^{™} HRP substrate was added to the wells, and each plate was incubated for 10 min in the dark. The fluorescent signal in the wells was measured at 550ex/600em nm. Figures 15A and 15B show the data points at each analyte concentration analyzed, for phospho-AKT and phospho-ERK, respectively. Both assays demonstrated sensitivity to less than 1 ng/mL.
Figure 16 shows detection of various concentrations of IL-2 using a peptide tag/anti-peptide tag antibody capture system.
Figure 17 shows detection of various concentrations of IL-2 using a peptide tag anti peptide tag antibody capture system.
Figure 18 shows detection of various concentrations of EGF, IL-2 & TNFα using a peptide tag anti peptide tag antibody capture system.
Figure 19 shows the signal obtained for various concentrations of analyte using a peptide tag anti peptide tag antibody capture system.
Figure 20 shows a comparison of a biotin-streptavidin capture system to a peptide tag - anti-peptide antibody capture system in various biological milieu.
Figure 21A shows that a streptavidin biotin capture system utilizing an antibody capture agent and an antibody detectable agent is not affected by increasing concentrations of irrelevant antibodies. Figure 21B shows the data from Figure 21A has been normalised in terms of signal:noise, where noise is the signal of the immunocomplex obtained for each condition compared to the signal obtained in the absence of analyte.
Figure 22A shows that anti peptide tag antibody - peptide capture system utilizing an antibody capture agent and an antibody detectable agent is not affected by increasing concentrations of irrelevant antibodies. Figure 22B shows the data from Figure 22A has been normalised in terms of signal:noise, where noise is the signal of the immunocomplex obtained for each condition compared to the signal obtained in the absence of analyte.

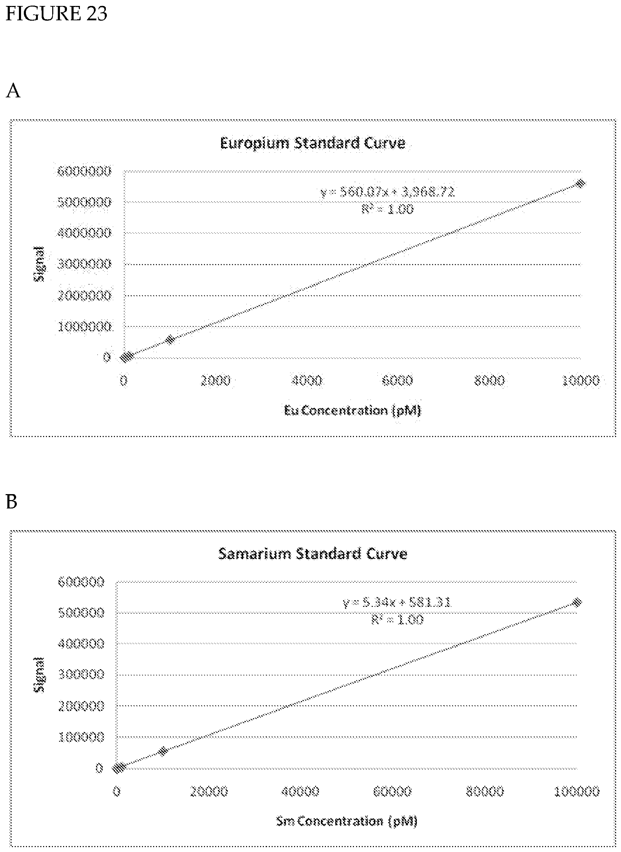

Figure 23 shows the detection of lanthanide-labelled antibodies. Two antibodies were labelled with lanthanide with PerkinElmer labelling kits: An anti-EGF antibody was labelled with Europium using an Eu-labelling kit, PerkinElmer cat number 1244-302. An IL-2 antibody was labelled with Samarium using a Sm-labelling kit, PerkinElmer cat number 1244-303. Antibodies were incubated with the respective lanthanide solutions for 16 hours at room temperature, and conjugates were then desalted using a PD10 column. Standard curves for each conjugate were constructed using log10 dilutions of the conjugates, maximal concentrations for Eu and Sm being 10nM and 100nM, respectively. The time-resolved fluorescence readings for these solutions were assessed in microtitre plates in a Victor II plate reader (PerkinElmer). Excitation and emission wavelengths for these lanthanides were: Europium: Excitation 340 nm/Emission 615 nm; Samarium: 340 nm/Emission 642 nm.
Figure 24 shows the duoplexed detection of EGF and IL-2 using lanthanide-labelled antibodies in a plate reader using time-resolved fluorescence detection.
Figure 25 shows the emission of methylumbelliferone at various concentrations in the presence of varying concentrations of fluorescein, with an interpolated concentration of resorufin of 5.5uM.
Figure 26 shows the emission of fluorescein at various concentrations in the presence of varying concentrations of methylumbelliferone, with an interpolated concentration of resorufin of 5.5uM.
Figure 27 shows the emission of resorufin at various concentrations in the presence of varying concentrations of methylumbelliferone, with an interpolated concentration of fluorescein of 5.5uM.
Figure 28 shows the emission of methylumbelliferone at various concentrations in the presence of varying concentrations of resorufin, with an interpolated concentration of fluorescein of 5.5uM.
Figure 29 shows the emission of fluorescein at various concentrations is shown in the presence of varying concentrations of resorufin, with an interpolated concentration of methylumbelliferone of 5.5uM.
Figure 30 shows the emission of resorufin at various concentrations in the presence of varying concentrations of fluorescein, with an interpolated concentration of methylumbelliferone of 5.5uM.

### DETAILED DESCRIPTION

The present disclosure relates to the detection of multiple analytes. Certain embodiments of the present disclosure are directed to methods and/or kits for detecting multiple analytes in a sample.

Certain disclosed embodiments provide methods and/or kits for detecting multiple analytes that have one or more combinations of advantages. For example, some of the advantages of the embodiments disclosed herein include one or more of the following: the ability to distinguish multiple analytes in a sample; the ability to distinguish multiple analytes in the same reaction environment; a reduction in the overlap in the detection signals used to detect the multiple analytes; a reduction in the interference between the detection signals used to detect the multiple analytes; providing sufficient sensitivity to allow the detection of low amounts of multiple analytes; reduced interference from other agents in a sample; the ease and/or economy of detection of multiple analytes; the ability to use existing hardware to detect the multiple analytes; and the ability to use existing hardware to detect the multiple analytes in the same reaction vessel, such as existing plate readers.

Other advantages of certain embodiments of the present disclosure are also disclosed herein. For example, certain embodiments may have one or more combinations of the following advantages: providing an assay system that uses low amounts of reagents for capturing and detecting the multiple analytes; providing an assay system that has a high capacity for binding of reagents; reducing the time taken to detect the multiple analytes; reducing the number of incubation steps to detect multiple analytes; providing reliable detection of multiple analytes; eliminating the need for pre-incubation of some components during the detection of the multiple analytes; reducing the number of dispensing steps during the detection of the multiple analytes; reducing the number of aspiration steps during the detection of the multiple analytes; reducing costs in time and/or handling needed to perform the assay; using a single assay platform to detect the multiple analytes; and providing kits that are easy to manufacture and/or less costly to manufacturer.

Kits for performing the method are also disclosed herein.

The present disclosure arises, at least in part, from the determination that multiple capture agents can be used to detect multiple analytes, using an assay system whereby the capture agents can be immobilised on a solid substrate.

In certain embodiments, the methods and/or kits for detection of different analytes comprise a single reaction vessel.

The detection of multiple analytes in the same reaction environment, such as a reaction vessel, is sometimes referred to as "multiplex detection", with the detection of two different analytes sometimes referred to as "duoplex detection", the detection of three different analytes sometimes referred to as "trioplex detection" and so forth.

Certain embodiments provide methods and/or kits for detecting at least 2, at least 3, at least 4, at least 5, at least 6 different analytes in the sample. In certain embodiments, the method comprises detecting at least 2 or at least 3 different analytes in the sample. Other aspects provide methods and/or kits for detecting 2 to 4, 2 to 5, 2 to 6, 3 to 5, 3 to 6, or 4 to 6 different analytes in the sample.

Multiplex detection of analytes, for example, provides a number of challenges over standard serial or parallel detection of analytes. Examples of such challenges include one or more of the following: inability to distinguish between different analytes, overlap and/or interference between detection signals, insufficient sensitivity to allow detection of low amount of analytes, interference from other agents in a sample, consistent results over multiple assays, the inability to use existing hardware or combinations thereof. Examples of hardware include readers (for example plate readers) utilising fluorescence, luminescence, absorbance, fluorescence polarisation, time resolved fluorescence resonance energy transfer, technologies utilising imaging such as CCD cameras for imaging.

The method provided herein has one or more of the following advantages: the ability to distinguish between different analytes; a reduction in overlap and/or interference between detection signals to allow detection of multiple analytes; improved sensitivity to allow detection of low amount of one or more different analytes; reduced interference from other agents in a sample; consistent results over multiple assays; and the ability to use existing hardware.

As described herein, certain embodiments provide methods and/or kits for detecting different non-nucleic acid analytes. The different non-nucleic acid analytes comprise at least two different non-nucleic acid analytes. In certain embodiments, the different non-nucleic acids comprise a plurality of different non-nucleic acid analytes.

Certain embodiments are based on the capture of an analyte by a capture agent via a mechanism that is not substantially based on nucleic acid - nucleic acid interactions, such as binding based on complementary base pairing. Certain embodiments may be based on the capture of one or more analytes by one or more capture agents via a mechanism that is not substantially based on nucleic acid - nucleic acid interactions, such as binding based on complementary base pairing. That being said, it will be understood that in certain embodiments, the one or more analytes may comprise a nucleic acid component. In certain embodiments, the mechanism may be a combination of different types of mechanisms. In certain embodiments the binding of an analyte by a capture agent is substantially based on hydrophobic, hydrophilic, non-ionic, ionic, hydrogen-bonding, polyanionic-polycationic, van der Waals interactions, or combinations thereof, substantially exclusive of nucleic acid - nucleic acid interactions.

The at least two different capture agents bind to the at least two different analytes. In certain embodiments, the at least two different capture agents primarily bind to the at least two different analytes. The term "primarily bind" refers to the binding of one agent (for example a capture agent or a detectable agent) to a target molecule as its primary binding partner, while not precluding the binding of the agent to one or more other molecules.

Examples of analytes that may be detected in the methods and/or kits include microbes, viruses, proteins, macromolecules, small molecules, drugs or suitable combinations thereof. Other types of analytes are also contemplated. In certain embodiments, one or more of the different analytes comprises a protein. In certain embodiments, one or more of the different analytes comprises a phosphoprotein. Examples of phosphoproteins are phosphorylated ERK, S6 RP p240/244, AKT pT308 or AKT pS473. In certain embodiments, one or more of the different analytes may be selected, for example, from a component of a cell signalling pathway, a cytokine, a tumour suppressor, an antibody or a fragment thereof, or combinations thereof. In certain embodiments, one or more of the different analytes comprise a particular form or state of a molecule, such as a particular form or state of a protein. For example, in certain embodiments the protein is phosphorylated, methylated, glycosylated or a combination thereof. In these embodiments, a capture may have specificity to only one form of the protein (for example a capture agent may only bind to a phosphorylated form of the protein and not to an unphosphorylated form of the protein). In certain embodiments, one or more of the different analytes may be selected, for example, from phospho-ERK 1/2; total ERK 1/2; phospho-Akt 1/2/3; total Akt 1/2/3; phospho-NF-Kβ p65; total NF-Kβ p65; phospho-1-kBα; total-kBβ; phospho-STAT3; total STAT3; phospho-STATS A/B; phospho-JNK 1/2/3; total JNK 1/2/3; phospho-p38 MAPKα; total p38 MAPKα; phospho-p53; total p53; phospho-p70S6K; total p70S6K; and GAPDH.

In certain embodiments, one or more of the different analytes is present in a sample at a concentration of 100 ng/ml or less, 10 ng/ml or less, 1 ng/ml or less, 100 pg/ml or less, 10 pg/ml or less, 1 pg.ml or less, 100 fg/ml or less, 10 fg/ml or less, or 1 fg/ml or less. In certain embodiments, one or more of the different analytes is present in the sample at a concentration of 100 ng/ml or greater, 10 ng/ml or greater, 1 ng/ml or greater, 100 pg/ml or greater, 10 pg/ml or greater, 1 pg/ml or greater, 100fg/ml or greater, 10 fg/ml or greater, 1 fg/ml or greater. In certain embodiments, one or more of the different analytes is present in the sample at a concentration of between 1 fg/ml to 100 ng/ml, 1 fg/ml to 10 ng/ml, 1 fg/ml to 1 ng/ml, 10 fg/ml to 100 ng/ml, 10 fg/ml to 10 ng/ml, 10 fg/ml to 1 ng/ml, 100 fg/ml to 100 ng/ml, 100 fg/ml to 10 ng/ml, 100 fg/ml to 1 ng/ml, 1pg/ml to 100 ng/ml, 1 pg/ml to 10 ng/ml, or 1 pg/ml to 1 ng/ml.

In certain embodiments, the detecting of two or more different analytes in a sample comprises a qualitative determination of whether the two or more different analytes are present or absent in the sample. In certain embodiments, the detecting of two or more different analytes comprises a quantitative assessment of the levels of two or more different analytes in the sample. For example, certain embodiments of the methods and/or kits allow for the quantification of the concentration of two or more different analytes in the sample. Methods for the calculation of the concentration of analytes are known.

Certain embodiments provide detecting different analytes in a sample. For example, the sample may be a mixture, a composition, a solution, a spiked sample, a sample that may or may not contain one or more different analytes or combinations thereof. In certain embodiments, the sample comprises a laboratory sample, a research sample, a medical sample, a biological sample, a cell sample, a water sample, a food sample, and an agricultural sample, a derivative of these samples, an extract of these samples, or combinations thereof. Certain embodiments provide detecting different analytes from a single sample.

In certain embodiments, the sample comprises a medical sample or a cell sample, such as a blood sample, a serum sample, a tissue sample, a urine sample, a milk sample, a cell lysate, a derivative or extract of these, or suitable combinations thereof. In certain embodiments, the sample is pre-treated before one or more of the different analytes are detected. For example, the sample may be pre-cleared, concentrated, diluted, induced, pre-treated, processed to remove one or more components or impurities from the sample or suitable combinations thereof. In certain embodiments, the sample does not comprise fixed cells.

In relation to biological samples, one deficiency of many previous methods for detection of multiple proteins is that they are not suitable for use with samples with moderate to high levels of proteins (for example as often found in samples comprising serum or cell lysates). In at least certain embodiments, the methods and/or kits disclosed are able to handle such samples. For example, in certain embodiments the method and/or kits may be performed in the presence of moderate or high protein concentrations. Moderate or high protein concentrations may, for example, be introduced during blocking steps and/or may be included in the sample itself. For example, the sample may comprise a serum sample which may have a protein concentration up to approximately 60-80 mg/ml, a cell lysate sample which may have a protein concentration of approximately 1-3 mg/ml, or a sample from a cell-based assay which may include protein contamination from fetal bovine serum (FBS), or the like, which may be used in cell culture media. Other combinations of samples are also contemplated. Protein contamination from media may also account for a percentage of the protein contamination, for example, 1-5% of the final protein contamination in a cell lysate, which may translate to approximately 0.6-4 mg/ml of protein in addition to cellular protein.

In certain embodiments, the sample comprises a protein concentration of 0.01 mg/ml or greater, a protein concentration of 0.1 mg/ml or greater, a protein concentration of 1 mg/ml or greater, a protein concentration of 2 mg/ml or greater, a protein concentration of 10 mg/ml or greater, a protein concentration of 20 mg/ml or greater, a protein concentration of 30 mg/ml or greater, a protein concentration of 40 mg/ml, , a protein concentration of 50 mg/ml or greater, or a protein concentration of 60 mg/ml or greater. In certain embodiments, the sample may comprise a protein concentration of 0.01 mg/ml or less, a protein concentration of 0.1 mg/ml or less, a protein concentration of 1 mg/ml or less, a protein concentration of 2 mg/ml or less, a protein concentration of 10 mg/ml or less, a protein concentration of 20 mg/ml or less, a protein concentration of 30 mg/ml or less, a protein concentration of 40 mg/ml or less, a protein concentration of 50 mg/ml less, or a protein concentration of 60 mg/ml or less. In certain embodiments, the sample comprises a protein concentration of 0.01 mg/ml to 60 mg/ml, 0.01 mg/ml to 50 mg/ml, 0.01 mg/ml to 40 mg/ml, 0.01 mg/ml to 30 mg/ml, 0.01 mg/ml to 20 mg/ml, 0.01 mg/ml to 10 mg/ml, 0.01 mg/ml to 2 mg/ml, 0.01 mg/ml to 1 mg/ml, 0.01 mg/ml to 0.1 mg/ml, 0.1 mg/ml to 60 mg/ml, 0.1 mg/ml to 50 mg/ml, 0.1 mg/ml to 40 mg/ml, 0.1 mg/ml to 30 mg/ml, 0.1 mg/ml to 20 mg/ml, 0.1 mg/ml to 10 mg/ml, 0.1 mg/ml to 2 mg/ml, 0.1 mg/ml to 1 mg/ml, 1 mg/ml to 60 mg/ml, 1 mg/ml to 50 mg/ml, 1 mg/ml to 40 mg/ml, 1 mg/ml to 30 mg/ml, 1 mg/ml to 20 mg/ml, 1 mg/ml to 10 mg/ml, 1 mg/ml to 2 mg/ml, 2 mg/ml to 60 mg/ml, 2 mg/ml to 50 mg/ml, 2 mg/ml to 40 mg/ml, 2 mg/ml to 30 mg/ml, 2 mg/ml to 20 mg/ml, 2 mg/ml to 10 mg/ml, 10 mg/ml to 60 mg/ml, 10 mg/ml to 50 mg/ml, 10 mg/ml to 40 mg/ml, 10 mg/ml to 30 mg/ml, or 10 mg/ml to 20 mg/ml.

The methods and/or kits of the present disclosure are compatible in certain embodiments for detection of different analytes in a range of biological milieu, such as cellular lysates, and/or serum.

Certain embodiments provide detecting different analytes in one or more reaction environments. For example, the detection of two or more analytes may occur in the same reaction environment, or in a first reaction environment followed by one or more further reaction environments. For example, the detection of one of the at least two analytes bound to the at least one substrate may occur in a first reaction vessel, followed by transfer of the at least one solid substrate to a second reaction vessel and detection of one or more further analytes. In certain embodiments, the detection of the at least two analytes occurs in a single reaction vessel.

The reaction vessel in certain embodiments is a physical container in which the different analytes are detected. This may be a single reaction vessel, however, other forms of reactions vessels may also be used. Examples of reaction vessels include a test tube, a micro centrifuge tube, a well, a liquid holding and dispensing device (such as a tip for a pipette) or a flask. In certain embodiments, the reaction vessel comprises a well of a multi-well plate, such as a microtitre plate, or a well or surface of a microfluidic device. Multi-well plates are an example of an assay platform comprising a plurality of reaction vessels. In certain embodiments, the reaction vessel comprises a pipette tip. In certain embodiments, the detection of the different analytes is performed in the pipette tip.

In certain embodiment, the reaction vessel is part of an assay platform comprising a plurality of reaction vessels. Examples of assay platforms include a multi-well plate or a microtitre plate.

In certain embodiments, the at least one solid substrate comprises one solid substrate. In certain embodiments, the at least one solid substrate comprises different solid substrates. In certain embodiments, the at least one solid substrate comprises at least two solid substrates. In some embodiments, the at least one solid substrates comprises at least three solid substrates. In certain embodiments, there is at least one solid substrate for every species of capture agent provided.

In certain embodiments, the at least one solid substrate is associated with all, a substantial portion or part of a reaction vessel. In certain embodiments, the solid substrate is all, substantially all or part of the reaction vessel. For example, the at least one solid substrate may be integral with substantially all, a substantial portion or part of the reaction vessel, the at least one solid substrate may form all, a substantial portion or part of the surface of the reaction vessel (such as the surface of a well of a microtitre plate), or may be attached to the reaction vessel. Other combinations are also contemplated.

In certain embodiments, the at least one solid substrate is separate to the reaction vessel. Again this may be a single reaction vessel if so desired. In certain embodiments, the at least one solid substrate is mobilisable and may be added to the reaction vessel. For example, in certain embodiments the at least one solid substrate is one or more beads, one or more suspendible particles, an affinity matrix, a resin, a gel, a slurry, a strip, or a dip stick. Combinations of different types of substrates, including the aforementioned substrates, are also contemplated. In certain embodiments, the at least one solid substrate is retained in the reaction vessel, such as being retained in a liquid holding and dispensing device, such as a tip used for pipetting. For example, in certain embodiments the at least one solid substrate may be one or more beads retained in a pipette tip. In this example, the sample and the at least two capture agents may, for example, be drawn into the tip and one or more of the capture agents immobilised on the solid substrate retained in the tip. In certain embodiments, the pipette tip comprises the at least one solid substrate. In certain embodiments, the at least one solid substrate comprises one or more beads and the one or more beads are retained in the pipette tip. In certain embodiments, one or more of the at least two capture agents may be pre-immobilised on the at least one solid substrate, for a example a pipette tip may comprise pre-immobilised capture agents

In certain embodiments, the at least one solid substrate comprises a plurality of beads. In certain embodiments, the beads are magnetic beads, beads including a dye, beads held in a pipette tip or a combination of one or more of the aforementioned. Methods for the use of beads, including magnetic beads and beads including dyes, are known.

In certain embodiments, the at least one solid substrate may be transparent, semitransparent, translucent, or coloured, depending whether the detection method involves a colorimetric, fluorescence and/or other forms of read outs.

In certain embodiments, detecting one or more different analytes in the sample comprises detecting the presence of the at least two different analytes bound to the at least one solid substrate via the at least two different capture agents immobilised on the at least one solid substrate in the reaction vessel.

In certain embodiments, the at least two different capture agents comprise at least three, at least four, at least five, at least six, at least seven, at least eight, at least nine, at least ten, at least eleven, or at least twelve different capture agents.

In certain embodiments, one or more of the at least two capture agents are not pre-bound or pre-immobilised to the at least one solid substrate. In certain embodiments, one or more of the at least two capture agents are pre-bound or pre-immobilised to the at least one solid substrate.

For example, in certain embodiments, one or more capture agents may be bound or immobilised on the at least one solid substrate prior to exposure to the sample and/or the at least two analytes and/or one or more other reagents. For example, one or more capture agents may be pre-bound or pre-immobilised on one or more beads retained in a pipette tip, and the sample and other reactants drawn into the tip.

In certain embodiments, one or more capture agents are not pre-bound or pre-immobilised on the at least one solid substrate.

Methods for passively binding or immobilising an agent to a solid substrate are known. Examples of such attachment include attachment via non-covalent interactions such as a hydrophilic interaction, a hydrophobic interaction, a charged (ionic) interaction, a van de Waals interaction, or combinations of such interactions. Examples of passive attachment include absorption to a solid substrate.

In certain embodiments, detecting two or more different analytes comprises two or more different capture agents and two or more different analytes forming a complex. In certain embodiments, at least two different capture agents and at least two different analytes form a complex.

In certain embodiments, detecting of two or more different analytes comprises binding of two or more complexes to at least one solid substrate to form one or more immobilised complexes.

In certain embodiments, the detecting of two or more different analytes comprises contacting the sample, two or more different capture agents and the least one solid substrate in the reaction vessel, to form a contacted mixture.

In certain embodiments, the components are brought into contact in the reaction vessel, to allow the formation of complexes between the two or more different capture agents and two or more of the different analytes, the complexes being able to be immobilised on the at least one solid substrate concurrently or after formation. As described herein, in certain embodiments this may provide advantages to the performance of certain methods and/or kits of the present disclosure.

In certain embodiments, one or more of the at least two different capture agents may be bound to the at least one solid substrate via a specific interaction with a binding partner on the at least one solid substrate.

In certain embodiments, one or more of the at least two different capture agents are not passively adsorbed or pre-coated on the at least one solid substrate. In certain embodiments, none of the at least two different capture agents is adsorbed or pre-coated on the at least one solid substrate.

Methods for covalently attaching an agent to a solid substrate are known. In certain embodiments, the at least one solid substrate does not comprise one or more linkers for covalent bonding of one or more of the at least two different capture agents to the at least one solid substrate.

In certain embodiments, one or more of the at the least two different capture agents are passively bound to the solid substrate and one or more of the remaining capture agents are actively bound to the solid substrate.

The at least one solid substrate comprises one or more immobilisation agents by which one or more of the at least two capture agents can be immobilised. For example, one or more immobilisation agents on the at least one solid substrate may be used to bind one or more of the at least two different capture agents, thereby immobilising one or more of the at least two different capture agents on the solid substrate. Methods for the attachment of an immobilisation agent to a solid substrate are known. In certain embodiments, the one or more immobilisation agents are bound to the at least one solid substrate by a covalent attachment to the solid substrate. Methods for covalently attaching an immobilisation agent to a solid substrate are known in the art.

In certain embodiments, the at least one solid substrate comprises a linker which facilitates covalent bonding of the one or more immobilisation agents to the at least one solid substrate. For example, the linker may comprise glutathione, maleic anhydride, a metal chelate, maleimide or combinations thereof. The immobilisation agent may then be bound to the at least one solid substrate via the linker.

In certain embodiments, the immobilisation agent is bound to the at least one solid substrate via a passive attachment. Methods for passively attaching an immobilisation agent to a solid substrate are known. Examples of such non-covalent or passive attachment include interactions such as a hydrophilic interaction, a hydrophobic interaction, a charged (ionic) interaction, a van de Waals interaction, or combinations of such interactions. For example, antibodies or streptavidin (and/or derivates thereof) may be passively bound to a hydrophobic solid substrate.

In certain embodiments, the reaction vessel comprises a bound immobilisation agent. For example, a reaction vessel may have an immobilisation agent bound to the reaction vessel. In certain embodiments, a reaction vessel comprises a bound immobilisation agent. In certain embodiments, an assay platform comprises a plurality of reaction vessels and at least one reaction vessel comprises a bound immobilisation agent.

Binding of one or more of the at least two capture agents to an immobilisation agent occurs via a specific ligand (associated with, or part of, the capture agent) binding to a specific receptor (associated with, or part of, the immobilisation agent).

In certain embodiments, the at least one solid substrate comprising one or more bound immobilisation agents may be treated with a blocking agent(s) that binds non-specifically to and substantially saturates binding sites to prevent or reduce unwanted binding of ligand(s) or other components to excess sites on a solid substrate. Examples of blocking agents include gelatin, BSA, egg albumin, casein, non-fat milk or combinations thereof.

In certain embodiments, a blocking agent is included during binding reactions. For example, the at least one solid substrate is treated with a blocking agent(s) prior to the addition of one or more of the at least two different capture agents. The at least one solid substrate may also be treated with a blocking agent(s) concurrent with the addition of one or more of the at least two different capture agents.

In certain embodiments, the at least one solid substrate comprises a substance that promotes binding of an immobilisation agent, and/or is treated to promote binding of an immobilisation agent. The at least one solid substrate may comprise a plastic surface including, for example, a polystyrene surface, a polyvinyl chloride surface, a cyclo-olefin surface or combinations thereof. In certain embodiments, the at least one solid substrate comprises a hydrophobic surface.

In certain embodiments, the at least one solid substrate is treated to increase the binding affinity of an immobilisation agent to the solid substrate. For example, the at least one solid substrate may be irradiated and/or functionalised to allow covalent bonding between the substrate and an immobilisation agent.

In certain embodiments, the binding capacity of the at least one solid substrate for the at least two different capture agents is 200 ng/ml or greater, 500 ng/ml, 1 ug/ml or greater, 2 ug/ml or greater, 3 ug/ml or greater, 4 ug/ml or greater, or 5 ug/ml or greater. In certain embodiments, the binding capacity is 5 ug/ml or less, 4 ug/ml or less, 3 ug/ml or less, 2 ug/ml or less, 1 ug/ml or less, 500 ng/ml or less or 200 ng/ml or less. For example, in certain embodiments where one or more of the at least two different capture agents is an antibody capture agent, the binding capacity of the at least one solid substrate is typically 2 ug/ml for a standard microtitre plate. In certain embodiments, the at least one solid substrate has a binding capacity of the aforementioned amounts for protein. In certain embodiments, the binding capacity of the least one solid substrate for the at least two different capture agents is 200 ng/ml to 500 ng/ml, 200 ng/ml to 1 ug/ml, 200 ng/ml to 2 ug/ml, 200 ng/ml to 3 ug/ml, 200 ng/ml to 4 ug/ml, 200 ng/ml to 5 ug/ml, 500 ng/ml to 1 ug/ml, 500 ng/ml to 2 ug/ml, 500 ng/ml to 3 ug/ml, 500 ng/ml to 4 ug/ml, 500 ng/ml to 5 ug/ml, 1 ug/ml to 2 ug/ml, 1 ug/ml to 3 ug/ml, 1 ug/ml to 4 ug/ml, 1 ug/ml to 5 ug/ml, 2 ug/ml to 3 ug/ml, 2 ug/ml to 5 ug/ml, 3 ug/ml to 4 ug/ml, 3 ug/ml to 5 ug/ml, or 4 ug/ml to 5ug/ml.

The use of one or more bound immobilisation agents may provide one or more advantages to certain embodiments of the methods and/or kits for detecting different analytes. For example, the use of one or more bound immobilisation agents on the at least one solid substrate may provide advantages to the flexibility in the selection of the substrate that may be used. For example, the immobilisation agent may allow a particular capture agent to bind to a solid substrate (via the immobilisation agent) to which it would otherwise not bind. In certain embodiments, the use of an immobilisation agent - ligand binding pair provides an advantage of allowing the method to be modular in that a range of capture agents may be produced that bind to a particular solid substrate by incorporation of a ligand for the immobilisation agent into a capture agent.

In certain embodiments, the use of an immobilisation agent - ligand pair to effect binding of a capture agent to the solid substrate may facilitate a more sensitive assay. Capture agents binding directly to a solid substrate may bind in a fashion that blocks the specific analyte binding site. The addition of a ligand to a capture agent that is specific to the immobilisation agent pre-bound to the solid substrate may allow the capture agent to bind to the solid substrate via the immobilisation in a way that does not diminish its affinity for the analyte, allowing a greater proportion of the capture agent to be available for the assay.

In certain embodiments, the use of one or more bound immobilisation agents may also diminish the potential influence of substrate-reactive proteins in the sample. For example, hydrophobic proteins in the sample are less likely to affect detection even if it is performed on a hydrophobic solid substrate, as the immobilisation agent is already bound to the solid substrate and a ligand on the capture agent may be specific for the immobilisation agent.

In certain embodiments, the at least one solid substrate comprises a single type of immobilisation agent. For example, one or more of the at least two different capture agents may bind to the same type of immobilisation agent. In certain embodiments, the at least one solid substrate comprises two or more different types of immobilisation agents. For example, one or more of the at least two different capture agents may comprise one type of ligand that binds to one type of immobilisation agent and one or more of the remaining capture agents may comprise another type of ligand that binds to another type of immobilisation agent.

One or more of the at least two different capture agents comprises, at a plurality of sites, a ligand for the immobilisation agent. In certain embodiments, each of the at least two capture agents comprises a plurality of ligands for the immobilisation agent.

In certain embodiments, an immobilisation agent and the ligand form a binding pair. A range of different immobilisation agent and ligand binding pairs may be used. In certain embodiments, the immobilisation agent and the ligand may be interchangeable (i.e. a first compound may be bound to the solid substrate or the capture agent and a second compound, which is part of the same binding pair, may be bound to the other).

In certain embodiments, an immobilisation agent and a ligand are a binding pair that is not a polyanionic-polycationic binding pair. In certain embodiments, the use of an immobilisation agent-ligand binding pair which do not bind substantially through an ionic interaction between a substantially polyanionic molecule and a substantially polycationic molecule may provide one or more advantages for detecting an analyte in a sample. Examples of such polyionic molecules include polymeric ionic substances. In certain embodiments, advantages of using a immobilisation agent - ligand binding pair that is not a polyanionic-polycationic binding pair include, for example, the binding between the pair of molecules may be less dependent upon the pH of the solution contacting the binding pair and/or the ability to reduce non-specific interactions may be difficult with such polyionic binding pairs. Furthermore, many proteins present in biological milieu are known to specifically bind either polyanions or polycations, making these components potentially difficult to detect with such an immobilisation system.

In addition, in certain embodiments the use of an immobilisation agent-ligand binding pair that is not a polyanionic-polycationic binding pair may provide other advantages including one or more of: (i) promoting the formation of a complex including the capture agent and the analyte and/or other agents; (ii) improving the access of such a complex to the solid substrate; and/or (iii) promote the ability of other agents, such as a detectable agent, to access the analyte for detection purposes.

The ligand and the immobilisation agent are a binding pair comprising a peptide-tag and an anti-peptide antibody.

According to the invention, a peptide tag is used as a ligand and an anti peptide antibody is used as an immobilisation agent (a peptide/antibody capture system). Such a system may provide one or more additional advantages.

For example, it has been determined that a peptide/antibody capture system may have advantage over a streptavidin/biotin capture system in some embodiments, as peptide/antibody capture systems may provide one or more of increased signal, reduced variability and reduced interference depending on the sample type.

In addition, in embodiments utilising a reduced number of washing steps of the solid substrate, a peptide/antibody capture system may provide an advantage, particularly in embodiments where the solid substrate is only washed after a complex has been immobilised. Further, in embodiments utilising a reduced time of the assay, a peptide/antibody capture system may also provide an advantage to assist in reducing assay time.

Other advantages of a peptide/antibody capture system are described herein. For example, specific peptides can be designed and prepared that are not naturally occurring, at least for the organism in which an analyte is to be detected. Bioinformatics may be used to select sequences that are unique.

Different peptides may also be selected for different applications or assays. As such, they are readily expandable if more than one affinity system is required. For example, in embodiments relating to the detection of an analyte in different wells of an assay plate, each well may be coated with a specific subset of anti-peptide antibodies which would allow the specific immobilisation of particular capture antibodies from a mixture of such antibodies with different peptide tags.

Further, in some embodiments the use of a peptide/antibody capture system may provide one or more advantages over other types of capture systems. For example, the use of a peptide/antibody system in certain embodiments may also provide an advantage over capture systems utilising poly-charged ligands (for example His tags) and metal ions (for example Ni²⁺ ions), as the peptide/antibody system may have greater affinity and/or be less likely to be affected by the presence of other charges molecules. Similarly, the use of a peptide/antibody system in certain embodiments may provide an advantage over glutathione/GST systems in that the peptide/antibody capture system also has greater affinity.

A peptide/antibody capture system may also provide in some embodiments one or more advantages over the use of anti-species antibodies as an immobilisation agent, since the system is then not restricted to the use of species of antibodies immobilised on the surface. For example, an anti-rabbit immobilised antibody can only be used to bind to rabbit capture antibodies. In addition, anti-species antibodies may suffer from reduced specificity to the species of antibody they are designed to bind, which may minimize their utility in assays using samples containing endogenous antibodies such as serum and plasma, as these will block the binding of assay antibodies.

Further, a peptide/antibody capture system may also provide in some embodiments one or more advantages over capture systems utilising immobilised protein A and/or protein G type capture systems. For example, proteins A and G will bind many antibodies in a solution. Protein A and protein G may also demonstrate reduced utility in samples containing endogenous antibodies, such as serum or plasma, as these may block binding of antibodies. In addition, such a capture system may have disadvantages in embodiments where both the capture agent and the detectable agent are antibodies, since Protein A or Protein G will not discriminate between the capture and detection antibodies, and will bind both, therefore eliminating the assay discrimination for analyte.

As described herein, peptide tags are polypeptide tags that can be conjugated to another molecule, such as a protein (eg an antibody) or added to a protein using recombinant DNA technology. One example of a peptide tag is the octapeptide DYKDDDDK (SEQ ID NO.1), otherwise referred to as a FLAG-tag, which can be used in different assays that utilize recognition by an antibody. Other examples of peptide tags are described herein. In certain embodiments, the peptide tag does not comprise a plurality of consecutive amino acids with the same charge.

A range of anti peptide tag antibodies may be obtained or produced by a person skilled in the art. For example, commercially available anti- DYKDDDDK (SEQ ID NO.1) antibodies are described herein. Commercially available anti- DYKDDDDK (SEQ ID NO.1) antibodies include Sigma-Aldrich product codes F7425, F3040, F1804, F3165, F4042, F2555 and SAB4200071. Some commercially available antibodies recognize the DYKDDDDK (SEQ ID NO.1) tag only in certain positions on a protein, for example exclusively N-terminal. However, other available antibodies are position-insensitive.

In certain embodiments, the peptide tag comprises a peptide derived from a member of a signalling pathway and/or a peptide from a cytokine. Peptide tags are as described herein, and the addition of a peptide tag to the capture agent to form a conjugate may be achieved by a suitable known method. Other peptide tags are contemplated. Peptide tags may be naturally occurring or non-naturally occurring. In certain embodiments, the peptide tag has greater than 75%, 80%, 85%, 90% or 95% sequence identity to a naturally occurring polypeptide sequence. In certain embodiments, the peptide tag has greater than 75%, 80%, 85%, 90% or 95% sequence homology to a naturally occurring polypeptide sequence. Methods for determining sequence identity and sequence homology are known.

In certain embodiments, the peptide tag comprise KRITVEEALAHPYLEQYYDPTDE (SEQ ID NO.2), being a sequence derived from the carboxy terminus of the human ERK proteins (ERK C-term peptide). Antibodies to this peptide tag may be produced by known methods.

Adding a peptide tag to a protein allows the protein to be bound and/or immobilised by an agent that binds the peptide tag, for example an antibody raised against the peptide tag sequence. In certain embodiments, a peptide tag may also be used in conjunction with other affinity tags for example a polyhistidine tag (His-tag), HA-tag or myc-tag.

In certain embodiments, the use of a peptide tag conjugated to a capture agent and an anti-peptide antibody (as the immobilization agent) may provide one or more advantages over other types of immobilization agent - ligand binding pairs. For example, in certain embodiments, peptide tag conjugates and anti-peptide antibodies may be less susceptible to variation over different sample types and/or provide improved sensitivity of detection.

As mentioned above, the ligand and one of the one or more immobilisation agents are a binding pair comprising a peptide-tag and an anti-peptide antibody. An additional ligand and one of the one or more immobilisation agents may be selected from the ligand-immobilisation agent binding pairs comprising: biotin (or derivatives thereof) and avidin or streptavidin (or derivates thereof); metal chelate (e.g. copper, nickel, cobalt) and Histidine (e.g. histidine tagged proteins); maleic anhydride and amine (e.g. amine containing proteins); and maleimide and sulfhydryls (e.g. sulfhydryl peptides) such that, for example, one or more of the ligands comprises biotin or a derivative thereof and one or more of the immobilisation agents comprises avidin or streptavidin, and/or a derivative thereof.

Derivatives of avidin or streptavidin are known and may include forms of avidin or streptavidin that have been modified to increase their binding affinity to modified and/or unmodified solid substrates or ligands. For example, streptavidin may be modified to add one or more amine groups, histidine residues or sulfhydryl groups to the molecule. In another example, the derivative of streptavidin may comprise neutravidin, captavidin or streptavidin mutants (e.g. H127C or S139C).

One or more of the at least two different capture agents comprises a plurality of ligands selected from binding pair comprising a peptide tag and an anti-peptide tag antibody.

In certain embodiments, a ligand may be part of a capture agent. For example, a capture agent may comprise histidine residues, amine groups or sulfhydryl groups that are able to bind to an immobilisation agent. In certain embodiments, a ligand is bound to a capture agent. For example, the ligand may be amine reactive, carbohydrate reactive, carboxyl reactive, or sulfhydryl reactive and thus may bind to the capture agent via primary amines (e.g. lysine or the N-terminus), carbohydrate modifications, carboxyl groups (e.g. on aspartic acid residues, glutamic acid residues and the C-terminus), or sulfhydryl groups. A ligand may also comprise iodinatable and/or photoactivatable groups. A ligand may also comprise tetrafluorophenyl azide (TFPA) groups that, once activated by UV light, are able to covalently bind at sites containing C-H or N-H bonds (e.g. the ligand may comprise TFPA-PEG3-Biotin). Methods for labelling proteins and other molecules with the above ligands are known.

In certain embodiments, one or more ligands comprise biotin or a derivative thereof, for example iminobiotin, D-desthiobiotin, DSB-X-biotin, biotin dimers or arylstannyl-biotin trimer. Biotin and derivatives thereof may be bound to the capture agent by biotinylation. Biotinylation reagents and methods for biotinylation of a target molecule are known. Biotinylation may comprise, for example, primary amine biotinylation, sulfhydryl biotinylation, carboxyl biotinylation, or glycoprotein biotinylation.

In certain embodiments, one or more ligands comprise a peptide tag, such as a DYKDDDDK (SEQ ID NO.1) tag. As described herein, peptide tags are polypeptide protein tags that can be conjugated to another molecule such as a protein. In certain embodiments, a peptide tag may be conjugated to an antibody or added to a protein using recombinant DNA technology. One example of a peptide conjugate tag is a DYKDDDDK (SEQ ID NO.1) tag, which can be used in different assays that utilize recognition by an antibody.

Other examples of peptide tags comprise a polypeptide sequence comprising one or more of the following sequences: HHHHHH (SEQ ID NO.3); EQKLISEEDL (SEQ ID NO.4); YPYDVPDYA (SEQ ID NO.5); YTDIEMNRLGK (SEQ ID NO.6); and QPELAPEDPED (SEQ ID NO.7).

Adding a peptide conjugate tag to a molecule, such as a protein, allows the molecule to be bound and/or immobilised by an antibody against the peptide tag sequence. In certain embodiments, a peptide tag may also be used in conjunction with other affinity tags.

As described herein, in certain embodiments the use of an immobilization agent - ligand binding pair system for detection of different analytes provides a number of advantages. For example, in certain embodiments the use of such system provides one or more of the following advantages: the ability to use reduced amounts of capture agent, the ability to increase the binding capacity of the solid substrate for capture agent; and providing a system that can use multiple types of antibodies without saturating the solid substrate.

As discussed herein, in certain embodiments one or more of the at least two different capture agents comprises a plurality of ligands for the immobilisation agent. In certain embodiments, the use of one or more capture agents comprising a plurality of ligands for the immobilisation agent may assist in the formation and/or detection of complexes between a capture agent, an analyte and other molecules. In addition, the use of a capture agent comprising a plurality of ligands may reduce the amount of a capture agent that binds to an immobilisation agent in an orientation that masks the analyte binding domain. In certain embodiments as a result of more efficient use of one or more of the at least two different capture agents, the amount of a capture agent that is required to detect an analyte may also be reduced relative to previous methods. In addition, more efficient use of a capture agent may also lead to a reduction in the area of solid substrate required to produce a given level detectable signal relative to previous assays.

Two or more of the at least two different capture agents comprises an antibody. Additional capture agents may comprise an aptamer, or either component of a receptor - ligand pair. Other combinations are also contemplated. Aptamers may be obtained commercially or generated by known methods. For example, the receptor may be a protein receptor and the ligand a molecule that binds to the receptor, or vice-versa.

Two or more of the at least two different capture agents comprise an antibody or a binding fragment thereof, the antibody being able to bind to an analyte.

Reference herein to an "antibody" means an immunoglobulin molecule with the ability to bind an antigenic region of another molecule, and includes monoclonal antibodies, polyclonal antibodies, multivalent antibodies, chimeric antibodies, multispecific antibodies, diabodies and fragments of an immunoglobulin molecule or combinations thereof that have the ability to bind to the antigenic region of another molecule with the desired affinity including a Fab, Fab', F(ab')2, Fv, a single-chain antibody (scFv) or a polypeptide that contains at least a portion of an immunoglobulin that is sufficient to confer specific antigen binding, such as a molecule including one or more CDRs. Antibodies to specific analytes may be obtained commercially or generated by known methods.

In certain embodiments where one or more of the at least two different capture agents comprises an antibody, the methods and kits of the present disclosure represent an immunoassay for detecting multiple analytes in a sample. In certain embodiments, the detecting of one or more different analytes comprises an immunoassay method, such as a non-competitive immunoassay or a competitive immunoassay. In certain embodiments, the immunoassay comprises a combination of both a non-competitive and a competitive immunoassay. In certain embodiments, each of the at least two different capture agents comprises an antibody.

In certain embodiments, the detection of the at least two different analytes comprises detection of the at least two different analytes in a reaction vessel followed by detection of one or more remaining different analytes in another reaction vessel.

In certain embodiments, the detecting of one or more of the different analytes comprises detecting two or more of the different analytes when the analytes are bound to the at least one solid substrate. In certain embodiments, the detecting of one or more of the different analytes comprises detecting two or more different analytes that are spatially separated when bound to the solid substrate. In certain embodiments, the spatial separation is achieved by spatial separation of immobilised capture agent(s). In certain embodiments, detecting of one or more of the different analytes does not comprise an immobilised capture agent at discrete sites.

In certain embodiments, the detecting of one or more of the different analytes comprises detecting one or more of the different analytes simultaneously or concurrently. In certain embodiments, the detecting of one or more of the different analytes comprises detecting one or more of the different analytes sequentially. In certain embodiments, the detecting of one or more of the at least two analytes occurs in a first reaction environment, followed by detection of further analytes in one or more other reaction environments.

In certain embodiments, the detecting of the one or more of the different analytes comprises reading of the various detectable signals by a machine or platform, including for example, plate readers, FACS machines, high content imagers, and monochromoters. In certain embodiments, the machine or platform is a plate reader. Examples of the types of readout include fluorescence, time-resolved fluorescence, luminescence, colourimetric, absorbance, and fluorescence polarisation. Other readouts are also contemplated. In certain embodiments, different fluorescent wavelengths are used to differentiate the detectable signals.

In certain embodiments, the detecting of one or more of the different analytes comprises one or more of immunological detection, luminescent detection, detection with a fluorophore, detection with a lanthanide ion and/or a complex thereof, detection with an enzyme that converts a substrate into a detectable product, and detection with a bead comprising a detectable dye.

In certain embodiments, the use of a method and/or kits that does not involve imaging, such as CCD imaging, may provide an advantage to detection of multiple analytes.

In certain embodiments, the detecting of one or more of the different analytes comprises detection using a product with an emission wavelength in one or more of following light ranges: 430-470 nm; 380 to 490 nm; 440 to 490 nm; 450 to 475 nm; 500 to 540 nm; 570 to 610 nm; 590 to 630 nm; 495 to 590 nm; 500 to 570 nm; 520 to 570 nm; 580 to 750 nm; 610 to 750 nm; 620 to 740 nm; 630 to 660 nm, and 595 to 750 nm; 495to 590 nm; 520 to 570 nm; 595 to 750 nm; and 610 to 750 nm.

In certain embodiments, the light has an emission wavelength in the range of blue light. In certain embodiments, the detecting of one or more of the different analytes comprises detection using a product with an emission wavelength in one or more of following ranges: 495 to 590 nm; 500-540 nm; 500 to 570 nm; 520 to 570 nm. In certain embodiments, the light has an emission wavelength in the range of green light. In certain embodiments, the detecting of one or more of the different analytes comprises detection using a product with an emission wavelength in one or more of following ranges: 570-610 nm; 590-630 nm; 580 to 750 nm; 610 to 750 nm; 620 to 740 nm. In certain embodiments, the light has an emission wavelength in the range of red light. In certain embodiments, the detecting of one or more of the different analytes comprises detection using a product with an excitation wavelength that differs from the emission wavelength by 100nm or less, 90 nm or less, 80 nm or less, 70 nm or less, 60 nm or less, or 50 nm or less.

In certain embodiments, the detecting of one or more of the different analytes comprises detection using a product with an emission wavelength in one or more of following ranges: 430-470 nm; 500-540 nm; 570-610 nm; and 590-630 nm.

In certain embodiments, detection comprises a substrate that produces a blue fluorescent product. For example, the substrate 4-Methylumbelliferyl phosphate (excitation wavelength 360 nm) produces the blue fluorescent product 7-hydroxy-4-methylcoumarin [4-Methylumbelliferone] (excitation wavelength 450 nm) with the enzyme alkaline phosphatase. In certain embodiments, detection comprises a substrate that produces a green fluorescent product. For example, the substrate Fluorescein di-beta-D-galactopyranoside (excitation wavelength 460 nm) produces the green fluorescent product Fluorescein (excitation wavelength 520 nm) with the enzyme Beta-galactosidase. In certain embodiments, detection comprises a substrate that produces a red fluorescent product. For example, the substrate 10-Acetyl-3,7-dihydroxyphenoxazine (excitation wavelength 550 nm) produces the red fluorescent product Resorufin (excitation wavelength 610 nm) with the enzyme horseradish peroxidase.

In certain embodiments, the detection of at least two different analytes comprises detection with a blue substrate/product with an emission wavelength of 430 to 470 nm and a red substrate/product with an emission wavelength of 570 to 610 nm.

In certain embodiments, the detecting of one or more of the different analytes comprises uses of one or more fluorophores. In certain embodiments, the one or more fluorophores comprises one or more lanthanide ions, such as one or more of Eu³⁺, Sm³⁺, Tb³⁺, and Dy³⁺.

In certain embodiments, the detecting of one or more of the different analytes comprises use of one or more enzymes that convert a substrate into a detectable product. For example, the one or more enzymes may be a peroxidase (eg horse radish peroxidase), an alkaline phosphatase, and beta-galactosidase.

In certain embodiments, the detecting of two or more of the different analytes comprises use of two or more agents that allow detection of two or more specific analytes bound to one or more specific capture agents. In certain embodiments, the detecting comprises contacting two or more agents with two or more different capture agents to produce two or more distinguishable detectable signals. For example, two different analytes immobilised on the at least one solid substrate may be contacted with a first agent that produces a first detectable signal associated with one of the analytes and contacted with a second agent that produces from the other analyte a second detectable signal distinguishable from the first detectable signal.

In certain embodiments, the at least two different analytes may be detected by sequentially contacting two or more agents with the at least two different analytes to produce one or more detectable signals. By way of illustration for example, two different analytes that are both detected using a red light fluorophore may be detected by sequentially contacting the at least two analytes with a first agent that produces a red detectable signal from one of the analytes. Following detection of this detectable signal and washing away of the first agent, contacting the at least two analytes with a second agent that produces a red detectable signal from the other analyte may then be used to detect the second analyte.

In certain embodiments, the method comprises production of at least two different detectable signals. In certain embodiments, the at least two detectable signals comprise one or more of a detectable signal selected from the detectable signals with a peak emission wavelength in the following ranges: 590-629 nm; 570-662 nm; 380-490 nm; 500-560 nm; and 570 to 750 nm.

In certain embodiments, the at least two different detectable signals comprise at least one detectable signal with a peak emission wavelength in the range from 500-560nm and another detectable signal with a peak emission wavelength in the range from 570-750 nm.

In certain embodiments, the at least two different detectable signals comprise at least one detectable signal with a peak emission wavelength in the range from 380-490 nm and another detectable signal with a peak emission wavelength in the range from 500-560.

In certain embodiments, the at least two different detectable signals comprise at least one detectable signal with a peak emission wavelength in the range from 380-490 nm and another detectable signal with a peak emission wavelength in the range from 570 to 750 nm.

In certain embodiments, the at least two different detectable signals comprise at least one detectable signal with a peak emission wavelength in the range from 500-560 nm and another detectable signal with a peak emission wavelength in the range from 570 to 750 nm.

In certain embodiments, the detecting of two or more of the different analytes comprises detecting the presence of two or more different analytes bound to the solid substrate via at least two different capture agents immobilised on the solid substrate (via an immobilisation agent).

One or more of the at least 2 different capture agents are immobilised on the at least one solid substrate via one or more immobilisation agents. In certain embodiments, one or more capture agents may be immobilised on the at least one solid substrate prior to exposure to the sample and/or the at least two analytes and/or one or more other reagents. In certain embodiments, one or more of the different capture agents may be pre-immobilised on the at least one solid substrate, such as being pre-immobilised on one or more beads retained in a pipette tip, and the sample and other reactants drawn into the tip. In certain embodiments, one or more capture agents are not pre-immobilised on the at least one solid substrate.

In certain embodiments, the detecting of one or more of the different analytes comprises detecting the presence of one or more of the different analytes bound to the solid substrate by a capture agent passively bound to the solid substrate. In certain embodiments, the detecting of one or more of the different analytes comprises detecting the presence of one or more of the analytes bound to the solid substrate by a capture agent actively bound to the solid substrate.

The detecting of two or more of the different analytes comprises detecting the presence of one or more of the analytes bound to the solid substrate by a capture agent immobilised on the solid substrate via the binding of one or more of ligands on a capture agent to an immobilisation agent on the at least one solid substrate.

For example, methods of detecting an analyte may comprise the analyte being brought into proximity with the solid substrate and/or the immobilisation agent. Examples of such methods involving proximity detection include energy transfer mechanisms, quenching mechanisms, and transfer of reactive molecules, between species that are brought into close proximity, which are known.

In certain embodiments, the methods and/or kits comprise a reduced amount of one or more of the at least two different capture agents, relative to previous assays, as a result of the more efficient binding of a capture to the solid substrate. For example, in certain embodiments the capture agent may be present at a concentration of 1000 ng/ml or less, 900 ng/ml or less, 800 ng/ml or less, 700 ng/ml or less, 600 ng/ml or less, 500 ng/ml or less, 400 ng/ml or less, 300 ng/ml or less, 200 ng/ml or less, 100 ng/ml or less, 50ng/ml or less, 25 ng/ml or less, or 10 ng/ml or less. In certain embodiments, the antibody capture agent may be present at a concentration of 10 ng/ml or greater, 25 ng/ml or greater, 50 ng/ml or greater, 100 ng/ml or greater, 200 ng/ml or greater, 300 ng/ml or greater, 400 ng/ml or greater, 500 ng/ml or greater, or 600 ng/ml greater, 700 ng/ml or greater, 800 ng/ml or greater, 900 ng/ml or greater, 1000 ng/ml or greater. In certain embodiments, the antibody detectable agent is present at a concentration of 10 ng/ml to 1000 ng/ml, 10 ng/ml to 900 ng/ml, 10 ng/ml to 800 ng/ml, 10 ng/ml to 700 ng/ml 10 ng/ml to 600 ng/ml, 10 ng/ml to 500 ng/ml, 10 ng/to 400 ng/ml, 10 ng/ml to 200 ng/ml, 10 ng/ml to 100 ng/ml, 25 ng/ml to 1000 ng/ml, 25 ng/ml to 900 ng/ml 25 ng/ml to 800 ng/ml, 25 ng/ml to 700 ng/ml, 25 ng/ml to 600 ng/ml, 25 ng/ml to 500 ng/ml, 25 ng/to 400 ng/ml, 25 ng/ml to 200 ng/ml, 25 ng/ml to 100 ng/ml, 50 ng/ml to 1000 ng/ml, 50 ng/ml to 900 ng/ml, 50 ng/ml to 800 ng/ml, 50 ng/ml to 700 ng/ml, 50 ng/ml to 600 ng/ml, 50 ng/ml to 500 ng/ml, 50 ng/to 400 ng/ml, 50 ng/ml to 200 ng/ml,
or 50 ng/ml to 100 ng/ml. For example, in an anti-peptide conjugate system, an antibody capture agent may be typically used at a concentration of 50 ng/ml, and in a streptavidin - biotin system an antibody capture agent may typically be used at a concentration of 200 ng/ml. In certain embodiments, the methods and/or kits comprises providing one or more of the different capture agents at one of the abovementioned concentrations. In certain embodiments, the methods and/or kits comprises providing one or more of the at least two different capture agents at a concentration of 10 ng/ml to 500 ng/ml.

In certain embodiments, a detectable agent is used to detect an analyte bound to a solid substrate via a capture agent. In such embodiments, detecting the presence of the two or more different analytes bound to the solid substrate comprises detecting two or more of the different analytes with one or more different detectable agents.

In certain embodiments, the detecting of two or more different analytes comprises providing two or more different detectable agents that are able to detect one or more different analytes. In certain embodiments, the detecting of at least two different analytes comprises providing at least two different detectable agents.

In certain embodiments, at least two different detectable agents bind to at least two different analytes. In certain embodiments, each of the at least two different detectable agents bind to a different analyte.

In certain embodiments, detecting two or more of the different analytes comprises detecting the presence of two or more detectable agents bound to the at least one solid substrate. The binding to the at least one solid substrate is via two or more analytes.

In certain embodiments, detecting at least two different analytes comprises detecting the presence of at least two detectable agents bound to the solid substrate via one or more analytes.

In certain embodiments, a detectable agent comprises a molecule that binds to the analyte. In certain embodiments, a different detectable agent binds to a different analyte. In certain embodiments, different detectable agents binds to the same analytes or to multiple analytes.

In certain embodiments, a detectable agent comprises one or more of an antibody, an aptamer, or either component of a receptor - ligand pair, suitable binding fragments of the aforementioned or combinations thereof.

In certain embodiments, one or more of the at least two different detectable agents comprises an antibody or a fragment thereof. In certain embodiments, each of the at least two different detectable agents comprises an antibody or a fragment thereof. Antibodies are as described herein. Antibodies may be obtained commercially or generated by known methods.

In certain embodiments, detecting the at least two different analytes comprises detecting the presence of at least two detectable agents bound to the at least one solid substrate via the at least two different analytes, which are in turn bound to the at least two different capture agents immobilised on the at least one solid substrate via at least one immobilisation agent. This may be in a single reaction vessel.

In certain embodiments, the at least two different capture agents and the at least two different detectable agents form at least two pairs of agents that bind to the least two different analytes.

In certain embodiments, the detecting of two or more of the different analytes comprises detecting two or more of the detectable agents simultaneously or concurrently. In certain embodiments, the detecting of two or more of the different analytes comprises detecting two or more of the different detectable agents sequentially. In certain embodiments, the detecting of one or more of the at least two analytes occurs in a first reaction environment, followed by detection of further analytes in one or more other reaction environments.

In certain embodiments, the methods and/or kits comprise a reduced amount of one or more different detectable agents, relative to previous assays, as a result of the more efficient capture of an analyte to a solid substrate. For example, in certain embodiments the detectable agents are present at a concentration of 1000 ng/ml or less, 900 ng/ml or less, 800 ng/ml or less, 700 ng/ml or less, 600 ng/ml or less, 500 ng/ml or less, 400 ng/ml or less, 300 ng/ml or less, 200 ng/ml or less, 100 ng/ml or less, 50ng/ml or less, 25 ng/ml or less, or 10 ng/ml or less. In certain embodiments, the detectable agents are present at a concentration of 10 ng/ml or greater, 25 ng/ml or greater, 50 ng/ml or greater, 100 ng/ml or greater, 200 ng/ml or greater, 300 ng/ml or greater, 400 ng/ml or greater, 500 ng/ml or greater, or 600 ng/ml greater, 700 ng/ml or greater, 800 ng/ml or greater, 900 ng/ml or greater, 1000 ng/ml or greater. In certain embodiments, the detectable agents are present at a concentration of 10 ng/ml to 1000 ng/ml, 10 ng/ml to 900 ng/ml, 10 ng/ml to 800 ng/ml, 10 ng/ml to 700 ng/ml 10 ng/ml to 600 ng/ml, 10 ng/ml to 500 ng/ml, 10 ng/to 400 ng/ml, 10 ng/ml to 200 ng/ml, 10 ng/ml to 100 ng/ml, 25 ng/ml to 1000 ng/ml, 25 ng/ml to 900 ng/ml 25 ng/ml to 800 ng/ml, 25 ng/ml to 700 ng/ml, 25 ng/ml to 600 ng/ml, 25 ng/ml to 500 ng/ml, 25 ng/to 400 ng/ml, 25 ng/ml to 200 ng/ml, 25 ng/ml to 100 ng/ml, 50 ng/ml to 1000 ng/ml, 50 ng/ml to 900 ng/ml, 50 ng/ml to 800 ng/ml, 50 ng/ml to 700 ng/ml, 50 ng/ml to 600 ng/ml, 50 ng/ml to 500 ng/ml, 50 ng/to 400 ng/ml, 50 ng/ml to 200 ng/ml, or 50 ng/ml to 100 ng/ml. For example, in a peptide -conjugate system or a biotin-streptavidin system, an antibody detectable agent may be typically used at a concentration of 50 ng/ml. In certain embodiments, the methods and/or kits comprises providing one or more of the different detectable agents at one of the aforementioned concentrations. In certain embodiments, the methods and/or kits comprise providing at least two different detectable agents at one or more of the aforementioned concentrations. In certain embodiments, the methods and/or kits comprises providing one or more of the different detectable agents at a concentration of 10 ng/ml to 100 ng/ml. In certain embodiments, the methods and/or kits comprises providing the at least two different detectable agents at a concentration of 10 ng/ml to 100 ng/ml.

In certain embodiments, at least two detectable agents produce at least two different detectable signals. In certain embodiments, the at least two different detectable signals comprise at least one detectable signal with a peak emission wavelength in the range from 570-629 nm and another detectable signal with a peak emission wavelength in the range from 630-662. In certain embodiments, the at least two different detectable signals comprise at least one detectable signal with a peak emission wavelength in the range from 380-490 nm and another detectable signal with a peak emission wavelength in the range from 500-560. In certain embodiments, the at least two different detectable signals comprise at least one detectable signal with a peak emission wavelength in the range from 380-490 nm and another detectable signal with a peak emission wavelength in the range from 570 to 750 nm. In certain embodiments, the at least two different detectable signals comprise at least one detectable signal with a peak emission wavelength in the range from 500-560 nm and another detectable signal with a peak emission wavelength in the range from 570 to 750 nm.

In certain embodiments, at least two detectable agents produce at least two different detectable signals. In certain embodiments, the at least two different detectable signals comprise at least one detectable signal with a peak emission wavelength in one or more of following light ranges: 430-470 nm; 380 to 490 nm; 440 to 490 nm; 450 to 475 nm; 500 to 540 nm; 570 to 610 nm; 590 to 630 nm; 495 to 590 nm; 500 to 570 nm; 520 to 570 nm; 580 to 750 nm; 610 to 750 nm; 620 to 740 nm; 630 to 660 nm, and 595 to 750 nm; 495to 590 nm; 520 to 570 nm; 595 to 750 nm; and 610 to 750 nm.

In certain embodiments, a detectable tag is associated with a detectable agent, for example bound to the detectable agent, or is a part of the detectable agent, for example the detectable agent may include the detectable tag as a fusion partner, a labelled amino acid or labelled nucleotide. Examples of suitable detectable tags include antigens, enzymes, fluorophores, quenchers, radioactive isotopes and luminescent compounds or labels. The detectable tag may be detected directly or indirectly via a further molecule that can produce a detectable signal. In certain embodiments, different detectable tags produce different detectable signals. In certain embodiments, at least two detectable tags produce at least two different detectable signals.

In certain embodiments, one or more detectable agents comprise an antigen. Examples of antigens that may be used as a detectable tag include suitable antigenic components of the detectable agent that may be targeted by a secondary detectable agent. For example, a secondary antibody may also be used to detect an antigen on a detectable agent. The secondary antibody may, for example, be fluorescently or enzymatically labelled. In embodiments where the detectable agent is a primary antibody (ie. an analyte binding antibody), the secondary antibody may have binding affinity to an antigen on the primary antibody. For example, the antigen may be derived from the host in which the detectable agent was raised.

In certain embodiments, the one or more detectable tags comprise a fluorophore. Examples of fluorophores that may be used as detectable tags include, for example, resorufin, fluorescein (fluorescein isothiocyanate, FITC), rhodamine (tetramethyl rhodamine isothiocyanate, TRITC), green fluorescent protein (GFP), phycobiliproteins (allophycocyanin, phycocyanin, phycoerythrin and phycoerythrocyanin, lanthanide ions such as one or more of Eu3+, Sm3+, Tb3+, and Dy3+ or complexes thereof, suitable derivatives of the foregoing or combinations thereof. In certain embodiments, the one or more detectable tags may be part of the one or more different detectable agents (e.g. in the form of a fusion protein or a protein comprising fluorescent amino acids). Fluorophores may be subjected to applied stimulation (for example light of a suitable excitation wavelength) to promote fluorescence.

In certain embodiments, the one or more detectable tags comprise a luminescent compound or label. Luminescent compounds or labels that may be used as detectable tags include, for example, chemiluminescent and/or bioluminescent compounds. These compounds may be used to label a detectable agent. The presence of a chemiluminescenttag may be determined by detecting the presence of luminescence that arises during the course of a chemical reaction. Examples of useful chemiluminescent labelling compounds are luminol, isoluminol, theromatic acridinium ester, imidazole, acridinium salt and oxalate ester or combinations thereof. Bioluminescence is a type of chemiluminescence found in biological systems in which a catalytic protein increases the efficiency of the chemiluminescent reaction. The presence of a bioluminescent antibody is determined by detecting the presence of luminescence. Examples of bioluminescent compounds include luciferin, luciferase and aequorin.

In certain embodiments, the one or more detectable tags comprise one or more lanthanide ions. In certain embodiments, the lanthanide ion may be Eu³⁺, Sm³⁺, Tb³⁺, and Dy³⁺. In certain embodiments, one of the detectable tags comprises Eu³⁺ and another of the detectable tags comprises Sm³⁺.In certain embodiments, one of the detectable tags comprises Eu³⁺, another of the detectable tags comprises Sm³⁺ and a further detectable tag comprises Tb³⁺,

In certain embodiments, the one or more detectable tags comprise one or more enzymes that converts a substrate into a detectable product. Enzymes that may be used as detectable tags include, for example, enzymes that result in the conversion of a substrate into a detectable product (generally resulting in a change in colour or fluorescence or generation of an electrochemical signal). Such enzymes may include, for example, horseradish peroxidase (HRP), alkaline phosphatase (AP), β-galactosidase, acetylcholinesterase, luciferase, catalase or combinations thereof. Depending on the enzyme and substrate used, detection may be performed with a spectrophotometer, fluorometer, luminometer, and/or other electrochemical detection means. In certain embodiments, the enzyme may be horse radish peroxidase, alkaline phosphatase, and beta-galactosidase. Other enzymes may also be used. In certain embodiments, one of the detectable tags comprises horse radish peroxidise and another of the detectable tags comprises alkaline phosphatase. In certain embodiments one of the detectable tags comprises horse radish peroxidase, another of the detectable tags comprises alkaline phosphatase, and a further detectable tag comprises beta-galactosidase.

In certain embodiments, one of the detectable tags comprises an enzyme pair selected from horse radish peroxidise and alkaline phosphatise, horse radish peroxidise and beta-galactosidase, and alkaline phoshatase and beta-galactosidase.

In certain embodiments, one or more of the detectable tags comprises a radioactive isotope, Radioactive isotopes that may be used as detectable tags include, for example, ³H, ¹⁴C, ³²P, ³⁵S, or ¹³¹I. The radioisotope may be conjugated to a detectable agent or incorporated into a detectable agent by translation of mRNA encoding the detectable agent in the presence of radiolabelled amino acids. Radioisotopes and methods for conjugating radioactive isotopes to molecules such as proteins are known. Radioisotopes may be detected using gamma, beta or scintillation counters.

In certain embodiments, the at least two different analytes may be detected by the at least two different detectable agents producing one or more detectable signals. In certain embodiments, the at least two different analytes may be detected by contacting one or more agents with the at least two different detectable agents to produce one or more detectable signals. In certain embodiments, the detectable signals may be substantially distinguishable. In certain embodiments, the detectable signals may be different.

In certain embodiments, the detectable signals may not be substantially distinguishable, and detecting of the at least two analytes comprises sequentially producing the detectable signals. In certain embodiments, the at least two different analytes may be detected by sequentially contacting one or more agents with the at least two different detectable agents to produce one or more detectable signals. By way of illustration for example, at least three different analytes that are all detected using a red light fluorophore may be detected by sequentially contacting the at least three analytes with a first agent that produces a red detectable signal from one of the analytes. Following detection of this detectable signal and washing away of the first agent, contacting the remaining analytes with a second agent that produces a red detectable signal from another analyte may then be used to detect the second analyte. Following detection of this second detectable signal and washing away of the second agent, contacting the remaining analyte with a third agent that produces a red detectable signal from this remaining analyte may then be used to detect the third analyte.

In certain embodiments, one or more of the different capture agents is provided in solution. In certain embodiments, the at least two different capture agents are provided in solution. In certain embodiments, each of the at least two different capture agents is provided in solution. In certain embodiments, use of one or more capture agents and/or one or more detectable agents in solution provides an advantage to detection of multiple analytes as it permits use of lower amounts of capture agent and/or detectable agent. In certain embodiments, this also permits multiple capture agents to bind to the solid substrate simultaneously, providing advantages to detection of multiple analytes, such as the efficiency of detection and/or reduce detection times.

The use of a capture agent in solution in certain embodiments provides one or more advantages. For example, the use of a capture agent in solution in certain embodiments may promote the binding of a capture agent to an analyte and thereby promote the formation of a complex of the capture agent and the analyte. In certain embodiments where a detectable agent is used and which binds to the analyte, the use of a capture agent in solution in certain embodiments promotes the binding of a capture agent to an analyte and thereby promotes the formation of a complex of a capture agent, an analyte and a detectable agent.

In this regard, it will be understood that in certain embodiments two or more different capture agents may be provided in solution prior to contacting the two or more different capture agents with the sample. Accordingly, in some embodiments two or more different capture agents are provided in a form where the two or more different capture agents are not immobilised to the least one solid substrate and provided in a substantially liquid state.

In certain embodiments, the use of two or more different capture agents in solution also reduces the amount of a capture agent that needs to be used as compared to previous assays, as more capture agent is required in assays when the capture agent is pre-adsorbed onto a solid substrate. In certain embodiments there is no pre-immobilisation or pre-adsorption of a capture agent on the at least one solid substrate.

As described herein, in certain embodiments, the at least one solid substrate does not comprise one or more pre-immobilised capture agents. However, in some embodiments, if desired, the at least one solid substrate may comprise one or more pre-immobilised capture agents immbobilised via one or more immobilisation agents.

In certain embodiments, one or more of the different detectable agents are provided in solution. Accordingly, in some embodiments one or more of the different detectable agents are provided in a form not immobilised to a solid substrate and provided in a substantially liquid state. In certain embodiments, the at least two different detectable agents are provided in solution. In certain embodiments, each of the at least two different detectable agents is provided in solution.

In certain embodiments, the use of one or more detectable agents in solution may provide one or more advantages, including promoting the formation and detection of a complex of a capture agent, an analyte and a detectable agent. In certain embodiments the methods and/or kits may promote the formation of a complex between a capture agent, an analyte and a detectable agent before or concurrent with contacting the complex with the solid substrate, which may prevent or inhibit binding of a capture agent to the solid substrate in an orientation which is not amenable to analyte binding. Thus, a greater proportion of a capture agent used may be available for analyte binding.

In certain embodiments, detecting two or more different analytes comprises a capture agent, an analyte and a detectable agent forming a complex. In certain embodiments, detecting of two or more different analytes comprises binding of one or more complexes (via the ligand) to the at least one solid substrate to form an immobilised complex. Thus, in certain embodiments a complex is immobilised on the solid substrate.

In certain embodiments, the detecting of two or more different analytes comprises contacting the sample, two or more different capture agents, two or more different detectable agents and the least one solid substrate in the reaction vessel, to form a contacted mixture.

In certain embodiments, the components are brought into contact in the reaction vessel, to allow the formation of complexes between the two or more different capture agents two or more of the different analytes and two or more different detectable agents, the complexes being able to be immobilised on the at least one solid substrate concurrently or after formation. As described herein, in certain embodiments this may provide advantages to the performance of certain methods and/or kits of the present disclosure.

As described herein, in certain embodiments, the detecting of the different analytes comprises contacting the sample, the at least two capture agents, the at least two detectable agents and the at least one solid substrate in the reaction vessel, to form a contacted mixture. In certain embodiments, the contacted mixture is mixed. In certain embodiments, mixing the contacted mixture may provide an increase in binding efficiency. In certain embodiments, the method comprises mixing the contacted mixture. In certain embodiments, the contacted mixture is not mixed. In certain embodiments, the method comprises not mixing the contacted mixture.

In certain embodiments, the components are brought into contact in the reaction vessel, to allow the formation of a complex between a capture agent, an analyte and a detectable agent, the complex being able to be immobilised on the solid substrate (concurrently or after formation). As described herein, in certain embodiments this may provide advantages to the performance of certain methods and/or kits of the present disclosure.

As described herein, in certain embodiments contacting the sample, two or more different capture agents, two or more different detectable agents and the at least one solid substrate in the reaction vessel allows binding of the two or more different capture agents and the two or more different detectable agents to one or more different analytes to form two or more complexes. Upon formation, the one or more complexes may be immobilized on the at least one solid substrate.

In certain embodiments, prior to bringing the components into contact in the reaction vessel, specific individual components may be brought into contact prior with each other. In certain embodiments, contacting of one or more of the individual components may occur in the reaction vessel or may occur in a separate reaction vessel.

In certain embodiments, the sample, each of the different capture agents, each of the detectable agents and the solid substrate are not contacted in a separate vessel prior to contacting in the reaction vessel. Thus, the combination of the components is contacted together for the first time in the reaction vessel.

In certain embodiments, the sample and the at least one solid substrate are contacted prior to contacting with the two or more different capture agents and/or two or more different detectable agents. As discussed herein, in certain embodiments this particular method of contacting provides one or more advantages to the performance of some of the methods and/or kits of the present disclosure, as one or more of the different capture agents and/or one or more of the different detectable agents are exposed to the analyte in the presence of the solid substrate.

In certain embodiments, the sample and the at least one solid substrate are contacted prior to contacting with two or more of the different capture agents and/or two or more of the different detectable agents.

In certain embodiments, the sample and the at least one solid substrate are contacted concurrently with two or more different capture agents and/or two or more different detectable agents. In certain embodiments, the sample and the at least one solid substrate are first contacted in the reaction vessel.

In certain embodiments where the at least one solid substrate forms part of the reaction vessel, the sample may be added to the reaction vessel and subsequently two or more of the different capture agents and/or two or more of the different detectable agents are brought into contact with the sample and the solid substrate. In certain embodiments, two or more of the different capture agents are pre-immobilised on the at least one solid substrate (via at least one immobilisation agent) and subsequently contacted with the sample and/or two or more different detectable agents.

In certain embodiments, the sample and the at least one solid substrate are not substantially incubated prior to contacting with two or more of the different capture agents and/or two or more of the different detectable agents. In certain embodiments, this may provide an advantage by reducing the time required to detect two or more of the different analytes.

In certain embodiments, two or more of the different capture agents and two or more of the different detectable agents are brought into contact with each other before they are contacted with either or both of the sample and the at least one solid substrate. Typically, this may be achieved by first contacting two or more of the different capture agents and two or more of the different detectable agents in a separate vessel. In certain embodiments, specific pairs of one or more of the different capture agents and one or more of the different detectable agents are first contacted in a separate vessel.

In certain embodiments, two or more of the different capture agents and/or two or more of the different detectable agent are sequentially contacted with the previously contacted sample and/or the at least one solid substrate.

In certain embodiments, the methods and/kits may utilise a standard ELISA protocol. For example, in certain embodiments, the method comprises providing the capture agent and the sample to the reaction vessel, washing the solid substrate and subsequently providing the detectable agent to the reaction vessel.

In certain embodiments, complexes comprising an analyte, a capture and a detectable agent are formed prior to binding between the immobilisation agent and the ligand. The complexes may be formed by sequential or concurrent addition of a capture agent and a detectable agent to an analyte prior to contacting the complex with an immobilisation agent on the at least one solid substrate.

In certain embodiments, the detecting of the different analytes comprises an incubation of the contacted sample, the solid substrate, two or more of the different capture agents and/or two or more of the different detectable agents. In certain embodiments, the incubation is for a time of 2 hours or less, 90 minutes or less, 80 minutes or less, 70 minutes or less, 60 minutes or less, 50 minutes or less, 40 minutes or less, 30 minutes or less, or 15 minutes or less. In certain embodiments, the incubation is for a time of 2 hours greater, 90 minutes or greater, 80 minutes or greater, 70 minutes or greater, 60 minutes or greater, 50 minutes or greater, 40 minutes or greater, 30 minutes or greater, or 15 minutes or greater. In certain embodiments, the incubation is between 10 minutes to 2 hours, 10 minutes to 1 hour, 15 minutes to 2 hours, 15 minutes to 1 hour, 30 minutes to 2 hours, 30 minutes to 1 hour, or 1 hour to 2 hours.

Generally, the detecting of the different analytes may comprise washing the at least one solid substrate in the reaction vessel to remove one or more different capture agents and/or one or more different detectable agents not bound to the at least one solid substrate. The washing may be performed, for example, after contacting the at least one solid substrate with each capture agent and/or each detectable agent, after contacting the at least solid substrate with all the capture agents and/or detectable agents, at any intermediate step, or a combination of one or more of the aforementioned in the protocol.

In certain embodiments, the detecting of the different analytes comprises no additional washing of the at least solid substrate after contacting of the solid substrate with one or more of the samples, one or more of the different capture agents and/or one or more of the different detectable agents.

For example, in embodiments utilising a peptide tag/antibody capture system may assist in reducing the steps involving washing of the solid substrate.

In certain embodiments, there is an incubation of the contacted sample, the solid substrate, one or more of the different capture agents and/or one or more of the different detectable agents prior to washing of the solid substrate. In certain embodiments, the incubation is for a time of 2 hours or less, 90 minutes or less, 80 minutes or less, 70 minutes or less, 60 minutes or less, 50 minutes or less, 40 minutes or less, 30 minutes or less, 15 minutes or less, or 10 minutes or less. In certain embodiments, the incubation is for a time of 2 hours greater, 90 minutes or greater, 80 minutes or greater, 70 minutes or greater, 60 minutes or greater, 50 minutes or greater, 40 minutes or greater, 30 minutes or greater, 15 minutes or greater or 10 minutes or greater. In certain embodiments, the incubation is between 10 minutes to 2 hours, 10 minutes to 1 hour, 15 minutes to 2 hours, 15 minutes to 1 hour, 30 minutes to 2 hours, 30 minutes to 1 hour, or 1 hour to 2 hours.

In certain embodiments, the detecting of two or more different analytes comprises washing the at least one solid substrate in the reaction vessel, in order to remove one or more capture agents and/or one or more detectable agents not bound to the at least one solid substrate.

The washing of the at least one solid substrate may be performed using a suitable method sufficient to remove desired capture agents and/or desired detectable agents not bound to the at least one solid substrate.

In certain embodiments, washing the at least one solid substrate prior to detection of one or more detectable agents allows the removal of unbound detectable agents and/or detectable agents not bound via a capture agent, which can decrease the level of background signal and hence improve sensitivity. Methods for washing steps are known in the art and generally involve repeated addition and removal of buffer.

The at least one solid substrate may be washed one or more times, and with one or more buffers. In certain embodiments, the at least one solid substrate may be washed two or more times, and with one or more buffers. In some embodiments, the at least one solid substrate may be washed three or more times, and with one or more buffers.

As described herein, but not embodied in the invention, there is no additional washing of the at least one solid substrate after contacting of the at least one solid substrate with one or more of the samples, one or more different capture agents and/or one or more different detectable agents. As described herein, in embodiments utilising a peptide/antibody capture system , such systems may assist in reducing the steps involving washing of the solid substrate.

In certain embodiments, if desired, additional washing of the at least solid substrate may be conducted. In certain embodiments, one, two, three or four washings of the at least one solid substrate may be conducted.

In certain embodiments, the detecting of the different analytes may be performed using only a single/one wash step conducted during the entire method. The use of a one wash protocol may provide one or more advantages. For example, in certain embodiments the use of a one wash protocol may provide an advantage to the number of handling steps involved and the time required to conduct the detection, and may also provide an improvement in the efficiency and performance of the method.

However, in certain embodiments, the one wash protocol may be varied, if desired, to add additional quick washes or rinses at various stages of the protocol, or to add additional washes or rinses at various stages of the protocol. For example, the solid substrate may be washed before contacting with sample, washed before contacting with a capture agent, washed after contacting with a capture agent, washed before contacting with a detectable agent, washed after contacting with a detectable agent, washed before an incubation step, washed after an incubation step, washed before detection, washed after detection, washed before addition of a substrate to a detectable agent, washed after addition of a substrate to a detectable agent or a combination of one or more of these washing steps. In certain embodiments, the reaction vessel comprises a device such as pipette tip comprising the at least one solid substrate, and washes performed during the protocol occur in the tip, for example by drawing liquid up and down one or more times into the tip.

In certain embodiments, there is no additional washing of the at least one solid substrate after contacting of the at least one solid substrate with one or more of the sample, one or more different capture agents and/or one or more different detectable agents.

In certain embodiments, the detecting of the two or more different analytes is achieved in a time of 2 hours or less, 90 minutes or less, 80 minutes or less, 70 minutes or less, 60 minutes or less, 50 minutes or less, 40 minutes or less, 30 minutes or less, 20 minutes or less, or 15 minutes or less from contacting the sample with two or more of different capture agents and/or two or more different detectable agents. In certain embodiments, the detecting of on two e or more different analytes is achieved in a time of 2 hours or greater, 90 minutes or greater, 80 minutes or greater, 70 minutes or greater, 60 minutes or greater, 50 minutes or greater, 40 minutes or greater, 30 minutes or greater, 20 minutes or greater, or 15 minutes or greater from contacting the sample with two or more different capture agents and/or two or more different detectable agents. In certain embodiments, the incubation is between 15 minutes to 2 hours, 15 minutes to 1 hour, 30 minutes to 2 hours, 30 minutes to 1 hour, or 1 hour to 2 hours.

Many assays for detecting multiple analytes can take over 6 hours to complete and may utilises multiple separate incubation and washing steps throughout the protocol. In certain embodiments of the methods and/or kits of the present disclosure, detecting of the different analytes may be performed in a shorter time and/or with fewer washes. In certain embodiments, this may result in a reduction in handling steps, which may allow a reduction in common sources of variation that are introduced by multiple handling steps, plate washing, and extra pipetting steps.

In certain embodiments, a reduced number of washes may allow the detection to be performed in a simpler and more time-efficient manner. Furthermore, in certain embodiments, the reduced number of washes allows for capture agents and/or detectable agents that may have a lower binding affinity to the analyte.

For example, in certain embodiments the reduced number of washes allows the detection to use antibodies (as capture agents and/or detectable agents) that may have a low or lower, binding affinity to the analyte.

In certain embodiments, the two or more different capture agents and/or the two or more detectable agents have a Kd for binding with the analyte of greater than 10⁻⁶M. In further embodiments, a Kd for binding is greater than 10⁻⁷M, 10⁻⁸M or 10⁻⁹M. In certain embodiments, the Kd is in the range from 10⁻⁸M to 10⁻¹²M.

In certain embodiments the detecting of the different analytes comprises detecting an analyte by detecting the presence of a detectable agent bound to the solid substrate in the reaction vessel.

In certain embodiments the detection of an analyte is achieved by detecting the presence of a detectable agent present in a complex immobilised to the at least one solid substrate in the reaction vessel. The detection of an analyte by detecting the presence of a detectable agent bound to the at least one solid substrate may be achieved by a suitable method. Examples of detectable agents are disclosed herein.

As described herein, the time taken to perform a method for detecting the different analytes may be important consideration. In certain embodiments the present disclosure minimises the number of incubation, handling and/or washing steps. In certain embodiments, this may make the detecting of the different analytes amenable to automation. Previous assays are difficult to automate as multiple handling steps are needed, including several aspiration, dispensing, and washing steps.

In certain embodiments, reducing the number of incubation steps and/or washing steps that are required may allow the duration of the binding step of a complex to solid substrate to be maximised without increasing the total duration of the method. This may also increase the sensitivity of the methods and/or kits. Certain embodiments of the present disclosure contemplate, if desired, various combinations as to the number of incubation, handling and/or washing steps.

In some embodiments, the detection of the different analytes is achieved in a time of 2 hours or less from contacting the sample with the different capture agents and/or the different detectable agents. In certain embodiments, the detection of the different analytes is achieved in a time of 90 minutes or less, 70 minutes or less, 60 minutes or less, 50 minutes or less, 45 minutes or less, 40 minutes or less, 30 minutes or less, 20 minutes or less, 15 minutes or less, or 10 minutes or less from contacting the sample with the capture agents and/or the detectable agents. In some embodiments, the detection of the analyte is achieved in a time period of between 10 minutes to 120 minutes, 10 minutes to 90 minutes, 10 minutes to 60 minutes, 15 minutes to 120 minutes, 15 minutes to 90 minutes, 15 minutes to 60 minutes, 15 minutes to 30 minutes, 30 minutes to 120 minutes, 30 minutes to 90 minutes, 30 minutes to 60 minutes, 45 minutes to 120 minutes, 45 to 90 minutes, or 45 minutes to 60 minutes from contacting the sample with the capture agents and/or the detectable agents. In certain embodiments, the detection of the different analytes is achieved in a time of less than 60 minutes from contacting the sample with the capture agents and/or the detectable agents. In certain embodiments, the detection of the different analytes is achieved in a time of less than 30 minutes from contacting the sample with the different capture agents and/or the different detectable agents.

In certain embodiments, the detection of the different analytes is achieved in a time of 2 hours or less, 90 minutes or less, 80 minutes or less, 70 minutes or less, 1 hour or less, 45 minutes or less, 30 minutes or less, 20 minutes or less, 15 minutes or less, 10 minutes or less, in a time period of between 10 minutes to 120 minutes, 10 minutes to 90 minutes, 10 minutes to 60 minutes, 10 minutes to 30 minutes, 15 minutes to 120 minutes, 15 minutes to 90 minutes, 15 minutes to 60 minutes, 30 minutes to 120 minutes, 30 minutes to 90 minutes, 30 minutes to 60 minutes, 45 minutes to 120 minutes, 45 to 90 minutes, 45 minutes to 75 minutes, or 45 minutes to 60 minutes, 10 minutes to 120 minutes, 10 minutes to 90 minutes, 10 minutes to 60 minutes, 10 minutes to 30 minutes, 15 minutes to 120 minutes, 15 minutes to 90 minutes, 15 minutes to 60 minutes, 30 minutes to 120 minutes, 30 minutes to 90 minutes, 30 minutes to 60 minutes, 45 minutes to 120 minutes, 45 to 90 minutes, 45 minutes to 75 minutes, or 45 minutes to 60 minutes.

In certain embodiments, the detectable signals may be produced with less capture agent and/or reduced solid substrate surface area. As such, certain embodiments may be suitable for microfluidic systems, where miniaturisation of structures and minimisation of reagents used is desirable. In certain embodiments, the method and/or kits may be performed in a microfluidic system.

Examples of microfluidic systems may include, for example, microfluidic "lab-on-a-chip" type devices; high density microtitre plates, such as 384, 1536, 3456 or 9600 well microtitre plates; microarrays and the like.

In certain embodiments the methods and/or kits shows a low variability for detecting an analyte between reactions. In certain embodiments, the methods and/or kits show a low intra-plate variability. In certain embodiments, the intra-plate variability is 30% or less, 20% or less, or 10% or less. For example, in certain embodiments the methods and/or kits shows a low intra-plate variability for detecting an analyte, such as an intra-plate variability of 30% or less, 20% or less, or 10% or less.

In certain embodiments, the detecting of the different analytes comprises providing one or more filters and/or one or more light sources and/or one or more light detectors to a reader device, such as a plate reader, to enable detection of the different analytes.

In certain embodiments, the method and/or kits comprises providing one or more filters and/or one or more light sources and/or one or more light detectors to a plate reader device to enable detection of the at least two different analytes.

Certain embodiments provide a method for detecting different analytes having one or more of the advantages as herein described, for example, a method of detecting different analytes having at least one of the following advantages: the ability to distinguish at least two different analytes in the reaction vessel; a reduction in the overlap in the detection signals used to detect the at least two analytes; a reduction in the interference between the detection signals used to detect the at least two analytes; providing sufficient sensitivity to allow the detection of low amounts of the at least two analytes; reduced interference from other agents in the sample; the ease and/or economy of detection of multiple analytes; the ability to use existing hardware to detect the at least two analytes in the reaction vessel; the ability to use existing plate readers; using low amounts of reagents for capturing and/or detecting the multiple analytes; a high capacity for binding of reagents; reducing the time for suitably detecting the at least two analytes; reducing the number of incubation steps to detect the at least two analytes; providing reliable detection of the at least two analytes; eliminating the need for pre-incubation of some components during the detection of the multiple analytes; reducing the number of dispensing steps during the detection of the at least two analytes; reducing the number of aspiration steps during the detection of the at least two analytes; reducing costs in time and handling needed to perform the assay; using a single assay platform to detect the at least two analytes; and providing kits and/or assay platforms that are easy to manufacture and/or less costly to manufacturer.

In certain embodiments, the present disclosure also provides kits for detecting different analytes. In certain embodiments, a kit for performing the methods as described herein is provided.

In certain embodiments, the kit comprises one or more of the reagents as discussed herein for performing the method described herein. In certain embodiments, the kit comprises instructions for performing one or more steps of the method as described herein.

In certain embodiments, the kit comprises one or more of the following: a solution for washing the reaction vessel to remove capture agents and/or detectable agents not bound to the reaction vessel; a cell lysis buffer, a signal enhancing agent, and instructions for detection of the different analytes.

In certain embodiments, the methods and/or kits of the present disclosure may also be performed by utilising reagents and/or instructions. The kit may comprise one or more of the reagents herein described and/or instructions to assist in the performance of the method.

In certain embodiments, the kit comprises instructions for detecting the different analytes in a reaction vessel. In certain embodiments, the kit comprises instructions for detecting the different analytes in a single reaction vessel.

In certain embodiments, kit comprises instructions for detecting the different analytes in a time of 2 hours or less, 90 minutes or less, 80 minutes or less, 70 minutes or less, 1 hour or less, 45 minutes or less, 30 minutes or less, 20 minutes or less, 15 minutes or less, 10 minutes or less, in a time period of between 10 minutes to 120 minutes, 10 minutes to 90 minutes, 10 minutes to 60 minutes, 10 minutes to 30 minutes, 15 minutes to 120 minutes, 15 minutes to 90 minutes, 15 minutes to 60 minutes, 30 minutes to 120 minutes, 30 minutes to 90 minutes, 30 minutes to 60 minutes, 45 minutes to 120 minutes, 45 to 90 minutes, 45 minutes to 75 minutes, or 45 minutes to 60 minutes, 10 minutes to 120 minutes, 10 minutes to 90 minutes, 10 minutes to 60 minutes, 10 minutes to 30 minutes, 15 minutes to 120 minutes, 15 minutes to 90 minutes, 15 minutes to 60 minutes, 30 minutes to 120 minutes, 30 minutes to 90 minutes, 30 minutes to 60 minutes, 45 minutes to 120 minutes, 45 to 90 minutes, 45 minutes to 75 minutes, or 45 minutes to 60 minutes from contacting the sample with the capture agents and/or the detectable agents.

In certain embodiments, the instructions for detecting the different analytes comprise instructions for utilising only a single wash of the solid substrate.

In certain embodiments, a single assay plate or assay platform that is suitable for many different assay kits may be used. This may provide manufacturers with a number of benefits, including reduced cost, labor and quality control requirements. In addition, in certain embodiments inputs can be reduced by the ability to use less of the target-specific agents (such as antibodies), reducing costs and quality control requirements, as single batches of target-specific agents can be used for more assay kits.

In certain embodiments, the reaction vessel is a single reaction vessel. In certain embodiments, the different analytes are one or more different non-nucleic acid analytes.

The kits of the present disclosure detect at least 2 different analytes. In certain embodiments of the kits of the present disclosure at least 3 different analytes.

In certain embodiments of the kits of the present disclosure, one or more of the at least two different capture agents are passively bound to the at least one solid substrate. In certain embodiments, each of the at least two different capture agents is passively bound to the solid substrate.

In the kits of the present disclosure, the at least one solid substrate comprises one or more immobilisation agents.. In certain embodiments, the at least two capture agents comprises a plurality of ligands for the immobilisation agent. In certain embodiments, each of the at least two capture agents comprises a plurality of ligands for the immobilisation agent.

In the kits of the present disclosure, one or more of the at least two different capture agents comprises an antibody or a binding fragment thereof. In certain embodiments, the at least two different capture agents comprises an antibody or a binding fragment thereof. In certain embodiments, each of the at least two different capture agents comprises an antibody or a binding fragment thereof.

In certain embodiments of the kits of the present disclosure, the kits comprise providing one or more of the at least two different capture agents at a concentration of 10 ng/ml to 1000 ng/ml, or 10 ng/ml to 500 ng/ml.

In the kits of the present disclosure, the ligand and the immobilisation agent are a binding pair comprising a peptide tag and an anti-peptide tag antibody.

In certain embodiments of the kits of the present disclosure, the ligand and the immobilisation agent are a binding pair that is not a poly anionic-poly cationic binding pair. In certain embodiments, the ligand and the immobilisation agent are selected from the ligand-immobilisation agent binding pairs comprising biotin and avidin or streptavidin or derivates thereof, histidine and metal chelate, maleic anhydride and amine, and maleimide and sulfhydryl.

In certain embodiments of the kits of the present disclosure, one or more of the at least two different capture agents is provided in solution. In certain embodiments, each of the at least two different capture agents is provided in solution.

In the kits of the present disclosure, the kits comprise providing at least two different detectable agents. The at least two different detectable agents bind to the at least two different analytes. In certain embodiments, each of the at least two different detectable agents bind to a different analyte.

In certain embodiments, the at least two detectable agents produce at least two different detectable signals. In certain embodiments, the at least two different detectable signals are detected simultaneously. In certain embodiments, the at least two different detectable signals are detected sequentially.

In certain embodiments, the at least two detectable agents produce the same detectable signals. In certain embodiments, the same detectable signals are detected sequentially.

In the kits of the present disclosure, the kits comprise detecting the at least two analytes by detecting the presence at least two different detectable agents bound to the solid substrate via the at least two analytes.

In certain embodiments of the kits of the present disclosure, one or more of the at least two different detectable agents are provided in solution. In certain embodiments, each of the at least two different detectable agents is provided in solution.

In certain embodiments of the kits of the present disclosure, one or more of the at least two different detectable agents comprises an antibody or a fragment thereof. In certain embodiments, each of the at least two different detectable agents comprises an antibody or a fragment thereof.

In certain embodiments of the kits of the present disclosure, the kit comprises providing one or more of the at least two different detectable agents at a concentration of 10 ng/ml to 100 ng/ml.

In certain embodiments of the kits of the present disclosure, one or more of the at least two detectable agents comprises one or more detectable tags. In certain embodiments, each of the at least two detectable agents comprises one or more detectable tags. In certain embodiments, the one or more detectable tags comprise a fluorophore. In certain embodiments, the fluorophore comprises a lanthanide ion.

In certain embodiments, the lanthanide ion is selected from the group consisting of Eu³⁺, Sm³⁺, Tb³⁺, and Dy³⁺. In certain embodiments, one of the detectable tags comprises Eu³⁺ and another of the detectable tags comprises Sm³⁺. In certain embodiments, one of the detectable tags comprises Eu³⁺, another of the detectable tags comprises Sm³⁺ and a further detectable tag comprises Tb³⁺.

In certain embodiments of the kits of the present disclosure, the one or more of the detectable tags comprises an enzyme that converts a substrate into a detectable product.

In certain embodiments, the enzyme is selected from the group consisting of horse radish peroxidase, alkaline phosphatase, and beta-galactosidase. In certain embodiments, one of the detectable tags comprises horse radish peroxidise and another of the detectable tags comprises alkaline phosphatase. In certain embodiments, one of the detectable tags comprises horse radish peroxidase, another of the detectable tags comprises alkaline phosphatase, and a further detectable tag comprises beta-galactosidase.

In certain embodiments, the kit comprises contacting the sample, each of the at least two different capture agents, each of the at least two different detectable agents and the at least one solid substrate in the reaction vessel. In certain embodiments, the sample, one or more of the at least two different capture agents, one or more of the at least two different detectable agents and the at least one solid substrate are not contacted in a separate vessel prior to contacting in the reaction vessel. In certain embodiments, the sample, each of the at least two different capture agents, each of the at least two different detectable agents and the at least one solid substrate are not contacted in a separate vessel prior to contacting in the reaction vessel. In certain embodiments, the sample and the solid substrate are contacted prior to contacting with one or more of the different capture agents and/or one or more of the different detectable agents. In certain embodiments, the sample and the at least one solid substrate are contacted prior to contacting with each of the at least two different capture agents and each of the at least two different detectable agents. In certain embodiments, the sample and the at least one solid substrate are first contacted in the reaction vessel.

In certain embodiments of the kits of the present disclosure, the sample and the at least one solid substrate are not substantially incubated prior to contacting with one or more of the at least two different capture agents and/or one or more of the at least different detectable agents. In certain embodiments, each of the at least two different capture agents and each of the at least two different detectable agents are contacted prior to contacting with either or both of the sample and the solid substrate. In certain embodiments, the at least two different capture agents and the at least two different detectable agents are contacted in a separate vessel.

In certain embodiments of the kits of the present disclosure, the kit comprises washing the at least one solid substrate in the reaction vessel to remove the at least two different capture agents and the at least two different detectable agents not bound to the solid substrate via the one or more ligands. In certain embodiments, there is no additional washing of the at least one solid substrate after contacting of the at least one solid substrate with one or more of the samples, one or more of the at least two different capture agents and one or more of the at least two different detectable agents.

In certain embodiments, there is an incubation of the contacted sample, the at least one solid substrate, one or more of the at least two different capture agents and one or more of the at least two different detectable agents prior to washing of the at least one solid substrate. In certain embodiments, the incubation is 2 hours or less, 1 hour or less or 30 minutes or less.

In certain embodiments of the kits of the present disclosure, the kit comprises detecting two or more of the different analytes when two or more of the different analytes are bound to the at least one solid substrate. In certain embodiments, the detecting one or more of the different analytes in solution when the one or more of the different analytes have been released from binding to the at least one solid substrate.

In certain embodiments of the kits of the present disclosure, the one or more of the different analytes is detected sequentially.

In certain embodiments of the kits of the present disclosure, the reaction vessel comprises the solid substrate. In certain embodiments, the reaction vessel comprises a well of a multi-well plate, such as a microtitre plate.

In certain embodiments of the kits of the present disclosure, the at least one solid substrate has a total binding capacity of 5 ug/ml or less for protein.

In certain embodiments of the kits of the present disclosure, the sample is a blood sample, a serum sample, a urine sample, a cell sample, a cell lysate or a suitable combination of the aforementioned.

In certain embodiments of the kits of the present disclosure, one or more of the different analytes are selected from the group consisting of a component of a cell signalling pathway, a cytokine, or a tumour suppressor. In certain embodiments, one or more of the different analytes comprises a protein. In certain embodiments, the protein is a phosphoprotein. In certain embodiments, one or more of the different analytes is selected from the group consisting of phospho-ERK 1/2; total ERK 1/2; phospho-Akt 1/2/3; total Akt 1/2/3; phospho-NF-Kβ p65; total NF-Kβ p65; phospho-1-kBα; total-kBα; phospho-STAT3; total STAT3; phospho-STATS A/B; phospho-JNK 1/2/3; total JNK 1/2/3; phospho-p38 MAPKα; total p38 MAPKα; phospho-p53; total p53; phospho-p70S6K; total p70S6K; and GAPDH.

In certain embodiments of the kits of the present disclosure, the detection of one or more of the different analytes is achieved in a time of 2 hours or less from contacting the sample with one or more of the at least two different capture agents. In certain embodiments, the detection of one or more of the different analytes is achieved in a time of 1 hour or less from contacting the sample with one or more of the at least two different capture agents. In certain embodiments, the detection of one or more of the different analytes is achieved in a time of 30 minutes or less from contacting the sample with one or more of the at least two different capture agents.

In certain embodiments of the kits of the present disclosure, the detection of one or more of the different analytes is achieved in a time of 2 hours or less from contacting the sample with one or more of the at least two different capture agents and/or one or more of the at least two different detectable agents. In certain embodiments, the detection of one or more of the different analytes is achieved in a time of 1 hour or less from contacting the sample with one or more of the at least two different capture agents and/or one or more of the at least two different detectable agents. In certain embodiments, the detection of one or more of the different analytes is achieved in a time of 30 minutes or less from contacting the sample with one or more of the at least two different capture agents and/or one or more of the at least two different detectable agents.

In certain embodiments of the kits of the present disclosure, the kits comprises one or more filters and/or one or more light sources and/or one or more light detectors to a plate reader device to enable detection of the different analytes.

In certain embodiments, the at least two detectable agents produce at least two different detectable signals. In certain embodiments, the detectable signals are detected sequentially. In certain embodiments, the detectable signals are detected simultaneously. In certain embodiments, the at least two detectable agents produce the same detectable signals. In certain embodiments, the detectable signals are detected sequentially.

Certain embodiments provide a kit for detecting different analytes having one or more of the advantages as herein described, for example, a kit for detecting different analytes having at least one of the following advantages in use: the ability to distinguish at least two different analytes in the reaction vessel; a reduction in the overlap in the detection signals used to detect the at least two analytes; a reduction in the interference between the detection signals used to detect the at least two analytes; providing sufficient sensitivity to allow the detection of low amounts of the at least two analytes; reduced interference from other agents in the sample; the ease and/or economy of detection of multiple analytes; the ability to use existing hardware to detect the at least two analytes in the reaction vessel; and the ability to use existing plate readers; using low amounts of reagents for capturing and/or detecting the multiple analytes; a high capacity for binding of reagents; reducing the time for suitably detecting the at least two analytes; reducing the number of incubation steps to detect the at least two analytes; providing reliable detection of the at least two analytes; eliminating the need for pre-incubation of some components during the detection of the multiple analytes; reducing the number of dispensing steps during the detection of the at least two analytes; reducing the number of aspiration steps during the detection of the at least two analytes; reducing costs in time and handling needed to perform the assay; using a single assay platform to detect the at least two analytes; and providing kits and/or assay platforms that are easy to manufacture and/or less costly to manufacturer.

The present disclosure is further described by the following examples. It is to be understood that the following description is for the purpose of describing particular embodiments only.

### EXAMPLE 1

### Materials

Antibodies used in the following examples include: anti-pERK mouse monoclonal (+/- biotinylation); anti-total ERK rabbit monoclonal (+/- HRP); donkey anti-rabbit-HRP conjugate; anti-S6 p240/44 rabbit polyclonal (HRP conjugated); anti-S6 mouse monoclonal (biotinylated); anti-AKT pT308 rabbit monoclonal (HRP conjugated); anti-AKT mouse monoclonal (biotinylated); anti-AKT pS473 mouse monoclonal (biotinylated); and anti-AKT rabbit monoclonal (HRP conjugated).

Other reagents and materials used in the following examples include: QuantaRed^{™} enhanced chemifluorescent HRP substrate (Thermo Scientific); SIGMAFAST^{™} OPD tablets (Sigma); 96 well clear immunoassay Maxisorp^{™} plates (Nunc); 384 well clear immunoassay Maxisorp^{™} plates (Nunc); Streptavidin (Sigma); Blocking solution (1% BSA in PBS containing 0.05% Tween 20); and A431 cell lysate containing pERK.

### EXAMPLE 2

### Methods

### 1-wash ELISA protocol

Nunc 96 well Maxisorp^{™} plates were passively coated with streptavidin and blocked. pERK cell lysates (50 µL) were added to wells followed by the addition of a reaction buffer (50 µL) containing pre-optimised concentrations of biotinylated anti-pERK mouse mAb and anti-total ERK-HRP rabbit mAb (alternatively a reaction buffer containing biotinylated anti-pERK mouse mAb, anti-total ERK rabbit mAb and anti-rabbit IgG-HRP can be used).

In certain cases a pre-incubation of pERK cell lysate with the antibodies was performed in a sample plate prior to transfer to the streptavidin coated plate. Plates were incubated for a minimum of 30 min before washing 3x with PBS-T, addition of HRP substrate (100 µL) and measurement of product. A similar 1-wash protocol was followed when using Nunc 384 well Maxisorp^{™} plates. The specific kinase antibodies were supplemented into the protocol and the final reaction volume was 20 µL.

### Comparative Multi-wash ELISA protocol - streptavidin coated plate

Nunc 96 well Maxisorp^{™} plates were passively coated with streptavidin and blocked. Biotinylated anti-pERK mouse mAb was added to wells and incubated for a minimum of 30 min (100 µL). Plates were washed 3x with PBS-T. pERK cell lysates were added to wells and incubated for a minimum of 30 min (100 µL). Plates were washed 3x with PBS-T. Anti-total ERK-HRP rabbit mAb was added to wells and incubated for a minimum of 30 min (100 µL). Plates were washed 3x with PBS-T before addition of HRP substrate (100 µL) and measurement of product.

### Comparative Multi-wash ELISA protocol - anti-pERK IgG coated plate

Nunc 96 well Maxisorp^{™} plates were passively coated with anti-pERK mouse mAb and blocked. pERK cell lysates were added to wells and incubated for a minimum of 30 min (100 µL). Plates were washed 3x with PBS-T. Anti-total ERK-HRP rabbit mAb was added to wells and incubated for a minimum of 30 min (100 µL). Plates were washed 3x with PBS-T before addition of HRP substrate (100 µL) and measurement of product.

### EXAMPLE 3

### Results - Assay Characteristics

### Speed/Simplicity

In their optimized formats, the 1-wash assay performed comparably to the multi-wash assay in terms of sensitivity (Figure 1). This was evident on both streptavidin (Protocols 1 & 2) and anti-pERK IgG (Protocol 3) coated plates for the 30 min (Figure 1A) and 60 min incubation periods (Figure 1B). Generally, there was approximately a 10% greater signal obtained at each respective pERK concentration in the 1-wash ELISA compared to the multi-wash ELISAs but this did not translate to a significant improvement in the assay detection limit. Importantly, this demonstrated that the 1-wash assay could be performed with less handling steps and in less than half the time of the multi-wash ELISAs without negatively impacting on sensitivity. This translated to a much simpler ELISA assay format by the consolidation of multiple steps into a single 1-wash/step system.

### Sensitivity

When the 1-wash and multi-wash ELISAs were performed for the same total length of time of 1 h or less, the 1-wash ELISA was superior in sensitivity (Figure 2). Comparison of a 1 × 30 min incubation step to 3 × 10 min incubation steps on a streptavidin coated plate (2A) showed that the 1-wash system was approximately 10 times more sensitive than the multi-wash system. Although not as significant, this trend was also noticeable when comparing a 1 × 60 min 1-wash assay system on a streptavidin plate, to a 2 × 30 min multiwash system on an anti-pERK IgG coated plate (2B). The major benefit of the 1-wash ELISA protocol was that it allowed multiple antibody-antigen binding events to occur simultaneously in the single 30 or 60 minute incubation period thereby improving the pERK detection capabilities per unit time.

### Capture Antibody Efficiency

The concentration dependency of anti-pERK IgG (+/- biotinylation) for detecting pERK was assessed in each of the ELISA protocols (Figure 3). With or without a pre-incubation step, the 1-wash protocol required approximately 4x and 10x less anti-pERK IgG, to detect the same amount of pERK when compared to multi-wash ELISA protocols 3 and 4 respectively. The importance of a pre-incubation step (protocol 1 vs protocol 2) in the 1-wash ELISA was noticeable when the anti-pERK IgG concentrations were 100 ng/mL or less. At these lower concentrations, more pERK per unit antibody (approx 15% higher signal) was able to be detected when a pre-incubation step was incorporated into the 1-wash protocol. Collectively, these results indicated that the 1-wash protocol was more efficient with its use of anti-pERK IgG compared to the multi-wash format for detecting the same amount of pERK. A possible explanation for this phenomenon was that the 1-wash format allowed the formation of solution-phase pERK immune complexes, enabling their binding to the streptavidin or anti-pERK IgG coated surface in a more orientated fashion thereby enhancing antibody functionality. Conversely, in the absence of pERK and detection IgG, biotinylated or unbiotinylated anti-pERK IgG could bind randomly to the surface, which may have led to a portion of pERK IgG binding sites becoming inaccessible to pERK and/or sterically hindering subsequent binding events in the sandwich (i.e. detection IgG).

The improved anti-pERK IgG efficiency phenomenon highlighted in Figure 3 for the 1-wash ELISA format was investigated further by separating the multiple antibody-antigen binding events of the pERK assay (Figure 4). This highlighted that independent formation of pERK with anti-total ERK-HRP IgG or anti-pERK IgG (protocols 2 & 3 respectively), prior to binding to their immobilized partner on the plate, contributed to the more efficient use of anti-pERK IgG in the 1-wash ELISA format. Individually, protocols 2 & 3 were approximately 2 times more efficient with their use of anti-pERK IgG for detecting pERK compared to the multi-wash ELISA (protocol 4). Furthermore when the individual binding events of protocols 2 & 3 were allowed to occur simultaneously as part of the 1-wash ELISA (protocol 1), the use of anti-pERK IgG compared to the multi-wash procedure was 4-5 times less when measuring the same concentration of pERK. Ultimately this highlighted that the binding of both antibodies to pERK in solution were important for enhancing the functionality of the anti-pERK IgG used in the 1-wash ELISA. This would result in less reagent use (i.e. antibody) and therefore reduced assay cost, compared to the multi-wash ELISA format.

### Versatility

The 1-wash ELISA protocol was also challenged using a secondary detection antibody that was conjugated to HRP (Figure 5). This was achieved by replacing the anti-total ERK-HRP with the original unconjugated antibody (i.e. minus HRP) and introducing anti-rabbit IgG-HRP as the secondary detection antibody. That is, this experiment used a 3 antibody protocol in the 1-wash ELISA format and yielded an A450 signal for pERK of approximately 1.0 AU and a signal:noise value of 10. Although unoptimized, in principle this secondary detection approach was validated in a 1-wash protocol and highlighted the versatility of the 1-wash ELISA using at least 3 antibodies.

### Robustness

Detection of other phosphoproteins including S6 p240/44, AKT pT308 and AKT pS473 was also achieved in the 1-wash ELISA system (Figure 6). In 384 well streptavidin coated plates, signal:noise ratios of greater than 60 were achieved when assaying cell lysates containing the specific phosphoproteins of interest. Like the pERK protocol, the AKT pS473 assay also used an anti-phospho IgG as the capture antibody with an anti-total IgG used as the detection antibody (i.e. conjugated to HRP). Alternatively the S6 p240/44 and AKT pS473 assays used an anti-total IgG as the capture antibody, with a specific anti-phospho IgG-HRP completing the sandwich. These results demonstrated the robustness of the 1-wash ELISA with its ability to detect different targets in varying immune complex orientations.

### EXAMPLE 4

### Microfluidics

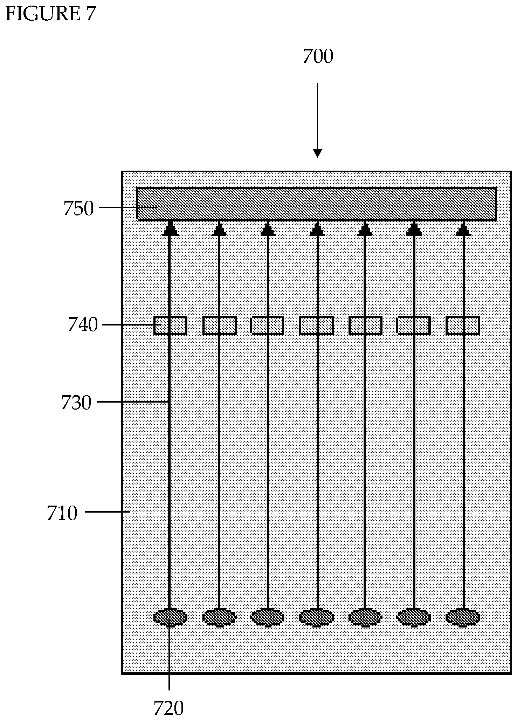

Microfluidic reactions were performed in a microfluidic cartridge as shown in Figure 7. Referring to Figure 7, the microfluidic cartridge 700 comprises a plastic substrate 710 into which a plurality of flow channels 730 are formed. A sample is introduced into the flow channel 730 via sample inlet 720. The sample is then driven along flow channel 730 by a pump (not shown). Detection region 740 comprises an electrode for electrochemical detection to which an immobilization agent is bound. In the embodiments described in the following examples, the immobilization agent is streptavidin. Moreover, although the present invention contemplates any suitable electrodes and methods for electrochemical detection, the method described in the following examples utilizes the electrodes and detection methods described in US patent 6,770,190. After passing over detection area 740, the sample is transported to waste collection area 750.

An example of the method of the present invention performed in the microfluidic cartridge is described below:

Samples were mixed with a reaction buffer (phosphate buffered saline, BSA 0.3%, Tween 0.1%) containing two antibodies to the analyte of interest. For each analyte, the two antibodies were raised against distinct epitopes on the analyte of interest, such that both antibodies could bind to the protein of interest simultaneously. One of the antibodies performed the function of a capture agent and had biotin attached to it, while the other antibody performed the function of a detectable agent and was linked to horse radish peroxidise (HRP).

The samples being measured contained varying amounts of an analyte of interest, in the present examples either phospho-ERK or phospho-AKT. A microfluidic cartridge (see Figure 7) was placed on a pumping and detection instrument, and samples were drawn onto the microfluidic cartridge into separate lanes of the cartridge. The cartridge bound the biotinylated antibody at the detection region. As set out above, the detection region comprised an electrode for electrochemical detection to which streptavidin is bound as an immobilisation agent. As such, a complex comprising biotinylated capture antibody, bound analyte and HRP-linked detectable antibody would become immobilised to the electrode via interaction of the biotin on the capture antibody and streptavidin on the electrode.

After capture, the cartridge was automatically washed with buffer without antibodies. Following this wash step, a solution containing HRP substrate (SigmaFAST OPD) was drawn over the cartridge, allowing bound HRP to convert the HRP substrate to products that could be detected electrochemically by the electrode and detection equipment present on the pumping device. The electrical signals generated were proportional to the level of HRP-induced product conversion, which was proportional to the amount of analyte bound to the capture antibodies.

### EXAMPLE 5

### Detection of pERK using a microfluidic system

Recombinant pERK was diluted in 1X lysis buffer, with four fold dilutions from a top concentration of 400 ng/ml (10nM). Samples were pre incubated with an equal volume of reaction buffer (see above).

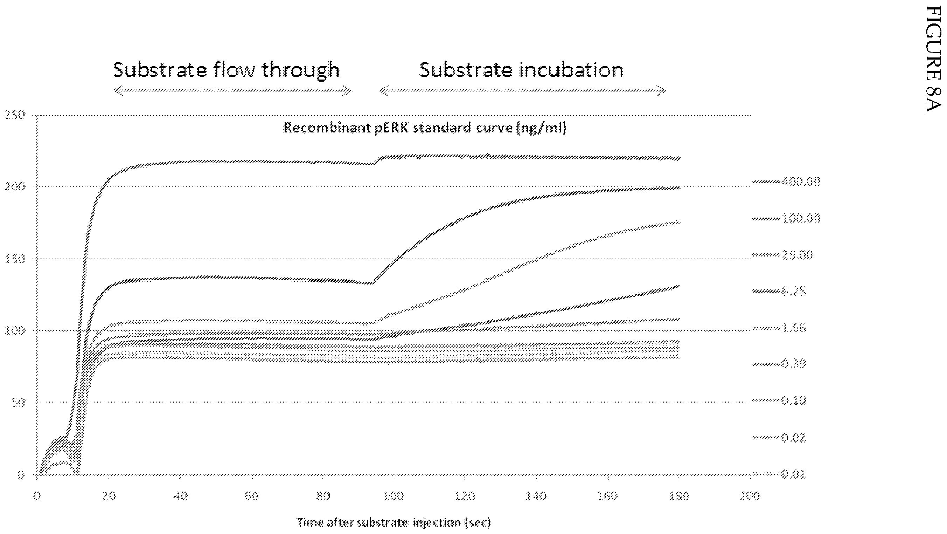

The sample/reaction buffer mix was then run on a microfluidic cartridge as described in Example 4. The results are shown in Figures 8A-8C. Each data point shown is the average of 3 flow cells from a single cartridge. The data was transformed by taking the point at which substrate injection begins as zero. Data was collected from the point at which substrate flow through begins up until the end of substrate incubation phase (180s after substrate injection).

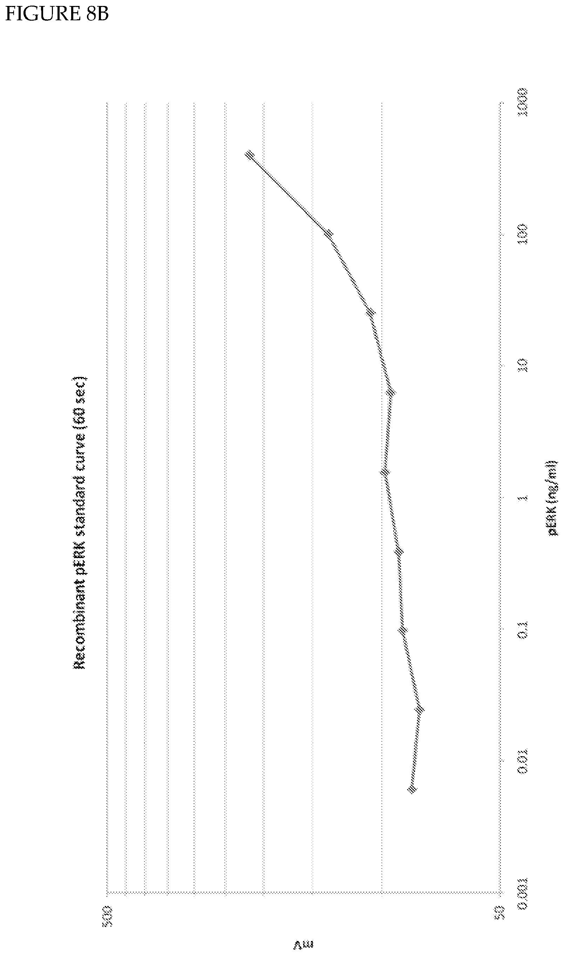

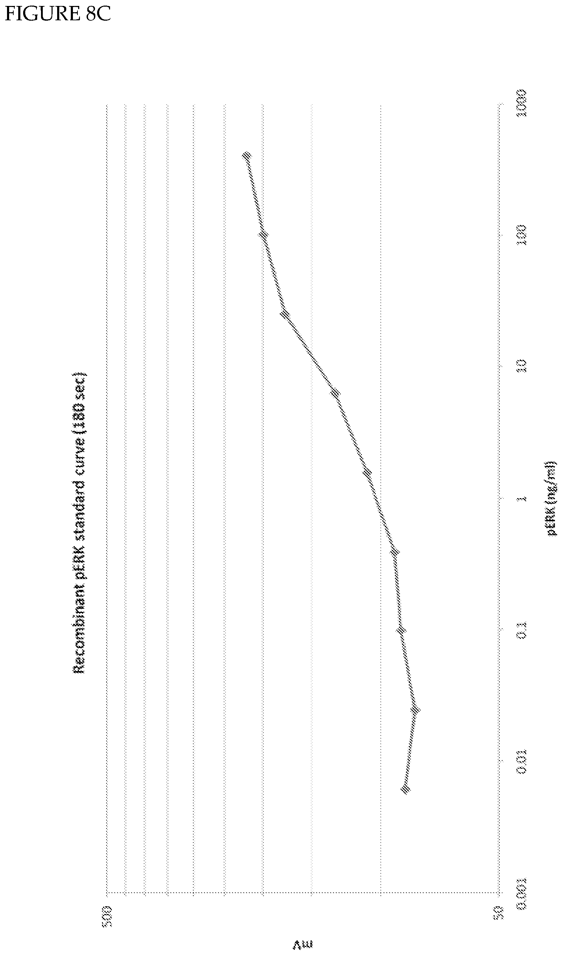

As can be seen by comparing Figures 8b and 8c, data collection at the end of the substrate incubation phase (180s after substrate injection) appeared to provide greater sensitivity. Using the data taken from 180 seconds after injection of substrate, the detection limit of the chip was about 2 ng/ml pERK.

### EXAMPLE 6

### Detection of pAKT using a microfluidic system

Recombinant pAKT473 was diluted in 1X lysis buffer, with five fold dilutions from a top concentration of 100 ng/ml. Samples were pre incubated for two hours with an equal volume of reaction buffer (see above) to equilibrate the interaction and so minimise incubation effects during the run.

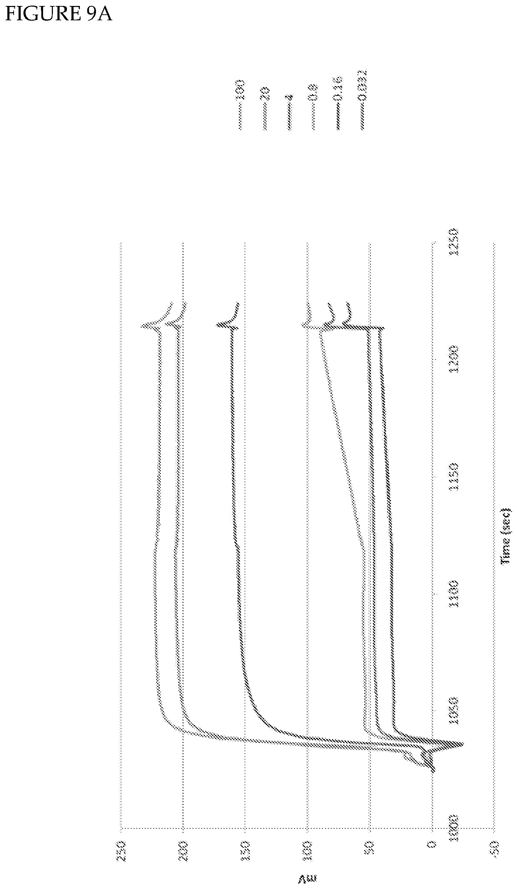

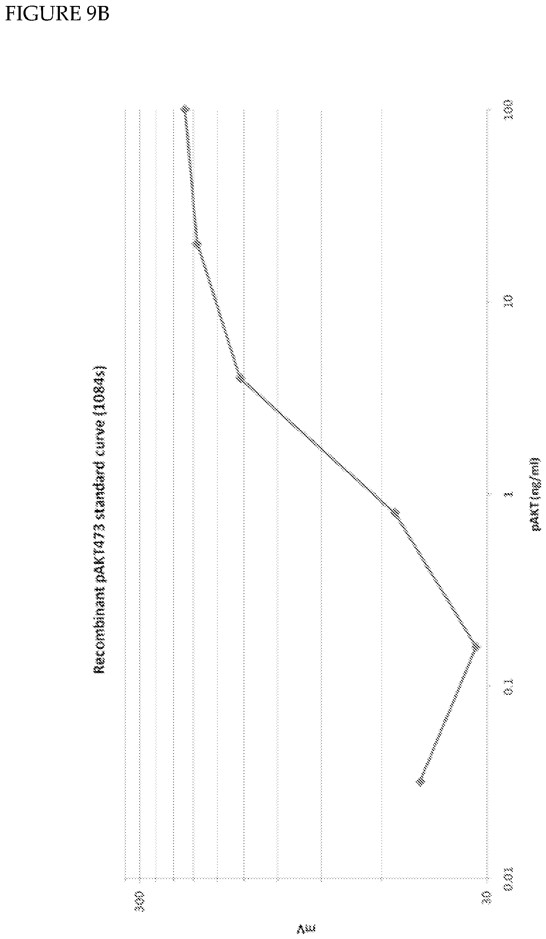

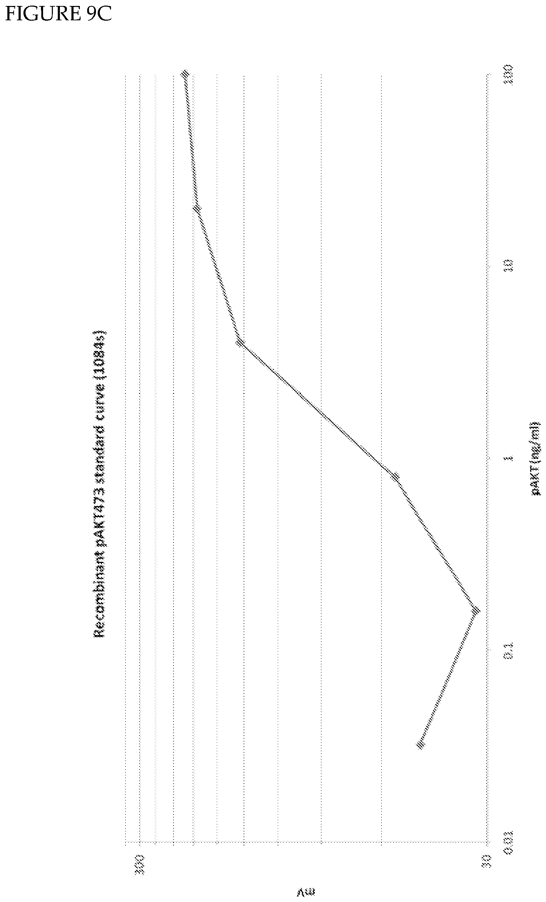

The sample/reaction buffer mix was then run on a microfluidic cartridge as described in Example 4. The results are shown in Figures 9A-9C. Each data point shown is the average of 3 flow cells from a single cartridge. The data was transformed by taking the point at which substrate injection begins as zero. Data was collected from the point at which substrate flow through begins up until the end of substrate incubation phase (180s after substrate injection).

Using the data taken from 180 seconds after injection of substrate, the detection limit of the chip was about 1 ng/ml pAKT.

### EXAMPLE 7

### Reagent order of addition permutations

Capture antibody (anti-pERK-peptide conjugate or anti-pERK-biotin conjugate), detection antibody (anti-total ERK-HRP conjugate), capture/detection antibody mixture, and varying concentrations of cell lysate containing pERK were added to (A) anti-peptide conjugate antibody coated plates or (B) streptavidin coated microplates, in 8 different permutations (refer to Table 1 & 2). Individual assay components were added 1 min apart to the plates, and incubated for 2 h. Plates were washed, incubated with HRP substrate, before detection of the fluorescent product.\

**Table 1: Reagent volumes for order of addition assessment**

| **Assay Component** | **Volume/Well** |
|---|---|
| Capture/Detection Antibody Mix | 50ul |
| Lysate | 50ul |
| Capture Antibody | 25ul |
| Detection Antibody | 25u1 |

**Table 2: Reagent order of addition permutations**

| **Trial #** | **1^{st} Addition** | **2^{nd} Addition** | **3^{rd} Addition** |
|---|---|---|---|
| 1 | Capture/Detection Ab Mix | Lysate | n/a |
| 2 | Lysate | Capture/Detection Ab Mix | n/a |
| 3 | Lysate | Capture Ab | Detection Ab |
| 4 | Lysate | Detection Ab | Capture Ab |
| 5 | Detection Ab | Capture Ab | Lysate |
| 6 | Detection Ab | Lysate | Capture Ab |
| 7 | Capture Ab | Lysate | Detection Ab |
| 8 | Capture Ab | Detection Ab | Lysate |

The effect of reagent order of addition on pERK detection in the single-incubation ELISA using different capture systems is shown in Figure 10. Across the 8 different permutations, and at several analyte concentrations, little signal difference were observed. This result demonstrates that equivalent results can be obtained in a single-incubation ELISA assay, irrespective of the order of addition of the individual components.

### EXAMPLE 8

### Recombinant protein standard curves in different biological milieu using the peptide conjugate capture system

A demonstration of the use of the single-incubation ELISA assay format for the detection of three recombinant human proteins diluted in human serum is provided in Figure 11. EGF, IL-2 and TNFα were measured in PBS/0.5% BSA and human serum. Detection limits of ≤ 10 pg/mL were ascertained for each assay in PBS/0.5% BSA, and similar sensitivity for both IL-2 and TNFα were observed for analyte diluted in human serum. The detection limit for EGF in human serum could not be detected due to the presence of a high level of endogenous EGF, which was confirmed using a standard commercial EGF ELISA kit (R&D Systems, data not shown). Figure 11 shows the mean and standard deviations for the duplicate data points for each target analyzed.

This data illustrates that the single-incubation ELISA assay format was robust to measuring analytes in different biological milieu. The assay clearly demonstrates efficacy for several different targets in serum, whereby the assay components are incubated concurrently. The high signal for EGF in human serum is due to the presence of endogenous EGF protein(s) in this medium.

### EXAMPLE 9

### Recombinant protein standard curves using the peptide conjugate capture system in a 10 min single-incubation ELISA

Nunc 96 well Maxisorp^{™} plates were passively coated with an anti peptide tag antibody overnight at 4°C. Plates were washed 3x with PBS-T and blocked with 200 µL/well of a 1% BSA solution in PBS-T (0.05%). Blocking solution was aspirated prior to assay. Analyte (eg 50 µL of recombinant protein) were added to the wells followed by the addition of an antibody antibody mixture (50 µL) containing pre-optimised concentrations of peptide tag conjugated anti-analyte capture antibody and HRP-conjugated anti-analyte detection antibody. Plates were incubated for 10 min before washing 3× with PBS-T. Fluorescent HRP substrate (100 µL) was added to the wells and incubated for 5 mins before measurement of fluorescent product.

Recombinant human proteins EGF, IL-2 and TNFα were prepared in PBS/0.5% BSA at concentrations ranging from 100 ng/mL down to 1 pg/mL and 50 µL/well added to an anti peptide tag antibody ELISA plate. Capture/detection antibody mix for EGF (A), IL-2 (B) and TNFα (C) were added to the appropriate ELISA plate wells and incubated for 10 min. Plates were washed before incubation with HRP substrate for 5 min and detection of the fluorescent product.

Figure 12 shows the detection of three recombinant human proteins using a 10 min single-incubation ELISA assay format on an anti peptide tag antibody coated ELISA plate. EGF, IL-2 and TNFα standard curves were measured successfully in PBS/0.5% BSA with detection limits of ≤ 32 pg/mL ascertained for each assay. This data illustrated that the simplified peptide conjugate capture/single-incubation ELISA assay format was amenable to measuring multiple analytes on the same plate in as little as 10 minutes. As can be seen, in the 10 minute single-incubation ELISA the assay was still able to efficiently detect the three analytes, even at a concentration of the analytes less than 100 pg/ml.

### EXAMPLE 10

### Intra-plate variation

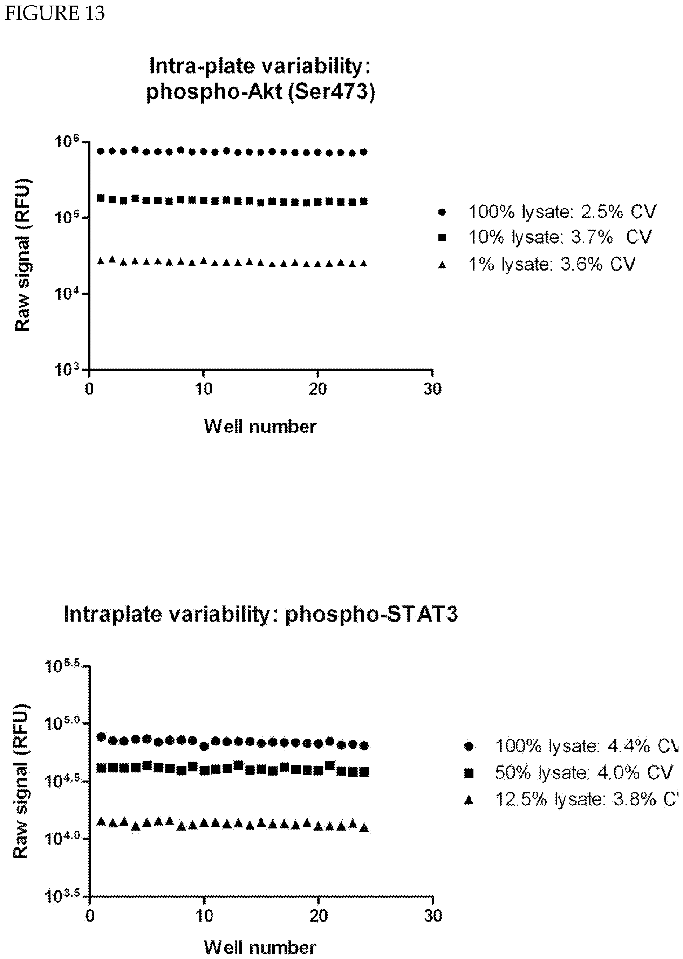

Figure 13 shows intra-plate variation observed for 2 separate single-incubation ELISAs for either phospho-AKT (pSer473) or phospho-STAT3. For each target, cellular lysate was diluted to 3 different concentrations using 1X Lysis buffer as indicated, and added to 24 replicate wells of a 96-well streptavidin-coated microplate. To initiate the assay reaction, for either target, a mixture of the biotin-conjugated capture antibody, and the HRP-conjugated detection antibody were added to the lysates, and incubated for 1 hour. The wells were subjected to a standard wash cycle for each assay. After the wash cycle, QuantaRed^{™} HRP substrate was added to the wells, and each plate was incubated for 10 min in the dark. The fluorescent signal in the wells was measured at 550ex/600em nm. Figures 13A and 13B show the data points at each lysate concentration analyzed, for phospho-AKT and phospho-STAT3, respectively. The coefficient of variation (CV%) for each analyte concentration was calculated by dividing the standard deviation observed over the 24 wells at each concentration, by the mean of the 24 wells at the same concentration, and transforming this fraction to a percentage value. Typically, a value of less than 10% is desired for many assays, for example, in certain high quality assays, and the data presented here demonstrates suitable low intra-plate variability characteristics.

### EXAMPLE 11

### Detection of TNFα

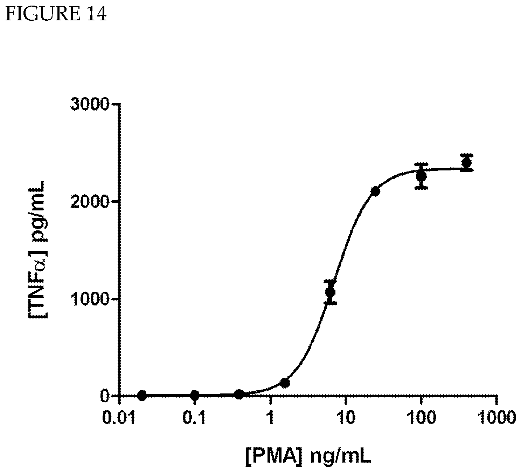

Figure 14 shows detection of TNFα in tissue culture supernates. THP-1 cells were seeded into 96-well tissue culture microplates in RMPI cell culture medium containing 10% (v/v) foetal bovine serum and various other standard cell culture additives. The cells were then treated with a various concentrations of PMA diluted in the same medium, and incubatd overnight in a humidified 37°C incubator. The following day 50 µL of medium was aspirated from the cell culture wells, and added to the wells of a peptide-coated 96-well assay plate. The assay reaction was initiated by the addition of 50 µL of an antibody mixture containing the capture antibody-peptide conjugate, and the detection antibody-HRP conjugate, and incubated for 1 hour. The wells were subjected to a standard wash cycle for each assay. After the wash cycle, fluorescent HRP substrate was added to the wells, and each plate was incubated for 10 min in the dark. The fluorescent signal in the wells was measured at 540ex/590em nm, and quantitated using a standard curve generated against the same target. Figure 14 shows the mean and standard deviations for the duplicate data points for each target analyzed. In this Figure, the assay demonstrates efficient detection of specific target analyte in tissue culture supernates using the certain embodiments, whereby the assay components are incubated concurrently.

### EXAMPLE 12

### Detection of phospho-AKT (pSer473) or phospho-ERK in a 25 min total assay time

Figure 15 shows detection of either phospho-AKT (pSer473) or phospho-ERK in a 25 min total assay time. For each target, recombinant active (A) phospho-AKT or (B) phospho-ERK was diluted as indicated, to various concentrations using 1X Lysis buffer containing 0.1% BSA and added to 4 replicate wells of a 96-well streptavidin-coated microplate. To initiate the assay reaction, for either target, a mixture of the biotin-conjugated capture antibody, and the HRP-conjugated detection antibody were added to the lysates, and incubated for 1 hour. The wells were subjected to a standard wash cycle for each assay. After the wash cycle, QuantaRed^{™} HRP substrate was added to the wells, and each plate was incubated for 10 min in the dark. The fluorescent signal in the wells was measured at 550ex/600em nm. Figures 15A and 15B show the data points at each analyte concentration analyzed, for phospho-AKT and phospho-ERK, respectively. Both assays demonstrated sensitivity to less than 1 ng/mL.

### EXAMPLE 13

### Detection of IL-2 in using a ERK peptide-anti peptide capture pair

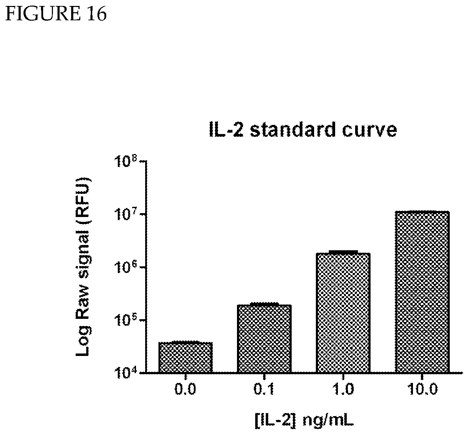

Figure 16 shows detection of IL-2 in using a ERK peptide-anti peptide capture pair. Recombinant interleukin 2 (IL-2) was diluted as indicated, to various concentrations using 1X PBS containing 0.1% BSA and added to duplicate wells of a 96-well anti-ERK-peptide antibody-coated microplate. To initiate the assay reaction, for either target, a mixture of the ERK peptide capture antibody, and the HRP-conjugated detection antibody were added to the lysates, and incubated for 1 hour. The wells were subjected to a standard wash cycle for each assay. After the wash cycle, fluorescent HRP substrate was added to the wells, and each plate was incubated for 10 min in the dark. The fluorescent signal in the wells was measured at 540ex/590em nm. Figure 16 shows the data points at each analyte concentration analyzed, demonstrating sensitivity to 100 pg/mL or less.

### EXAMPLE 14

### General Discussion

The single-incubation ELISA uses an immuno-sandwich format, but with at least one difference. For the single-incubation ELISA assay, both the analyte and the assay reagents are added to the assay microplate at the same time, in solution. After a short incubation period, unbound assay reagents and analytes are washed away, and immuno-complexes containing both antibodies are detected. The single-incubation ELISA allows the user a higher degree of assay flexibility. In contrast to other ELISA formats, in particular sets of examples no target-specific antibodies are present on the assay microplate itself, so assays for several different targets can be performed in different wells on the same microplate. For example, a cellular lysate can be analyzed on the same assay microplate in parallel for p38-MAPK phosphorylation, ERK phosphorylation, AKT phosphorylation and JNK phosphorylation, giving fast, accurate and quantifiable information on key cell signalling events. However, if desired target antibodies may be immobilized on the plate.

The single-incubation ELISA provides the high quality results desired from a sandwich immunoassay, and the assay allows for the use of self-contained kits to conduct the assay.

For example, a kit may contain one or more of the following components:
Capture Antibody Reagent
Detection Antibody Reagent
Lysis Buffer (for example supplied at 5X concentration) containing a mixture of detergents for cellular lysis, and phosphatase inhibitors.
Enhancer Solution - containing factors for enhancing assay performance, such as anti-HAMA components, and target-specific additives to increase assay performance.
ADHP Dilution Buffer - containing cofactors necessary for the HRP-mediated conversion of ADHP to resorufin.
ADHP (for example supplied at 100X concentration)
Wash Buffer (for example supplied at 10X concentration)
Stop Solution - for stopping HRP activity when necessary
Assay Control Lysate
Assay microplate
Assay diluent - for the dilution of concentrated samples

### EXAMPLE 15

### General Assay protocols

### (i) Protocol for use with samples such as cellular lysates and tissue culture supernates

### Assay protocol

1. Add 50 ul/well of sample to the assay microplate. 50 ul /well assay controls may be added to separate wells if desired.
2. Add 50 ul/well of antibody mix to the wells. Generally a concentration of antibodies in the mix of 50-500 ng/mL is suitable. Cover the microplate and incubate at room temp on a microplate shaker (-300 rpm).
3. Wash wells with 200 ul /well wash buffer (repeat 3 times). After final wash, remove any remaining wash solution from wells. A suitable wash buffer is PBS containing Tween 20.
4. Immediately prior to use, prepare substrate mix. A suitable substrate mix is TMB, ADHP, OPD, or other suitable HRP substrates, diluted with co-factors suitable for mediating their conversion to measurable by-products. Add 100 ul/well of substrate mix. Cover microplate with foil, and incubate for 10 minutes at room temp on a microplate shaker (-300 rpm).
5. Add 10 ul/well stop solution, and mix briefly (5-10 sec) on a microplate shaker. A suitable stop solution is a dilute acid such as HCl, or a strong detergent such as SDS.
6. Read fluorescence signal with a compatible filter set.

### (ii) Protocol for serum samples, or other samples that may carry sample-specific interferences

### Assay protocol

1. Add 25ul/well Enhancer mix. Enhancer mix containing general components for the neutralization of HAMAs, as well other components for the neutralization of target-specific binding proteins carried in serum.
2. Add 50 ul/well of sample to the assay microplate. 50 ul /well assay controls may be added to separate wells if desired.
3. Add 25 ul/well of antibody mix to the wells. Cover the micro plate and incubate for 1 hour at room temp on a microplate shaker (-300 rpm).
3. Wash wells with 200 ul /well wash buffer (repeat 3 times). After final wash, remove any remaining wash solution from wells.
4 Prepare substrate prior to use and add 100 ul/well. Cover microplate with foil, and incubate for 10 minutes at room temp on a microplate shaker (-300 rpm).
5. Add 10 ul/well stop solution, and mix briefly (5-10 sec) on a microplate shaker.
6. Read fluorescence signal with a compatible filter set.

### EXAMPLE 16

### Detection of various concentrations of IL-2 using a peptide tag anti peptide tag antibody capture system

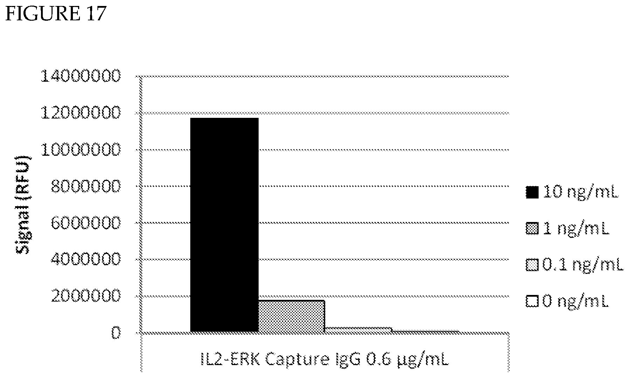

Figure 17 shows detection of various concentrations of IL-2 using a peptide tag anti peptide tag antibody capture system.

Antibodies were generated in mice as monoclonal antibodies to a 23 amino acid peptide, KRITVEEALAHPYLEQYYDPTDE (SEQ ID NO.2), a sequence derived from the carboxy terminus of the human ERK proteins (ERK C-term peptide). Purified antibodies (TGR, 12D4) to this peptide were passively coated onto a maxisorb Nunc immunoassay plate, and the plate then blocked against further non-specific protein attachment. The ERK C-term peptide was also used to conjugate to antibodies to the human IL-2 protein (R&D Systems), so that the peptide would act to anchor this antibody to the plate surface. A second IL-2 antibody (R&D Systems) was conjugated to horse radish peroxidase (HRP) to be used as the reporter antibody. Recombinant human IL-2 was mixed with PBS/BSA (0.1%) at various concentrations shown, and to these solutions were added the IL-2 antibodies. After an hour incubation, the wells were washed with a wash buffer, and fluorescent HRP substrate added for 10min, followed by reading of the plate at 540/590nm ex/em wavelengths in a plate reader.

It can be seen that the assay system measured the concentrations of IL-2 present in each sample and that the variation between samples was low as indicated by the small error bars.

### EXAMPLE 17

### Detection of various concentrations of EGF, IL-2 & TNFα using a peptide tag anti peptide tag antibody capture system

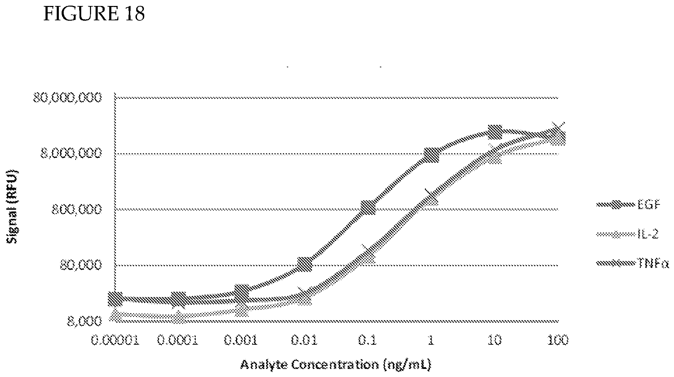

Figure 18 shows detection of various concentrations of EGF, IL-2 & TNFα using a peptide tag anti peptide tag antibody capture system.

Antibodies specific to the peptide DYKDDDDK (SEQ ID NO.1; Sigma, catalog number F1804) were passively coated onto a maxisorb Nunc immunoassay plate at 5 µg/mL overnight in PBS, and the plate then blocked against further non-specific protein attachment. The peptide DYKDDDDK (SEQ ID NO.1) was also used to conjugate to IgG antibodies to the human IL-2 protein (R&D Systems), human EGF or human TNFα, so that the peptide would act to anchor this antibody to the plate surface. A second detactable antibody to each analyte (R&D Systems) was also conjugated to horse radish peroxidase (HRP) to be used as the reporter antibody. EGF, IL-2 & TNFα peptide (C-terminal acid) capture IgG's & and their respective HRP detection were IgG's prepared in reaction buffer. Pure analytes as standards were diluted in PBS/BSA (0.5%) at various concentrations shown. Analyte (50 µL/well) was added to the coated plate and then added 50 µL/well of corresponding antibody mix (Capture 200 ng/mL; detection 50ng/mL). After an hour incubation with shaking, the wells were washed three time with a wash buffer, and fluorescent HRP substrate (ADHP ) added for 10min, followed by reading of the plate at 540/590nm ex/em wavelengths in a plate reader. The data shows the sensitive detection of each of EGF, IL-2 and TNFα in separate wells of a microtitre plate using a single-wash, peptide tag antibody capture system.

### EXAMPLE 18

### Detection of various concentrations of analyte using a peptide tag anti peptide tag antibody capture system

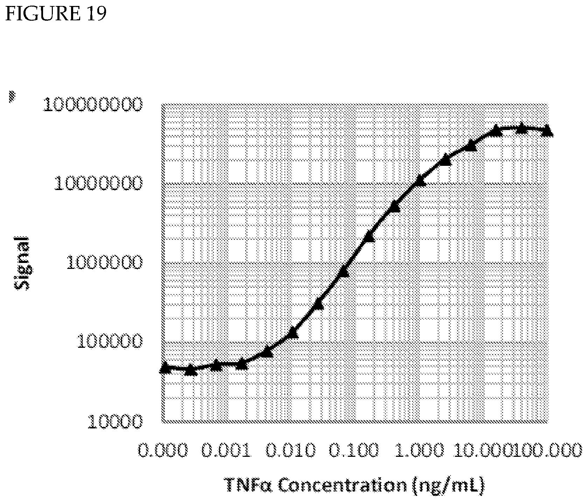

Figure 19 shows the signal obtained for various concentrations of analyte using a peptide tag anti peptide tag antibody capture system.

Antibodies were generated in mice as monoclonal antibodies to the peptide DYKDDDDK (SEQ ID NO.1). Purified antibodies to this peptide were coated onto a maxisorb Nunc immunoassay plate at 10 ug/ml, and the plate then blocked against further non-specific protein attachment. The peptide DYKDDDDK (SEQ ID NO.1) was also used to conjugate to antibodies to the human TNFα, protein (R&D Systems), so that the peptide would act to anchor this antibody to the plate surface. A second TNFα antibody (R&D Systems) was conjugated to horse radish peroxidase (HRP) to be used as the reporter antibody. TNFα was mixed with PBS/BSA (0.5%) at various concentrations shown, and to these solutions were added the IL-2 antibodies (Capture 200ng/mL; detection 50ng/mL). After an hour incubation with shaking, the wells were washed with a wash buffer, and fluorescent HRP substrate added for 10min, followed by reading of the plate at 540/590nm ex/em wavelengths in a plate reader. The data shows that the use of a peptide tag antibody capture system, whereby in this case the peptide tag was DYKDDDDK (SEQ ID NO.1), and the system was a single-wash ELISA format, enabled the sensitive measurement of TNFα with a total assay time of approximately 1 hour.

### EXAMPLE 19

### Comparison of a biotin-streptavidin capture system to a peptide tag - anti-peptide antibody capture system in various biological milieu

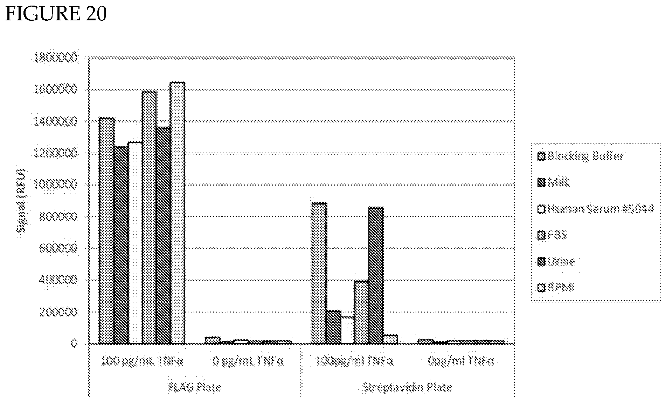

Figure 20 shows a comparison of a biotin-streptavidin capture system to a peptide tag - anti-peptide antibody capture system in various biological milieu.

Antibodies were generated in mice as monoclonal antibodies to the peptide DYKDDDDK (SEQ ID. NO.1). Purified antibodies to this peptide were coated onto a maxisorb Nunc immunoassay plate at 10 ug/ml overnight in carbonate buffer, and the plate washed and then blocked against further non-specific protein attachment. Separately, a commercial streptavidin-coated plate (Nunc Immobiliser) was used for biotin-conjugated antibodies assays. The peptide DYKDDDDK (SEQ ID NO.1) was used to conjugate to antibodies to the human TNFα protein (R&D Systems), so that the peptide would act to anchor this antibody to the plate surface to which had been coated antibodies to this peptide. Separately, antibodies to the human TNFα protein (R&D Systems), were also linked with biotin, so that this would act to anchor this antibody to the plate surface to which had been coated streptavidin. A second species of TNFα antibody (R&D Systems) was conjugated to horse radish peroxidase (HRP) to be used as the reporter antibody. TNFα was mixed with various media (blocking buffer, milk, human serum, FBS, urine or RPMI) at 100pg/mL or not added at all, and to these solutions were added either to the TNFα antibodies linked with biotin (Capture 750ng/mL; detection 50ng/mL) or peptide DYKDDDDK (SEQ ID NO.1) (Capture 300ng/mL; detection 50ng/mL), and the HRP-linked TNFα antibodies. After an hour incubation, the wells were washed with a wash buffer, and fluorescent HRP substrate ADHP added for 10min, followed by reading of the plate at 540/590nm ex/em wavelengths in a plate reader. It can be seen from the data that the peptide tag - anti-peptide antibody capture systems was superior to the biotin - streptavidin system in detecting analytes, particularly when analytes were present in particular media. Of special note are the inhibitory effects on the assay of TNFα present in milk, serum, FBS and RPMI when using the biotin - streptavidin system, reflecting the presence of biotin in these samples that interferes with this capture system.

### EXAMPLE 20

### Streptavidin biotin capture systems utilizing an antibody capture agent and an antibody detectable agent is not affected by increasing concentrations of irrelevant antibodies

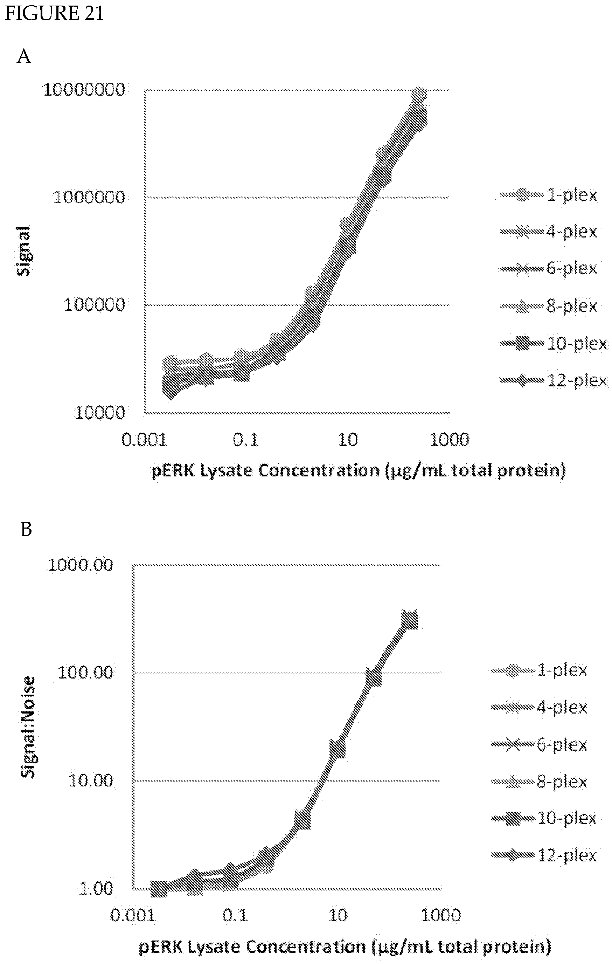

Figure 21A shows that a streptavidin biotin capture system utilizing an antibody capture agent and an antibody detectable agent is not affected by increasing concentrations of irrelevant antibodies.

Nunc Immobiliser plates, coated with streptavidin, were used in an assay to determine capacity of p-ERK antibody binding and p-ERK analyte measurement. Antibodies to the phosphorylation site of the ERK protein (TGR, Thr202/Tyr204) were linked with biotin, so that this would act to anchor this antibody to the plate surface to which has been coated streptavidin. Separately, a second ERK antibody (Santa Cruz) was linked to horse radish peroxidase (HRP) to act as a reporter antibody. Samples containing cellular lysates in which the p-ERK protein was present at various concentrations were then mixed with the ERK antibodies either in the absence (1-plex) or presence (4-12-plex) of increasing numbers of pairs of irrelevant antibodies at the same concentration as the ERK antibodies, such that one of the pair of the irrelevant antibodies was also biotinylated in the same way and extent as the ERK antibody. After 1 hour, the wells were washed with a wash buffer, and fluorescent HRP substrate ADHP added for 10min, followed by reading of the plate at 540/590nm ex/em wavelengths in a plate reader. Results are presented as absolute fluorescence signal.

Figure 21B shows the data from Figure 21A has been normalised in terms of signal:noise, where noise is the signal of the immunocomplex obtained for each condition compared to the signal obtained in the absence of analyte.

It can be seen from these graphs that the single-wash assay system with both antibodies being present with the analyte, in this case using the biotin - streptavidin pair, can use low concentrations of Capture antibodies, allowing the presence of up to 12 pairs of unrelated tagged antibodies to be present without there being any assay interference.

### EXAMPLE 21

### Anti peptide tag antibody - peptide capture systems utilizing an antibody capture agent and an antibody detectable agent is not affected by increasing concentrations of irrelevant antibodies

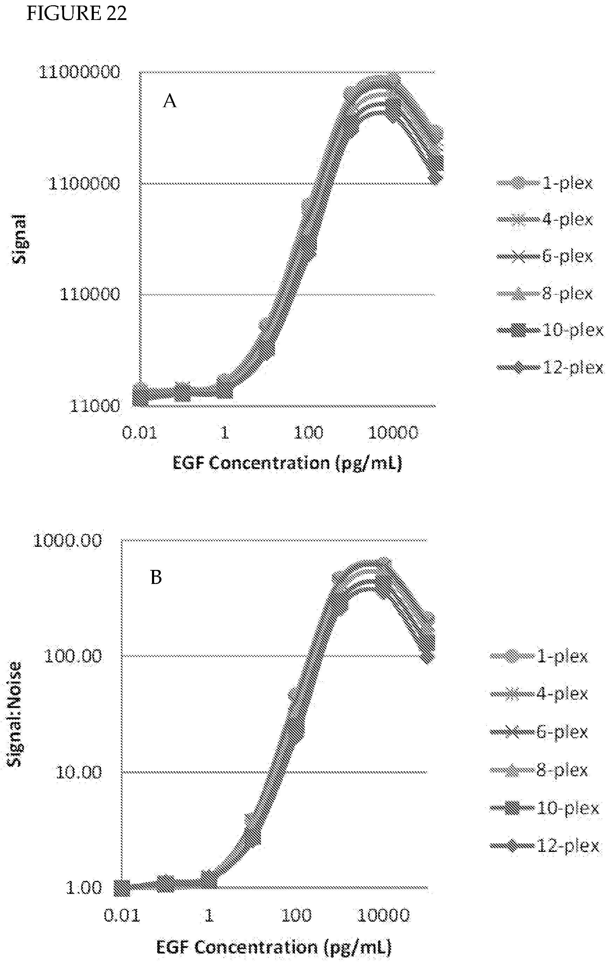

Figure 22A shows that anti peptide tag antibody - peptide capture system utilizing an antibody capture agent and an antibody detectable agent is not affected by increasing concentrations of irrelevant antibodies

Antibodies were generated in mice as monoclonal antibodies to the peptide DYKDDDDK (SEQ ID NO.1). Purified antibodies to this peptide were coated onto a maxisorb Nunc immunoassay plate, and the plate then blocked against further non-specific protein attachment. Antibodies to the human EGF protein (R&D Systems) were linked with the peptide DYKDDDDK (SEQ ID NO.1), so that this would act to anchor this antibody to the plate surface to which has been coated streptavidin. Separately, a second EGF antibody (R&D Systems) was linked to horse radish peroxidase (HRP) to act as a reporter antibody. Samples containing EGF at various concentrations were then mixed with the EGF antibodies either in the absence (1-plex) or presence (4-12-plex) of increasing numbers of pairs of irrelevant antibodies at the same concentration as the EGF antibodies, such that one of the pair of the irrelevant antibodies was also linked with the peptide DYKDDDDK in the same way and extent as the EGF antibody. After 1 hour, the wells were washed with a wash buffer, and fluorescent HRP substrate ADHP added for 10min, followed by reading of the plate at 540/590nm ex/em wavelengths in a plate reader. Results are presented as absolute fluorescence signal.

Figure 22B shows the data from Figure 22A has been normalised in terms of signal:noise, where noise is the signal of the immunocomplex obtained for each condition compared to the signal obtained in the absence of analyte.

It can be seen from these graphs that the single-wash assay system with both antibodies being present with the analyte, in this case using the peptide - anti-peptide antibody pair, can use low concentrations of Capture antibodies, allowing the presence of up to 12 pairs of unrelated tagged antibodies to be present without there being any assay interference.

### EXAMPLE 22

### Mutiplex detection of analytes

To demonstrate the ability to perform multiplex detection of two analytes, experiments were performed to detect the presence of IL2 and EGF at various concentrations by ELISA and to demonstrate that similar data were obtained when these analytes were either measured singly in each assay well or dually with two different detection tags.

EGF and IL2 were chosen as a two representative analytes for detection. Each was assayed using dual antibodies for each target, one antibody of each pair being labelled with a different lanthanide (either Europium or Samarium). The other antibody of each pair was tagged with a peptide conjugate (FLAG peptide) to facilitate capture and immobilisation on the plate coated with an anti-peptide antibody (anti-FLAG peptide antibody).

Antibodies were labelled with lanthanide with the following PerkinElmer labelling kits as per the protocols provides:
Eu-labelling kit, cat number 1244-302 - used to label the EGF antibody.
Sm-labelling kit, cat number 1244-303 - used to label the IL2 antibody.

Antibodies were incubated with the respective lanthanide solutions for 16 hours at room temperature, and conjugates were then desalted using a PD10 column.

Standard curves for each conjugate were constructed using log10 dilutions of the conjugates, maximal concentrations for Eu and Sm being 10nM and 100nM, respectively. The time-resolved fluorescence readings for these solutions were assessed in microtitre plates in a Victor II plate reader (PerkinElmer). Excitation and emission wavelengths for these lanthanides were: Europium: Excitation 340 nm/Emission 615 nm; Samarium: 340 nm/Emission 642 nm.

The data is shown in Figure 23.

It can be seen that the signal from Samarium is 1-2 log10 less at each concentration than Europium, which is consistent with the known fluorescence properties of these two molecular tags.

To perform the mono- and duo-plexing experiment for EGF and IL2, each pair of antibodies was either incubated alone with single analyte to detect (EGF or IL2), or with both antibody pairs together in the same well with both analytes.

As described, the capture antibodies for each analyte were conjugated with the FLAG peptide tag. The base of each assay well was coated with an antibody to FLAG, allowing the capture antibodies to be specifically immobilised out of solution onto the base of the plate. The detection antibody did not have the tag, and its binding and immobilisation to the base of the plate was, therefore, dependent on binding the analyte of interest.

The capacity of binding of the tagged antibodies to the base of the plate, therefore, was dependent on the binding capacity of the FLAG antibody immobilised on the base of the assay well. This binding capacity had been predetermined to be greater than the amount of tagged antibodies being presented to the well in the assays.

Antibodies were present at concentrations of 50ng/mL and 25ng/mL, for capture and detection antibodies, respectively.

In addition to measuring EGF and IL2 using antibodies tagged with the lanthanides, for comparison the same analytes were measured in separate wells of the assay plate with detection antibodies that were conjugated with HRP. In this case, the assay readout was from the enzymatic conversion of ADHP (10-Acetyl-3,7-dihydroxyphenoxazine) to resorufin. The latter was detected using standard fluorescence settings for this molecule.

The protocol of the experiment was as follows:
1. Prepare analyte(s) for measurement in PBS/BSA solution
2. Add analyte to assay plate (pre-coated with FLAG antibody) at various concentrations in different wells
3. Add both antibodies to the analyte(s) of interest to sample in wells. For single analyte detection, either EGF or IL2 antibody pair were added. For duoplex detection of both EGF and IL2 in the same well, both antibody pairs were added.
4. Incubate plate for 1 hour
5. Wash plate 3 times with PBS
6. For those samples where the detection antibody was HRP-tagged, ADHP was added and incubated for 10 min.
7. Read plate for time-resolved fluorescence at Eu and Sm wavelengths, or fluorescence (ADHP samples), depending on the detection antibody conjugate present.

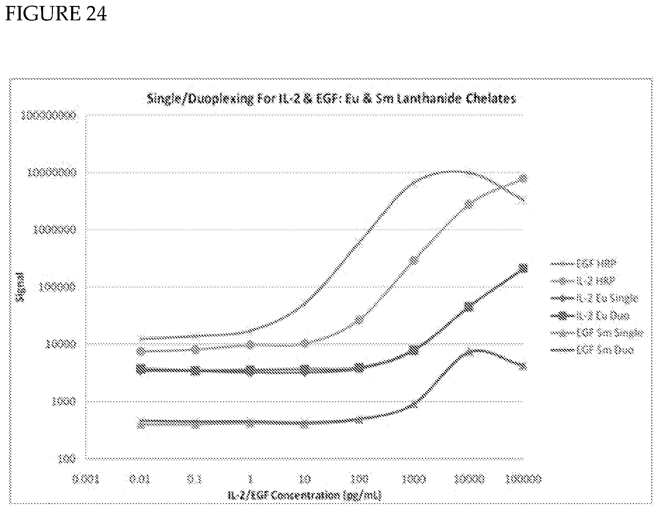

The data is shown in Figure 24.

Referring to Figure 24, the light blue line shows detection of EGF using antibodies conjugated with HRP. The orange line shows detection of IL2 using antibodies conjugated with HRP. The dark blue line shows detection of IL2 with europium tagged antibodies in a monoplex situation using europium wavelength settings on the plate reader. The red line shows detection of IL2 with europium-tagged antibodies using europium settings on the plate reader in the presence of the samarium-tagged antibodies specific for EGF. The green line shows monoplex detection of EGF with antibodies tagged with samarium, using plate reader wavelength settings specific for samarium detection. The purple line shows EGF detection with antibodies for EGF tagged with samarium using plate reader wavelength settings specific for samarium detection, in the presence of antibodies tagged with europium that are specific for IL2. For those samples measuring EGF and IL2 in the duoplex format, the readings for both EGF and IL2 have been made sequentially from the same wells containing both pairs of antibodies for each analyte.

The results demonstrate the detection of two analytes in a multiplex setting, with no apparent interference between antibodies and or lanthanide signalling.

### EXAMPLE 23

### Duoplex detection utilising enzyme tagged detection antibodies

For detection of two analytes in a single well, utilising an alternative detection system to that with lanthanides, secondary antibodies coupled to enzymes may be utilised. Similar to the method described in Example 22, primary antibodies may be tagged with the FLAG peptide to enable localisation to the base of the assay well, which has been pre-adsorbed with the anti-FLAG antibody. Using the example of the detection of EGF and IL-2, antibodies to each of these analytes may be tagged with FLAG peptide, as described above. The secondary antibodies to each analyte may be labelled with horse radish peroxidase (HRP) and alkaline phosphatase (AP), respectively.

To each assay well of a 96 well plate sample may be added to test for the presence of either EGF or IL-2. To each well is also be added both pairs of capture and detection antibody. After incubation for a period of 1 hour, the wells are washed 3 times with PBS, and then to the wells is added substrates for both HRP and AP. These substrates may be utilised as the emission characteristics of their products have a fluorescence signal that does not significantly overlapping with each other. Filter sets on the plate reader used to measure the fluorescence are chosen to provide wavelength cutoffs that allow clear measurement of each fluorescence peak. After a period of 15 minutes, the reactions in each well are stopped by the addition of a stop solution, and the fluorescence in each well determined.

### EXAMPLE 24

### Trioplex detection utilising enzyme tagged detection antibodies

For detection of three analytes in a single well, utilising an alternative detection system to that with lanthanides , secondary antibodies coupled to enzymes are utilised. Similar to the method described in Example 23, primary antibodies are tagged with the FLAG peptide to enable localisation to the base of the assay well, which had been pre-adsorbed with the anti-FLAG antibody. Using the example of the detection of EGF, IL-2 and TNFα, antibodies to each of these analytes may be tagged with FLAG peptide, as described above. The secondary antibodies to each analyte are labelled with horse radish peroxidase (HRP), alkaline phosphatase (AP), and beta-galactosidase (bGAL), respectively.

To each assay well of a 96 well plate, sample may be added to test for the presence of EGF, IL-2 or TNFα. To each well is also added pairs of capture and detection antibody for each analyte. After incubation for a period of 1 hour, the wells were washed 3 times with PBS, and then to the wells is added substrates for HRP, AP and bGAL. These substrates are selected by the emission characteristics of their products having a fluorescence signal that does not significantly overlap with each other. Filter sets on the plate reader used to measure the fluorescence are chosen to provide wavelength cutoffs that allowed clear measurement of each fluorescence peak. After a period of 15 minutes, the reactions in each well are stopped by the addition of a stop solution, and the fluorescence in each well determined.

### EXAMPLE 25

### Filter sets for fluorescence wavelength discrimination

In order to facilitate the detection of two or more analytes in a single well of a standard multi-well plate reader, it was necessary to install filter sets on the plate reader that allowed fluorescence wavelength discrimination to occur with respect to peak emission, and the bandwidth of the filters was such that it restricted wavelength overlap between the peak fluorophore outputs.

Fluorescence measurements, either of fluorophores directly attached to the detection antibodies, or of fluorescent substrates of enzymes attached to the detection antibodies, were made in each well, with sequential or simultaneous excitation and emission being carried out, depending on the capabilities of the plate reader.

### EXAMPLE 26

### Multiplex kits

Duoplex kit: The following components are contained in a duoplex kit for measurement of two analytes, where the secondary antibodies are directly tagged with different fluorophores: Primary antibody to Target 1, Primary antibody to Target 2, Secondary antibody to Target 1, Secondary antibody to Target 2, Wash buffer, Cell Lysis buffer (if required), fluorescence enhancer solution (if required), Assay plate (pre-coated with immobilisation agent to immobilise Primary Antibodies).

Trioplex kit: The following components are contained in a trioplex kit for measurement of three analytes, where the secondary antibodies are directly tagged with different fluorophores: Primary antibody to Target 1, Primary antibody to Target 2, Primary antibody to Target 3, Secondary antibody to Target 1, Secondary antibody to Target 2, Secondary antibody to Target 3, Wash buffer, Cell Lysis buffer (if required), fluorescence enhancer solution (if required), Assay plate (pre-coated with immobilisation agent to immobilise Primary Antibodies).

### EXAMPLE 27

### Sequential multiplex analysis

Cells are lysed with a buffer containing Triton X-100, buffer, and other components including phosphatase inhibitors, including sodium vanadate and sodium pyrophosphate. After addition of the lysis buffer, the plates are mixed gently, and then a sample (20uL) taken from each well and placed into a 96-well assay plate.

The assay plate has wells pre-coated an immobilisation agent, in this case streptavidin. To the assay plate is then added three pairs of antibodies, being capture and detection antibodies for detection of three different cellular analytes. The primary (capture) antibody of each pair is, in this example, labelled with biotin to facilitate capture by the immobilisation agent on the assay wells. Suitable concentrations of the primary (capture) and secondary (detection) antibodies are each 50ng/mL, for each pair of antibodies. The total binding capacity of the streptavidin in each well is approximately 2ug.

The secondary (detection) antibodies of each of the three pairs of antibodies are labelled with 3 different enzymes, to allow reaction with the subsequently added enzyme substrates that would produce products with specific fluorescence characteristics. Three suitable enzymes are horseradish peroxidase, alkaline phosphatase, and beta-galactosidase.

For example, antibodies specifically selected to measure three intracellular targets, such as p-ERK, p-AKT and p-CREB may be used. The p-ERK assay secondary (detection) antibody was labelled with beta-galactosidase, the p-AKT secondary (detection) antibody with alkaline phosphatase (AP), and the p-CREB secondary (detection) antibody with horseradish peroxidise (HRP).

After incubation for 1 hour with gentle mixing at optimal speed of 300 rpm, the wells are washed with wash buffer, and a solution containing two combined enzyme substrates added to each well to measure the HRP and AP levels, which gives a measure of p-CREB and p-AKT, respectively. These substrates are: (a) 4-Methylumbelliferyl phosphate, being the substrate for alkaline phosphatise; and (b) 10-Acetyl-3,7-dihydroxyphenoxazine (ADHP), being the substrate for horseradish peroxidase. Concentrations of these substrates may be optimised and pre-determined for the assay kit. The buffer utilised for these reactions is also optimised and pre-determined for the assay kit.

After incubation for 15 minutes with substrate solution, the plates are read in a plate reader to measure conversion of substrates to specific fluorescence products, the wavelengths of excitation and emission being shown below. These were assayed in a plate reader with capability to measure the necessary specific wavelengths following excitation at specific wavelengths.

After reading the wells for these two analytes, the wells are washed with buffer to remove the enzyme substrates. After this wash step, a solution containing a single enzyme substrate is added to each well to measure the beta-galactosidase levels, being a reflection of the levels of p-ERK in each well. This substrate is Fluorescein di-beta-D-galactopyranoside (FDG), being a specific substrate for beta-galactosidase. After incubation for 15 minutes with this substrate solution, the plates are read a second time in a plate reader for conversion of this substrate to specific fluorescence product, the wavelengths of excitation and emission as shown below. These are assayed in a plate reader with capability to measure the necessary specific wavelengths following excitation at specific wavelengths.

Wavelengths (excitation / emission):
Wavelengths: 360/450, Enzyme: Alkaline Phosphatase
Wavelengths: 460/520, Enzyme: Beta-galactosidase
Wavelengths: 550/610, Enzyme: Horseradish Peroxidase

### EXAMPLE 28

### Determination of interference between signals

The level of interference in fluorescence output between three fluorophores in solution was assessed in a 3-factor design experiment. The fluorescence emissions of the molecules methylumbelliferone, fluorescein and resorufin were assessed in PBS either alone or in combination at different combinations of concentrations between 1-10 uM in a black Maxisorg assay plate (Nunc). Fluorescence was measured at 360/450, 460/520 and 550/610nm for each mix of fluorophores, being the optimal excitation and emission wavelengths determined for each fluorophore, respectively.

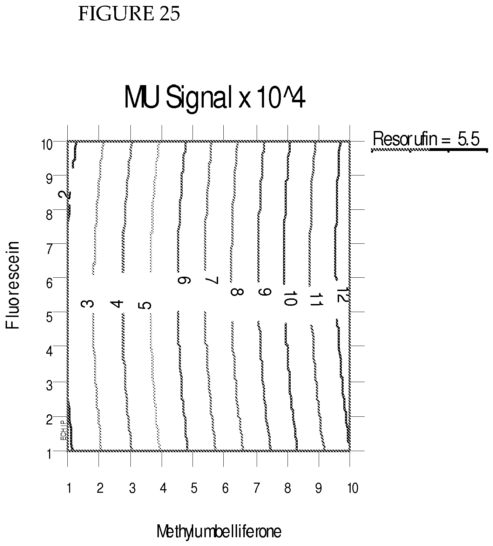

The data is shown in Figures 25 to 30.

Figure 25 shows the emission of methylumbelliferone at various concentrations in the presence of varying concentrations of fluorescein, with an interpolated concentration of resorufin of 5.5uM.

Fluorescent standards, specifically methylumbelliferone, fluorescein and resorufin, were diluted in PBS, and samples placed in wells of a 96 well microtitre plate. Concentrations of the standards ranged from 1 to 10 µM, with specific concentrations of 1, 4, 5.5, 7 and 10 µM used in a total assay volume of 100 µL. Twenty one combinations of the 3 fluorophores were analysed across this concentration range which were pre-determined using ECHIP Experimentation By Design^{™} methodology (3-factor partial cubic design). The fluorophores were mixed, and fluorescence of each well determined in a Flexstation II multiwall plate reader, at the excitation and emission wavelengths of 360/450nm, 460/520nm and 550/610nm. Using the ECHIP software, the fluorescence data was analysed using a continuous quadratic model to assess emission interference between each of the fluorescent dyes.

In this figure, the emission of methylumbelliferone at various concentrations is shown in the presence of varying concentrations of fluorescein, with an interpolated concentration of resorufin of 5.5uM. The data shows that at each concentration of methylumbelliferone, there was no interference or contribution to the methylumbelliferone fluorescence emission by fluorescein, as indicated by the vertical emission lines. Therefore, at the excitation and emission wavelengths used to measure the emission of methylumbelliferone, no interference by fluorescein was detected.

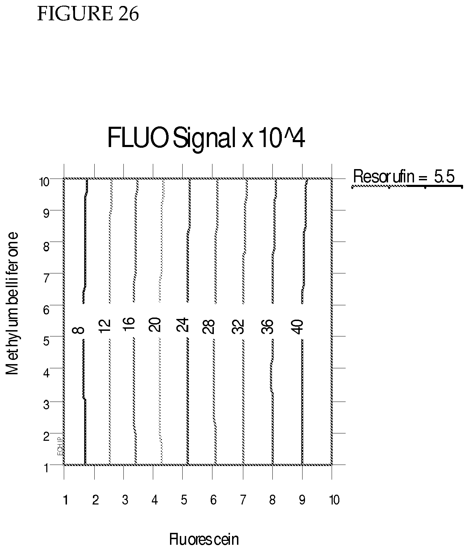

Figure 26 shows the emission of fluorescein at various concentrations in the presence of varying concentrations of methylumbelliferone, with an interpolated concentration of resorufin of 5.5uM.

Fluorescent standards, specifically methylumbelliferone, fluorescein and resorufin, were diluted in PBS, and samples placed in wells of a 96 well microtitre plate. Concentrations of the standards ranged from 1 to 10 µM, with specific concentrations of 1, 4, 5.5, 7 and 10 µM used in a total assay volume of 100 µL. Twenty one combinations of the 3 fluorophores were analysed across this concentration range which were pre-determined using ECHIP Experimentation By Design^{™} methodology (3-factor partial cubic design). The fluorophores were mixed, and fluorescence of each well determined in a Flexstation II multiwall plate reader, at the excitation and emission wavelengths of 360/450nm, 460/520nm and 550/610nm. Using the ECHIP software, the fluorescence data was analysed using a continuous quadratic model to assess emission interference between each of the fluorescent dyes.

In this figure, the emission of fluorescein at various concentrations is shown in the presence of varying concentrations of methylumbelliferone, with an interpolated concentration of resorufin of 5.5uM. The data shows that at each concentration of fluorescein, there was no interference or contribution to the fluorescein fluorescence emission by methylumbelliferone, as indicated by the vertical emission lines. Therefore, at the excitation and emission wavelengths used to measure the emission of fluorescein, no interference by methylumbelliferone was detected.

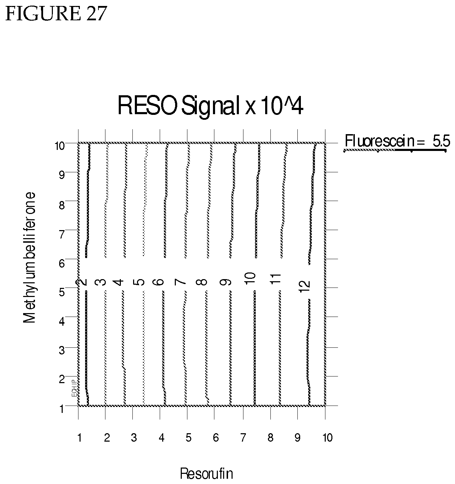

Figure 27 shows the emission of resorufin at various concentrations in the presence of varying concentrations of methylumbelliferone, with an interpolated concentration of fluorescein of 5.5uM.

Fluorescent standards, specifically methylumbelliferone, fluorescein and resorufin, were diluted in PBS, and samples placed in wells of a 96 well microtitre plate. Concentrations of the standards ranged from 1 to 10 µM, with specific concentrations of 1, 4, 5.5, 7 and 10 µM used in a total assay volume of 100 µL. Twenty one combinations of the 3 fluorophores were analysed across this concentration range which were pre-determined using ECHIP Experimentation By Design^{™} methodology (3-factor partial cubic design). The fluorophores were mixed, and fluorescence of each well determined in a Flexstation II multiwall plate reader, at the excitation and emission wavelengths of 360/450nm, 460/520nm and 550/610nm. Using the ECHIP software, the fluorescence data was analysed using a continuous quadratic model to assess emission interference between each of the fluorescent dyes.

In this figure, the emission of resorufin at various concentrations is shown in the presence of varying concentrations of methylumbelliferone, with an interpolated concentration of fluorescein of 5.5uM. The data shows that at each concentration of resorufin, there was no interference or contribution to the resorufin fluorescence emission by methylumbelliferone, as indicated by the vertical emission lines. Therefore, at the excitation and emission wavelengths used to measure the emission of resorufin, no interference by methylumbelliferone was detected.

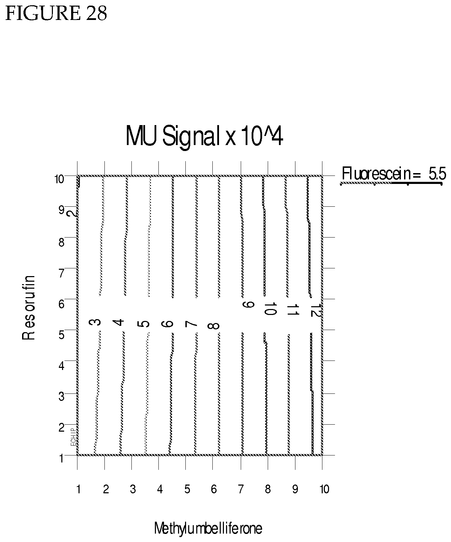

Figure 28 shows the emission of methylumbelliferone at various concentrations in the presence of varying concentrations of resorufin, with an interpolated concentration of fluorescein of 5.5uM.

Fluorescent standards, specifically methylumbelliferone, fluorescein and resorufin, were diluted in PBS, and samples placed in wells of a 96 well microtitre plate. Concentrations of the standards ranged from 1 to 10 µM, with specific concentrations of 1, 4, 5.5, 7 and 10 µM used in a total assay volume of 100 µL. Twenty one combinations of the 3 fluorophores were analysed across this concentration range which were pre-determined using ECHIP Experimentation By Design^{™} methodology (3-factor partial cubic design). The fluorophores were mixed, and fluorescence of each well determined in a Flexstation II multiwall plate reader, at the excitation and emission wavelengths of 360/450nm, 460/520nm and 550/610nm. Using the ECHIP software, the fluorescence data was analysed using a continuous quadratic model to assess emission interference between each of the fluorescent dyes.

In this figure, the emission of methylumbelliferone at various concentrations is shown in the presence of varying concentrations of resorufin, with an interpolated concentration of fluorescein of 5.5uM. The data shows that at each concentration of methylumbelliferone, there was no interference or contribution to the methylumbelliferone fluorescence emission by resorufin, as indicated by the vertical emission lines. Therefore, at the excitation and emission wavelengths used to measure the emission of methylumbelliferone, no interference by resorufin was detected.

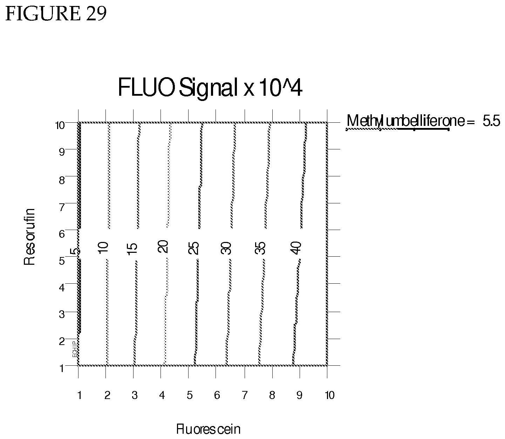

Figure 29 shows the emission of fluorescein at various concentrations is shown in the presence of varying concentrations of resorufin, with an interpolated concentration of methylumbelliferone of 5.5uM.

Fluorescent standards, specifically methylumbelliferone, fluorescein and resorufin, were diluted in PBS, and samples placed in wells of a 96 well microtitre plate. Concentrations of the standards ranged from 1 to 10 µM, with specific concentrations of 1, 4, 5.5, 7 and 10 µM used in a total assay volume of 100 µL. Twenty one combinations of the 3 fluorophores were analysed across this concentration range which were pre-determined using ECHIP Experimentation By Design^{™} methodology (3-factor partial cubic design). The fluorophores were mixed, and fluorescence of each well determined in a Flexstation II multiwall plate reader, at the excitation and emission wavelengths of 360/450nm, 460/520nm and 550/610nm. Using the ECHIP software, the fluorescence data was analysed using a continuous quadratic model to assess emission interference between each of the fluorescent dyes.

In this figure, the emission of fluorescein at various concentrations is shown in the presence of varying concentrations of resorufin, with an interpolated concentration of methylumbelliferone of 5.5uM. The data shows that at each concentration of fluorescein, there was no interference or contribution to the fluorescein fluorescence emission by resorufin, as indicated by the vertical emission lines. Therefore, at the excitation and emission wavelengths used to measure the emission of fluorescein, no interference by resorufin was detected.

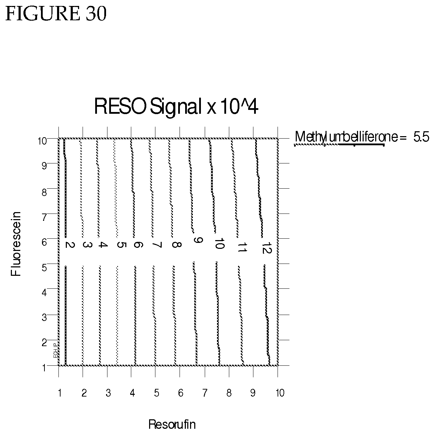

Figure 30 shows the emission of resorufin at various concentrations in the presence of varying concentrations of fluorescein, with an interpolated concentration of methylumbelliferone of 5.5uM.

Fluorescent standards, specifically methylumbelliferone, fluorescein and resorufin, were diluted in PBS, and samples placed in wells of a 96 well microtitre plate. Concentrations of the standards ranged from 1 to 10 µM, with specific concentrations of 1, 4, 5.5, 7 and 10 µM used in a total assay volume of 100 µL. Twenty one combinations of the 3 fluorophores were analysed across this concentration range which were pre-determined using ECHIP Experimentation By Design^{™} methodology (3-factor partial cubic design). The fluorophores were mixed, and fluorescence of each well determined in a Flexstation II multiwall plate reader, at the excitation and emission wavelengths of 360/450nm, 460/520nm and 550/610nm. Using the ECHIP software, the fluorescence data was analysed using a continuous quadratic model to assess emission interference between each of the fluorescent dyes.

In this figure, the emission of resorufin at various concentrations is shown in the presence of varying concentrations of fluorescein, with an interpolated concentration of methylumbelliferone of 5.5uM. The data shows that at each concentration of resorufin, there was no interference or contribution to the resorufin fluorescence emission by fluorescein, as indicated by the vertical emission lines. Therefore, at the excitation and emission wavelengths used to measure the emission of resorufin, no interference by fluorescein was detected.

The description provided herein is in relation to several embodiments which may share common characteristics and features. It is to be understood that one or more features of one embodiment may be combinable with one or more features of the other embodiments. In addition, a single feature or combination of features of the embodiments may constitute additional embodiments.

Also, it is to be noted that, as used herein, the singular forms "a", "an" and "the" include plural aspects unless the context already dictates otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

The subject headings used herein are included only for the ease of reference of the reader and should not be used to limit the subject matter found throughout the disclosure or the claims. The subject headings should not be used in construing the scope of the claims or the claim limitations.

## Claims

1. A method for detecting different non-nucleic acid analytes in a sample, the method comprising:
(a) providing to a reaction vessel:
(i) at least one solid substrate comprising one or more immobilisation agents;
(ii) the sample;
(iii) at least two different antibody capture agents that bind to at least two different analytes, wherein the at least two different capture agents can be immobilised on the at least one solid substrate;
wherein one or more of the at least two capture agents comprises a plurality of ligands for the immobilisation agent and wherein the ligand and the immobilisation agent are a binding pair comprising a peptide-tag and an anti-peptide antibody; and
(iv) at least two different detectable agents that bind to the at least two different analytes;
(b) washing the at least one solid substrate in the reaction vessel to remove one or more different capture agents and one or more different detectable agents not bound to the at least one solid substrate; and
(c) detecting the at least two different analytes in the sample by detecting the presence of the at least two different analytes bound to the at least one solid substrate, wherein said detecting is via detecting the presence of the at least two different detectable agents bound to the at least one solid substrate via the at least two analytes.

2. The method according to claim 1, wherein the method comprises
(a) providing one or more of the at least two different capture agents at a concentration of 10 ng/ml to 1000 ng/ml;
(b) providing one or more of the at least two different detectable agents at a concentration of 10 ng/ml to 1000 ng/ml; and/or
(c) providing one or more of the at least two different capture agents in solution.

3. The method according to claim 1 or 2, wherein one or more of the at least two different detectable agents comprises an antibody.

4. The method according to any one of claims 1-3, wherein one or more of the at least two detectable agents comprises one or more detectable tags.

5. The method according to claim 4, wherein the one or more detectable tags comprises a fluorophore.

6. The method according to claim 4 or 5, wherein the one or more detectable tags comprise one or more lanthanide ions, wherein the lanthanide ion is preferably one or more of Eu³⁺, Sm³⁺, Tb³⁺, and Dy³⁺, for example wherein one of the detectable tags comprises Eu³⁺ and another of the detectable tags comprises Sm³⁺.

7. The method according to claim 4, wherein the one or more of the detectable tags comprises an enzyme that converts a substrate into a detectable product, wherein the enzyme is preferably selected from the group consisting of horse radish peroxidase, alkaline phosphatase, and beta-galactosidase.

8. The method according to any one of claims 1-7, wherein the method comprises contacting the sample, one or more of the at least two different capture agents, one or more of the at least two different detectable agents and the at least one solid substrate in the reaction vessel.

9. The method according to any one of claims 1-8, wherein the sample and the solid substrate are contacted prior to contacting with one or more of the different capture agents and/or one or more of the different detectable agents and/or
wherein the sample and the at least one solid substrate are first contacted in the reaction vessel.

10. The method according to any one of claims 1 to 9, wherein
(a) the sample and the at least one solid substrate are not substantially incubated prior to contacting with one or more of the at least two different capture agents and/or one or more of the at least different detectable agents; or
(b) the method comprises an incubation of the contacted sample, the at least one solid substrate, one or more of the at least two different capture agents and one or more of the at least two different detectable agents.

11. The method according to any one of claims 1 to 10, wherein
(a) the at least two different analytes are detected simultaneously; or
(b) the at least two different analytes are detected sequentially.

12. The method according to any one of claims 1 to 11, wherein
(a) the reaction vessel comprises the at least one solid substrate;
(b) the reaction vessel comprises a pipette tip, wherein the pipette tip preferably comprises the at least one solid substrate; and/or
(c) the at least one solid substrate comprises a plurality of beads, wherein the plurality of beads are preferably retained in a pipette tip.

13. The method according to any one of claims 1 to 12, wherein
(a) the sample is a blood sample, a serum sample, a urine sample, a cell sample, a cell lysate or a combination of any of the aforementioned;
(b) one or more of the at least two different analytes are selected from the group consisting of a component of a cell signalling pathway, a cytokine, or a tumour suppressor; and/or
(c) one or more of the at two different analytes comprises a protein, wherein the protein is preferably a phosphoprotein, more preferably wherein one or more of the at least two different analytes is selected from the group consisting of phospho-ERK 1/2; total ERK 1/2; phospho-Akt 1/2/3; total Akt 1/2/3; phospho-NF-Kβ p65; total NF-Kβ p65; phospho-I-kBa; total-kBcc; phospho-STAT3; total STAT3; phospho-STAT5 A/B; phospho-JNK 1/2/3; total JNK 1/2/3; phospho-p38 MAPKcc; total p38 MAPKD ; phospho-p53; total p53; phospho-p70S6K; total p70S6K; and GAPDH.

14. The method according to any one of claims 1 to 13, wherein the method comprises production of
(a) at least one detectable signal with a peak emission wavelength in the range from 500-560 nm and another detectable signal with a peak emission wavelength in the range from 570-610;
(b) at least one detectable signal with a peak emission wavelength in the range from 380-490 nm and another detectable signal with a peak emission wavelength in the range from 500-560;
(c) at least one detectable signal with a peak emission wavelength in the range from 380-490 nm and another detectable signal with a peak emission wavelength in the range from 570 to 750 nm; or
(d) at least one detectable signal with a peak emission wavelength in the range from 500-560 nm and another detectable signal with a peak emission wavelength in the range from 570 to 750 nm.

15. The method according to any one of claims 1 to 14, wherein the detecting of the at least two different analytes is achieved in a time of 2 hours or less; and/or wherein the method comprises providing one or more filters and/or one or more light sources and/or one or more light detectors to a plate reader device to enable detection of the at least two different analytes.

16. A kit for performing the method of any one of claims 1-15, the kit comprising
(i) at least one solid substrate comprising one or more immobilisation agents;
(ii) at least two different antibody capture agents that bind to at least two different analytes, wherein the at least two different capture agents can be immobilised on the at least one solid substrate;
wherein one or more of the at least two capture agents comprises a plurality of ligands for the immobilisation agent and wherein the ligand and the immobilisation agent are a binding pair comprising a peptide-tag and an anti-peptide antibody;
and
(iii) at least two different detectable agents that bind to the at least two different analytes; and optionally
a reaction vessel; a solution for washing the reaction vessel to remove capture agents and/or detectable agents not bound to the reaction vessel; a cell lysis buffer, a signal enhancing agent, and/or instructions for detection of the different analytes.

## Patentansprüche

1. Verfahren zum Nachweis unterschiedlicher Nicht-Nukleinsäure-Analyten in einer Probe, wobei das Verfahren umfasst:
(a) Bereitstellen in einem Reaktionsgefäß:
(i) wenigstens ein festes Substrat, das ein oder mehrere Immobilisierungsmittel umfasst;
(ii) die Probe;
(iii) wenigstens zwei unterschiedliche Antikörper-Fangmittel, die an wenigstens zwei unterschiedliche Analyten binden, wobei die wenigstens zwei unterschiedlichen Fangmittel auf dem wenigstens einen festen Substrat immobilisiert werden können; wobei eines oder mehrere der wenigstens zwei Fangmittel eine Mehrzahl an Liganden für das Immobilisierungsmittel umfassen und wobei der Ligand und das Immobilisierungsmittel ein Bindungspaar sind, das ein Peptid-Tag und einen Anti-Peptid-Antikörper umfasst; und
(iv) wenigstens zwei unterschiedliche nachweisbare Mittel, die an die wenigstens zwei unterschiedlichen Analyten binden;
(b) Waschen des wenigstens einen festen Substrats in dem Reaktionsgefäß, um ein oder mehrere unterschiedliche Fangmittel und ein oder mehrere unterschiedliche nachweisbare Mittel, die nicht an das wenigstens eine feste Substrat gebunden sind, zu entfernen; und
(c) Nachweisen der wenigstens zwei unterschiedlichen Analyten in der Probe durch Nachweisen des Vorhandenseins der wenigstens zwei unterschiedlichen Analyten, die an das wenigstens eine feste Substrat gebunden sind, wobei das Nachweisen über das Nachweisen des Vorhandenseins der wenigstens zwei unterschiedlichen nachweisbaren Mittel erfolgt, die an das wenigstens eine feste Substrat über die wenigstens zwei Analyten gebunden sind.

2. Verfahren nach Anspruch 1, wobei das Verfahren umfasst
(a) Bereitstellen eines oder mehrerer der wenigstens zwei unterschiedlichen Fangmittel in einer Konzentration von 10 ng/mL bis 1000 ng/mL;
(b) Bereitstellen eines oder mehrerer der wenigstens zwei unterschiedlichen nachweisbaren Mittel in einer Konzentration von 10 ng/mL bis 1000 ng/mL; und/oder
(c) Bereitstellen eines oder mehrerer der wenigstens zwei unterschiedlichen Fangmittel in Lösung.

3. Verfahren nach Anspruch 1 oder 2, wobei eines oder mehrere der wenigstens zwei unterschiedlichen nachweisbaren Mittel einen Antikörper umfasst/umfassen.

4. Verfahren nach einem der Ansprüche 1-3, wobei eines oder mehrere der wenigstens zwei nachweisbaren Mittel ein oder mehrere nachweisbare Tags umfasst/umfassen.

5. Verfahren nach Anspruch 4, wobei das eine oder die mehreren nachweisbaren Tags einen Fluorophor umfasst/umfassen.

6. Verfahren nach Anspruch 4 oder 5, wobei das eine oder die mehreren nachweisbaren Tag ein oder mehrere Lanthanid-Ionen umfassen, wobei das Lanthanid-Ion vorzugsweise eines oder mehrere von Eu³⁺, Sm³⁺, Tb³⁺ und Dy³⁺ ist, wobei beispielsweise eines der nachweisbaren Tags Eu³⁺ umfasst und ein anderes der nachweisbaren Tags Sm³⁺ umfasst.

7. Verfahren nach Anspruch 4, wobei das eine oder die mehreren nachweisbaren Tags ein Enzym umfasst/umfassen, das ein Substrat in ein nachweisbares Produkt umwandelt, wobei das Enzym vorzugsweise ausgewählt ist aus der Gruppe bestehend aus Meerrettichperoxidase, alkalischer Phosphatase und beta-Galactosidase.

8. Verfahren nach einem der Ansprüche 1-7, wobei das Verfahren Inkontaktbringen der Probe, eines oder mehrerer der wenigstens zwei unterschiedlichen Fangmittel, eines oder mehrerer der wenigstens zwei unterschiedlichen nachweisbaren Mittel und des wenigstens einen festen Substrats in dem Reaktionsgefäß umfasst.

9. Verfahren nach einem der Ansprüche 1-8, wobei die Probe und das feste Substrat vor Inkontaktbringen mit einem oder mehreren der unterschiedlichen Fangmittel und/oder einem oder mehreren der unterschiedlichen nachweisbaren Mittel in Kontakt gebracht werden und/oder wobei die Probe und das wenigstens eine feste Substrat zuerst in dem Reaktionsgefäß in Kontakt gebracht werden.

10. Verfahren nach einem der Ansprüche 1 bis 9, wobei
(a) die Probe und das wenigstens eine feste Substrat vor Inkontaktbringen mit einem oder mehreren der wenigstens zwei unterschiedlichen Fangmittel und/oder einem oder mehreren der wenigstens unterschiedlichen nachweisbaren Mittel nicht wesentlich inkubiert werden; oder
(b) das Verfahren eine Inkubation der in Kontakt gebrachten Probe, des wenigstens einen festen Substrats, eines oder mehrerer der wenigstens zwei unterschiedlichen Fangmittel und eines oder mehrerer der wenigstens zwei unterschiedlichen nachweisbaren Mittel umfasst.

11. Verfahren nach einem der Ansprüche 1 bis 10, wobei
(a) die wenigstens zwei unterschiedlichen Analyten gleichzeitig nachgewiesen werden; oder
(b) die wenigstens zwei unterschiedlichen Analyten nacheinander nachgewiesen werden.

12. Verfahren nach einem der Ansprüche 1 bis 11, wobei
(a) das Reaktionsgefäß das wenigstens eine feste Substrat umfasst;
(b) das Reaktionsgefäß eine Pipettenspitze umfasst, wobei die Pipettenspitze vorzugsweise das wenigstens eine feste Substrat umfasst; und/oder
(c) das wenigstens eine feste Substrat eine Mehrzahl an Kügelchen umfasst, wobei vorzugsweise die Mehrzahl an Kügelchen in einer Pipettenspitze gehalten wird.

13. Verfahren nach einem der Ansprüche 1 bis 12, wobei
(a) die Probe eine Blutprobe, eine Serumprobe, eine Urinprobe, eine Zellprobe, ein Zelllysat oder eine beliebige Kombination der Vorgenannten ist;
(b) einer oder mehrere der wenigstens zwei unterschiedlichen Analyten ausgewählt ist/sind aus der Gruppe bestehend aus einer Komponente eines Zellsignalwegs, einem Zytokin oder einem Tumorsuppressor; und/oder
(c) einer oder mehrere der zwei unterschiedlichen Analyten ein Protein umfasst/umfassen, wobei das Protein vorzugsweise ein Phosphoprotein ist, besonders bevorzugt, wobei einer oder mehrere der wenigstens zwei unterschiedlichen Analyten ausgewählt ist/sind aus der Gruppe bestehend aus Phospho-ERK 1/2; Gesamt-ERK 1/2; Phospho-Akt 1/2/3; Gesamt-Akt 1/2/3; Phospho-NF-Kβ p65; Gesamt NF-Kβ p65; Phospho-I-kBa: Gesamt-kBcc; Phospho-STAT3; Gesamt-STAT3; Phospho-STAT5 A/B; Phospho-JNK 1/2/3; Gesamt-JNK 1/2/3; Phospho-p38 MAPKcc; Gesamt-p38 MAPKD; Phospho-p53; Gesamt-p53; Phospho-p70S6K; Gesamt-p70S6K; und GAPDH.

14. Verfahren nach einem der Ansprüche 1 bis 13, wobei das Verfahren die Herstellung von
(a) wenigstens einem nachweisbaren Signal mit einer Spitzenemissionswellenlänge in dem Bereich von 500-560 nm und einem weiteren nachweisbaren Signal mit einer Spitzenemissionswellenlänge in dem Bereich von 570-610:
(b) wenigstens einem nachweisbaren Signal mit einer Spitzenemissionswellenlänge in dem Bereich von 380-490 nm und einem weiteren nachweisbaren Signal mit einer Spitzenemissionswellenlänge in dem Bereich von 500-560;
(c) wenigstens einem nachweisbaren Signal mit einer Spitzenemissionswellenlänge in dem Bereich von 380-490 nm und einem weiteren nachweisbaren Signal mit einer Spitzenemissionswellenlänge in dem Bereich von 570 bis 750 nm; oder
(d) wenigstens einem nachweisbaren Signal mit einer Spitzenemissionswellenlänge in dem Bereich von 500-560 nm und einem weiteren nachweisbaren Signal mit einer Spitzenemissionswellenlänge in dem Bereich von 570 bis 750 nm umfasst.

15. Verfahren nach einem der Ansprüche 1 bis 14, wobei das Nachweisen der wenigstens zwei unterschiedlichen Analyten in einer Zeit von 2 Stunden oder weniger erreicht wird; und/oder
wobei das Verfahren Bereitstellen eines oder mehrerer Filter und/oder einer oder mehrerer Lichtquellen und/oder eines oder mehrerer Lichtdetektoren für eine Plattenlesevorrichtung umfasst, um ein Nachweisen der wenigstens zwei unterschiedlichen Analyten zu ermöglichen.

16. Kit zum Durchführen des Verfahrens nach einem der Ansprüche 1-15, wobei das Kit umfasst:
(i) wenigstens ein festes Substrat, das ein oder mehrere Immobilisierungsmittel umfasst;
(ii) wenigstens zwei unterschiedliche Antikörper-Fangmittel, die an wenigstens zwei unterschiedlichen Analyten binden, wobei die wenigstens zwei unterschiedlichen Fangmittel auf dem wenigstens einen festen Substrat immobilisiert werden können;
wobei eines oder mehrere der wenigstens zwei Fangmittel eine Mehrzahl an Liganden für das Immobilisierungsmittel umfassen und wobei der Ligand und das Immobilisierungsmittel ein Bindungspaar sind, das ein Peptid-Tag und einen Anti-Peptid-Antikörper umfasst;
und
(iii)wenigstens zwei unterschiedliche nachweisbare Mittel, die an die wenigstens zwei unterschiedlichen Analyten binden; und gegebenenfalls ein Reaktionsgefäß; eine Lösung zum Waschen des Reaktionsgefäßes, um Fangmittel und/oder nachweisbare Mittel, die nicht an das Reaktionsgefäß gebunden sind, zu entfernen; einen Zelllysepuffer, ein signalverstärkendes Mittel und/oder Anweisungen zum Nachweis der unterschiedlichen Analyten.

## Revendications

1. Procédé de détection de différents analytes d'acides non nucléiques dans un échantillon, le procédé comprenant les étapes consistant à :
(a) fournir à un récipient de réaction :
(i) au moins un substrat solide comprenant un ou plusieurs agent(s) d'immobilisation ;
(ii) l'échantillon ;
(iii) au moins deux agents de capture d'anticorps différents qui se lient à au moins deux analytes différents, où les au moins deux agents de capture différents peuvent être immobilisés sur l'au moins un substrat solide ;
dans lequel un ou plusieurs des au moins deux agents de capture comprend/comprennent une pluralité de ligands pour l'agent d'immobilisation et dans lequel le ligand et l'agent d'immobilisation sont une paire de liaison comprenant un marqueur peptidique et un anticorps anti-peptide ; et
(iv) au moins deux agents détectables différents qui se lient aux au moins deux analytes différents ;
(b) laver l'au moins un substrat solide dans le récipient de réaction pour retirer un ou plusieurs agent(s) de capture différent(s) et un ou plusieurs agent(s) détectable(s) différent(s) non liés à l'au moins un substrat solide ; et
(c) détecter les au moins deux analytes différents dans l'échantillon en détectant la présence des au moins deux analytes différents liés à l'au moins un substrat solide, où ladite détection se fait par la détection de la présence des au moins deux agents détectables différents liés à l'au moins un substrat solide par les au moins deux analytes.

2. Procédé selon la revendication 1, dans lequel le procédé comprend les étapes consistant à
(a) fournir un ou plusieurs des au moins deux agents de capture différents à une concentration allant de 10 ng/ml à 1000 ng/ml ;
(b) fournir un ou plusieurs des au moins deux agents détectables différents à une concentration allant de 10 ng/ml à 1000 ng/ml ; et/ou
(c) fournir un ou plusieurs des au moins deux agents de capture différents en solution.

3. Procédé selon la revendication 1 ou 2, dans lequel un ou plusieurs des au moins deux agents détectables différents comprend/comprennent un anticorps.

4. Procédé selon l'une quelconque des revendications 1 à 3, dans lequel un ou plusieurs des au moins deux agents détectables comprend/comprennent un ou plusieurs marqueur(s) détectable(s).

5. Procédé selon la revendication 4, dans lequel le ou les plusieurs marqueur(s) détectable(s) comprend/comprennent un fluorophore.

6. Procédé selon la revendication 4 ou 5, dans lequel le ou les plusieurs marqueur(s) détectable(s) comprend/comprennent un ou plusieurs ion(s) lanthanide, où l'ion lanthanide est de préférence un ou plusieurs parmi Eu³⁺, Sm³⁺, Tb³⁺ et Dy³⁺, par exemple où l'un des marqueurs détectables comprend Eu³⁺ et un autre des marqueurs détectables comprend Sm³⁺.

7. Procédé selon la revendication 4, dans lequel le ou les plusieurs des marqueurs détectables comprend/comprennent une enzyme qui convertit un substrat en un produit détectable, où l'enzyme est de préférence choisie dans le groupe consistant en la peroxydase de raifort, la phosphatase alcaline et la bêta-galactosidase.

8. Procédé selon l'une quelconque des revendications 1 à 7, dans lequel le procédé comprend l'étape consistant à mettre en contact l'échantillon, un ou plusieurs des au moins deux agents de capture différents, un ou plusieurs des au moins deux agents détectables différents et l'au moins un substrat solide dans le récipient de réaction.

9. Procédé selon l'une quelconque des revendications 1 à 8, dans lequel l'échantillon et le substrat solide sont mis en contact avant la mise en contact avec un ou plusieurs des agents de capture différents et/ou un ou plusieurs des agents détectables différents et/ou
dans lequel l'échantillon et l'au moins un substrat solide sont d'abord mis en contact dans le récipient de réaction.

10. Procédé selon l'une quelconque des revendications 1 à 9, dans lequel
(a) l'échantillon et l'au moins un substrat solide ne sont pas sensiblement incubés avant la mise en contact avec un ou plusieurs des au moins deux agents de capture différents et/ou un ou plusieurs des au moins différents agents détectables ; ou
(b) le procédé comprend une incubation de l'échantillon mis en contact, de l'au moins un substrat solide, d'un ou de plusieurs des au moins deux agents de capture différents et d'un ou de plusieurs des au moins deux agents détectables différents.

11. Procédé selon l'une quelconque des revendications 1 à 10, dans lequel
(a) les au moins deux analytes différents sont détectés simultanément ; ou
(b) les au moins deux analytes différents sont détectés successivement.

12. Procédé selon l'une quelconque des revendications 1 à 11, dans lequel
(a) le récipient de réaction comprend l'au moins un substrat solide ;
(b) le récipient de réaction comprend une pointe de pipette, où la pointe de pipette comprend de préférence l'au moins un substrat solide ; et/ou
(c) l'au moins un substrat solide comprend une pluralité de billes, où la pluralité de billes est de préférence retenue dans une pointe de pipette.

13. Procédé selon l'une quelconque des revendications 1 à 12, dans lequel
(a) l'échantillon est un échantillon de sang, un échantillon de sérum, un échantillon d'urine, un échantillon de cellule, un lysat cellulaire ou une combinaison de l'un des éléments susmentionnés ;
(b) un ou plusieurs des au moins deux analytes différents est/sont choisi(s) dans le groupe consistant en un composant d'une voie de signalisation cellulaire, une cytokine ou un suppresseur de tumeur ; et/ou
(c) un ou plusieurs des au moins deux analytes différents comprend/comprennent une protéine, où la protéine est de préférence une phosphoprotéine, plus préférablement, où un ou plusieurs des au moins deux analytes différents est/sont choisi(s) dans le groupe consistant en phospho-ERK 1/2 ; ERK 1/2 totale ; phospho-Akt 1/2/3 ; Akt 1/2/3 totale ; phospho-NF-Kβ p65 ; NF-Kβ p65 total ; phospho-I-kBa ; kBcc total ; phospho-STAT3 ; STAT3 total ; phospho-STAT5 A/B ; phospho-JNK 1/2/3 ; JNK 1/2/3 totale ; phospho-p38 MAPKcc ; p38 MAPKD total ; phospho-p53 ; p53 total ; phospho-p70S6K ; p70S6K total ; et GAPDH.

14. Procédé selon l'une quelconque des revendications 1 à 13, dans lequel le procédé comprend la production
(a) d'au moins un signal détectable avec une longueur d'onde d'émission de pic dans la plage allant de 500 à 560 nm et d'un autre signal détectable avec une longueur d'onde d'émission de pic dans la plage allant de 570 à 610 ;
(b) d'au moins un signal détectable avec une longueur d'onde d'émission de pic dans la plage allant de 380 à 490 nm et d'un autre signal détectable avec une longueur d'onde d'émission de pic dans la plage allant de 500 à 560 ;
(c) d'au moins un signal détectable avec une longueur d'onde d'émission de pic dans la plage allant de 380 à 490 nm et d'un autre signal détectable avec une longueur d'onde d'émission de pic dans la plage allant de 570 à 750 nm ; ou
(d) d'au moins un signal détectable avec une longueur d'onde d'émission de pic dans la plage allant de 500 à 560 nm et d'un autre signal détectable avec une longueur d'onde d'émission de pic dans la plage allant de 570 à 750 nm.

15. Procédé selon l'une quelconque des revendications 1 à 14, dans lequel la détection des au moins deux analytes différents est réalisée dans une durée de 2 heures ou moins ; et/ou dans lequel le procédé comprend l'étape consistant à fournir un ou plusieurs filtre(s) et/ou une ou plusieurs source(s) de lumière et/ou un ou plusieurs détecteur(s) de lumière à un dispositif lecteur de plaque pour permettre la détection des au moins deux analytes différents.

16. Kit de réalisation du procédé de l'une quelconque des revendications 1 à 15, le kit comprenant
(i) au moins un substrat solide comprenant un ou plusieurs agent(s) d'immobilisation ;
(ii) au moins deux agents de capture d'anticorps différents qui se lient à au moins deux analytes différents, où les au moins deux agents de capture différents peuvent être immobilisés sur l'au moins un substrat solide ;
dans lequel un ou plusieurs des au moins deux agents de capture comprend/comprennent une pluralité de ligands pour l'agent d'immobilisation et dans lequel le ligand et l'agent d'immobilisation sont une paire de liaison comprenant un marqueur peptidique et un anticorps anti-peptide ;
et
(iii) au moins deux agents détectables différents qui se lient aux au moins deux analytes différents ; et éventuellement
un récipient de réaction ; une solution pour laver le récipient de réaction pour retirer les agents de capture et/ou les agents détectables non liés au récipient de réaction ; un tampon de lyse cellulaire, un agent d'amélioration de signal et/ou des instructions pour la détection des différents analytes.
